# EUROPEAN PATENT SPECIFICATION

(11) **EP 2 300 484 B1**
(45) Date of publication and mention of the grant of the patent: **14.12.2011**
(21) Application number: 09742523.5
(22) Date of filing: 04.05.2009
(51) Int. Cl.: C07D 513/10, A61K 31/4436, A61P 25/28

(54) **NOVEL CLASS OF SPIRO PIPERIDINES FOR THE TREATMENT OF NEURODEGENERATIVE DISEASES**
NEUE KLASSE VON SPIRO-PIPERIDINEN ZUR BEHANDLUNG NEURODEGENERATIVER KRANKHEITEN
NOUVELLE CLASSE DE SPIRO PIPÉRIDINES POUR LE TRAITEMENT DE MALADIES NEURODÉGÉNÉRATIVES

(30) Priority: 05.05.2008 US 50423 P
(43) Date of publication of application: 30.03.2011
(73) Proprietor: Pfizer Inc., New York, NY 10017 (US)
(72) Inventor: BRODNEY, Michael, Aaron, Groton, CT 06340 (US); HELAL, Christopher, John, Groton, Connecticut 06340 (US); O'NEILL, Brian, Thomas, Groton, Connecticut 06340 (US)
(74) Representative: Laurent, Claire
(86) International application number: PCT/IB2009/051815
(87) International publication number: WO 2009/136350

(56) References cited:
- WO-A-2006/044497
- WO-A-2007/011810
- WO-A-2008/030412

## Description

### FIELD OF THE INVENTION

The present invention relates to the treatment of Alzheimer's disease and other neurodegenerative and/or neurological disorders in mammals, including humans. This invention also relates to inhibiting, in mammals, including humans, the production of A-beta peptides that can contribute to the formation of neurological deposits of amyloid protein. More particularly, this invention relates to spiro-piperidine compounds useful for the treatment of neurodegenerative and/or neurological disorders, such as Alzheimer's disease and Down's Syndrome, related to A-beta peptide production.

### BACKGROUND OF THE INVENTION

Dementia results from a wide variety of distinctive pathological processes. The most common pathological processes causing dementia are Alzheimer's disease (AD), cerebral amyloid angiopathy (CM) and prion-mediated diseases (see, e.g., Haan et a/. Clin. Neuro. Neurosurg. 1990, 92(4):305-310; Glenner et al., J. Neurol. Sci. 1989, 94:1-28). AD affects nearly half of all people past the age of 85, the most rapidly growing portion of the United States population. As such, the number of AD patients in the United States is expected to increase from about 4 million to about 14 million by the middle of the next century.

Treatment of AD typically is the support provided by a family member in attendance. Stimulated memory exercises on a regular basis have been shown to slow, but not stop, memory loss. A few drugs, for example Aricept™, provide treatment of AD.

Alzheimer's disease is characterized by two major pathological observations in the brain: neurofibrillary tangles and beta amyloid (or neuritic) plaques, comprised predominantly of an aggregate of a peptide fragment know as A-beta (also sometimes designated betaA4). Individuals with AD exhibit characteristic beta-amyloid deposits in the brain (beta amyloid plaques) and in cerebral blood vessels (beta amyloid angiopathy) as well as neurofibrillary tangles. Neurofibrillary tangles occur not only in Alzheimer's disease but also in other dementia-inducing disorders. On autopsy, large numbers of these lesions are generally found in areas of the human brain important for memory and cognition. Smaller numbers of these lesions in a more restricted anatomical distribution are found in the brains of most aged humans who do not have clinical AD. Amyloidogenic plaques and vascular amyloid angiopathy also characterize the brains of individuals with Trisomy 21 (Down's Syndrome), Hereditary Cerebral Hemorrhage with Amyloidosis of the Dutch-Type (HCHWA-D), and other neurodegenerative disorders.

Beta-amyloid plaques are predominantly composed of amyloid beta peptide. Several lines of evidence indicate that progressive cerebral deposition of beta-amyloid peptide (A-beta) plays a seminal role in the pathogenesis of AD and can precede cognitive symptoms by years or decades. See, for example, Selkoe, 1991, Neuron 6:487. Release of A-beta from neuronal cells grown in culture and the presence of A-beta in cerebrospinal fluid (CSF) of both normal individuals and AD patients has been demonstrated. See, for example, Seubert et al., 1992, Nature 359:325-327. It has been proposed that A-beta peptide accumulates as a result of APP processing by beta-secretase, thus inhibition of this enzyme's activity is desirable for the treatment of AD. In vivo processing of APP at the beta-secretase cleavage site is thought to be a rate-limiting step in A-beta production, and is thus a therapeutic target for the treatment of AD. See for example, Sabbagh, M., et al., 1997, Alz. Dis. Rev. 3, 1-19.

Beta-site APP-cleaving enzyme 1 (BACE1) knockout mice fail to produce A-beta, and present a normal phenotype. When crossed with transgenic mice that overexpress APP, the progeny show reduced amounts of A beta in brain extracts as compared with control animals (Luo et al., 2001 Nature Neuroscience 4:231-232). This evidence further supports the proposal that inhibition of beta-secretase activity and reduction of A-beta in the brain provides a therapeutic method for the treatment of AD and other beta amyloid disorders.

At present there are no effective treatments for halting, preventing, or reversing the progression of Alzheimer's disease. Therefore, there is an urgent need for pharmaceutical agents capable of slowing the progression of Alzheimer's disease and/or preventing it in the first place.

Compounds that are effective inhibitors of beta-secretase, that inhibit beta-secretase-mediated cleavage of APP, that are effective inhibitors of A beta production, and/or are effective to reduce amyloid beta deposits or plaques, are needed for the treatment and prevention of disease characterized by amyloid beta deposits or plaques, such as AD.

### SUMMARY OF THE INVENTION

The invention is directed to a compound, including the pharmaceutically acceptable salts thereof, having the structure of formula I: 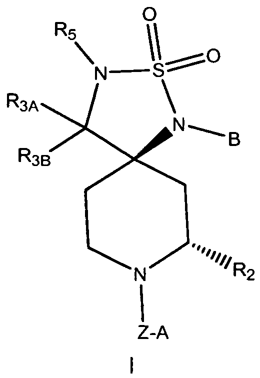 wherein the stereochemistry shown in formula I at the carbon bonded to R₂ and at the spirocyclic carbon is the absolute stereochemistry; B is aryl, heteroaryl, cycloalkyl, or heterocycloalkyl, wherein B is optionally substituted with zero to three R₄ groups;

Z is (CH₂)ₙ-Oₚ-(CH₂)_{q}, or a cycloalkylene moiety, provided that when Z is (CH₂)ₙ-Oₚ-(CH₂)_{q}, the terminal methylene of the (CH₂)ₙ- chain is bonded to the nitrogen of the piperidinyl ring;

A is independently aryl, cycloalkyl, heterocycloalkyl or heteroaryl wherein said aryl, cycloalkyl, heterocycloalkyl or heteroaryl is optionally substituted with one to three R₁;
each R₁ is independently alkyl, halogen, cyano, SO₂NHR₉, CON(R₈)₂, N(R₈)COR₈, SO₂N(R₈)₂, N(R₈)SO₂R₈, COR₈, SO₂R₈, (CH₂)cycloalkyl, (CH₂)ₜ-heterocycloalkyl, (CH₂)ₜ-aryl, (CH₂)ₜ-heteroaryl, (CH₂)ₜ-N(R₈)₂, or (CH₂)ₜ-OR₆ wherein each R₁ alkyl, (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆;
R₂ is alkyl, cycloalkyl, or alkenyl wherein said alkyl, cycloalkyl, or alkenyl is optionally substituted with halogen, hydroxyl, or cyano;
R_{3A} and R_{3B} are each independently hydrogen, aryl, heteroaryl, or alkyl optionally substituted with R₁;
or R_{3A} and R_{3B} together with the carbon they are bonded to form a C=O, C=NR₈, a cycloalkylene moiety or a heterocycloalkylene moiety;
each R₄ is independently halogen, hydroxyl, cyano, halo, O-alkyl, O-cycloalkyl, SO₂R₈, N(R₈)₂, COR₈, CON(R₈)₂, alkyl, (CH₂)ₜ-cycloalkyl, (CH₂)ₜ-heterocycloalkyl, (CH₂)ₜ-aryl, or (CH₂)ₜ-heteroaryl wherein said R₄ alkyl, (CH₂)ₜ-cycloalkyl, (CH₂)ₜ-heterocycloalkyl, (CH₂)ₜ-aryl, or (CH₂)ₜ-heteroaryl is optionally substituted with R₁;
each R₅ is independently hydrogen, alkyl, alkenyl, (CH₂)ₜ-cycloalkyl, (CH₂)ₜ-heterocycloalkyl, (CH₂)ₜ-aryl, or (CH₂)ₜ-heteroaryl, wherein said alkyl, alkenyl, (CH₂)ₜ-cycloalkyl, (CH₂)ₜ-heterocycloalkyl, (CH₂)ₜ-aryl, or (CH₂)ₜ-heteroaryl R₅ substituent is optionally substituted with one or more hydroxyl, aryl, heteroaryl, halogen, alkyl, cycloalkyl, SO₂R₈, -NR₈COR₈, -CON(R₈)₂, COOR₈, -C(O)R₈, -CN, or N(R₈)₂, wherein said aryl, alkyl, cycloalkyl and heteroaryl substituent is optionally substituted with one or more halogen, alkyl, hydroxyl, or -O-alkyl;
each R₆ is independently hydrogen, alkyl, (CH₂)ₜ-cycloalkyl, (CH₂)ₜ-heterocycloalkyl, (CH₂)ₜ-aryl, or (CH₂)ₜ-heteroaryl wherein said (CH₂)ₜ-cycloalkyl, (CH₂)ₜ-heterocycloalkyl, (CH₂)ₜ-aryl, or (CH₂)ₜ-heteroaryl is optionally substituted with one to three R₇;
each R₇ is independently alkyl, hydroxyl, alkoxy, halogen, cyano, amino, alkylamino, dialkylamino, (CH₂)ₜ-cycloalkyl, (CH₂)ₜ-heterocycloalkyl, (CH₂)ₜ-aryl, or (CH₂)ₜ-heteroaryl;
each R₈ is independently hydrogen, alkyl, (CH₂)ₜ-cycloalkyl, (CH₂)ₜ-heterocycloalkyl, (CH₂)ₜ-aryl, or ((CH₂)ₜ-heteroaryl, wherein said (CH₂)ₜ-cycloalkyl, (CH₂)ₜ-heterocycloalkyl, (CH₂)ₜ-aryl, or (CH₂)ₜ-heteroaryl are optionally substituted with alkyl, halo, or cyano;
R₉ is hydrogen or alkyl;
n is an integer selected from 1, 2 and 3;
p is an integer selected from 0 and 1, provided that if p is1, then n is 2 or 3;
q is an integer selected from 0 and 1, provided that if p is 0, then q is 0;
each t is an integer independently selected from 0, 1, 2 and 3;
or pharmaceutically acceptable salts thereof.

In one embodiment of the invention, n is 1.

In another embodiment of the invention, n is 2.

In another embodiment of the invention, n is 3.

In one embodiment of the invention, p is 0.

In one embodiment of the invention, R₁ is (CH₂)ₜ-heterocycloalkyl wherein t is zero and the heterocycloalkyl is a heterocycloalkyl containing a nitrogen, wherein the nitrogen is directly bonded to A, and wherein the heterocycloalkyl is optionally independently substituted by cyano, alkyl, halogen, or OR₆. In one example of this embodiment, the heterocycloalkyl is pyrrolidinyl, piperidinyl, or morpholinyl, and is optionally independently substituted by cyano, alkyl, halogen, or OR₆.

In another embodiment of the invention, R₁ is COR₈ wherein R₈ is (CH₂)ₜ-heterocycloalkyl and t is zero and the heterocycloalkyl contains a nitrogen wherein the nitrogen is directly bonded to the carbonyl carbon of COR₈ and said heterocycloalkyl is optionally substituted by alkyl, halo, or cyano. In one example of this embodiment, the heterocycloalkyl is pyrrolidinyl, piperidinyl, or morpholinyl, and is optionally substituted by alkyl, halo, or cyano.

In another embodiment of the invention, R₁ is SO₂R₈ wherein R₈ is (CH₂)ₜ-heterocycloalkyl and t is zero and the heterocycloalkyl contains a nitrogen wherein the nitrogen is directly bonded to the sulfonyl sulfur of SO₂R₈ and said heterocycloalkyl is optionally substituted by alkyl, halo, or cyano. In one example of this embodiment, the heterocycloalkyl is pyrrolidinyl, piperidinyl, or morpholinyl, and is optionally substituted by alkyl, halo, or cyano.

In one embodiment of the invention, A is aryl, heteroaryl, cycloalkyl or heterocycloalkyl, and A is optionally substituted with one R₁ substituent. In one example of this embodiment, R₁ is independently alkyl, halogen, cyano, SO₂NHR₉, CON(R₈)₂, N(R₈)COR₈, SO₂N(R₈)₂, N(R₈)SO₂R₈, COR₈, SO₂R₈, (CH₂)ₜ-cycloalkyl, (CH₂)ₜ-heterocycloalkyl, (CH₂)ₜ-aryl, (CH₂)ₜ-heteroaryl, (CH₂)ₜ-N(R₈)₂, or (CH₂)ₜ-OR₆ wherein each R₁ alkyl, (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆. In another example of this embodiment, R₁ is halogen, alkyl, OR₆, cyano, trifluoroalkyl, (CH₂)ₜ-cycloalkyl, (CH₂)ₜ-heterocycloalkyl, (CH₂)ₜ-aryl, or (CH₂)ₜ-heteroaryl, wherein each R₁ (CH₂)ₜ-cycloalkyl, (CH₂)ₜ-heterocycloalkyl, (CH₂)ₜ-aryl, or (CH₂)ₜ-heteroaryl, is optionally independently substituted by OR₆, alkyl, cyano, or halogen. In another example of this embodiment, R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In another example of this embodiment, R₁ is (CH₂)ₜ-heterocycloalkyl wherein t is zero and the heterocycloalkyl is pyrrolidinyl, piperidinyl, or morpholinyl, and is optionally substituted by cyano, alkyl, halogen, or OR₆. In yet another example of this embodiment, R₁ is COR₈ wherein R₈ is (CH₂)ₜ-heterocycloalkyl and t is zero and the heterocycloalkyl contains a nitrogen wherein the nitrogen is directly bonded to the carbonyl carbon of COR₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano. In another example of this embodiment, R₁ is SO₂R₈ wherein R₈ is -(CH₂)ₜ-heterocycloalkyl and t is zero and the heterocycloalkyl contains a nitrogen wherein the nitrogen is directly bonded to the sulfonyl sulfur of SO₂R₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano.

In another embodiment of the invention, A is aryl and is optionally substituted with one R₁ substituent. In an example of this embodiment, R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In another embodiment of the invention, A is heteroaryl, and is optionally substituted with one R₁ substituent. In an example of this embodiment, R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆.

In another embodiment of the invention A is aryl, heteroaryl, cycloalkyl or heterocycloalkyl, and A is optionally substituted with two R₁ substituents. In one example of this embodiment, each R₁ is independently alkyl, halogen, cyano, SO₂NHR₉, CON(R₈)₂, N(R₈)COR₈, SO₂N(R₈)₂, N(R₈)SO₂R₈, COR₈, SO₂R₈, (CH₂)ₜ-cycloalkyl, (CH₂)ₜ-heterocycloalkyl, (CH₂)ₜ-aryl, (CH₂)ₜ-heteroaryl, (CH₂)ₜ-N(R₈)₂, or (CH₂)ₜ-OR₆ wherein each R₁ alkyl, (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆. In another example of this embodiment, each R₁ is independently alkyl, halogen, cyano, -N(R₈)COR₈, -COR₈, -(CH₂)ₜ-cycloalkyl, (CH₂)ₜ-heterocycloalkyl, -(CH₂)ₜ-aryl, -(CH₂)ₜ-heteroaryl, (CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In another example of this embodiment, each R₁ is alkyl optionally independently substituted by cyano, alkyl, halogen, or OR₆. In another example of this embodiment, each R₁ is independently alkyl, halogen, (CH₂)ₜ-cycloalkyl, (CH₂)ₜ-heterocycloalkyl, (CH₂)ₜ-aryl, or (CH₂)ₜ-heteroaryl, wherein each R₁ (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆. In another example of this embodiments, at least one R₁ is (CH₂)ₜ-heterocycloalkyl wherein t is zero and the heterocycloalkyl is pyrrolidinyl, piperidinyl, or morpholinyl, and is optionally substituted by cyano, alkyl, halogen, or OR₆. In another example of this embodiment, at least one R₁ is COR₈ wherein R₈ is (CH₂)ₜ-heterocycloalkyl and t is zero and the heterocycloalkyl contains a nitrogen wherein the nitrogen is directly bonded to the carbonyl carbon of COR₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano. In another example of this embodiment, at least one R₁ is SO₂R₈ wherein R₈ is (CH₂)ₜ-heterocycloalkyl and t is zero and the heterocycloalkyl contains a nitrogen wherein the nitrogen is directly bonded to the sulfonyl sulfur of SO₂R₈ and said heterocycloalkyl is optionally substituted by alkyl, halo, or cyano. In an example of this particular embodiment, the heterocycolalkyl is pyrrolidinyl, piperidinyl, or morpholinyl, and is optionally substituted by alkyl, halo, or cyano.

In another embodiment of the invention, A is aryl and is optionally substituted with two R₁ substituents. In an example of this embodiment, each R₁ is independently alkyl, halogen, cyano, -N(R₈)COR₈, -COR₈, (CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or (CH₂)ₜ-OR₆ wherein each R₁ alkyl, (CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In one embodiment of the invention, one R₁ is alkyl, halogen, or -(CH₂)ₜ-OR₆, and the other R₁ is independently -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆.

In another embodiment of the invention, A is heteroaryl, and is optionally substituted with two R₁ substituents. In an example of this embodiment, each R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In one embodiment of the invention, one R₁ is alkyl, halogen, or -(CH₂)ₜ-OR₆, and the other R₁ is independently -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆.

In another embodiment of the invention, A is aryl, heteroaryl, cycloalkyl or heterocycloalkyl, and A is optionally substituted with three R₁ substituents. In one example of this embodiment, each R₁ is independently alkyl, halogen, cyano, SO₂NHR₉, CON(R₈)₂, N(R₈)COR₈, SO₂N(R₈)₂, N(R₈)SO₂R₈, COR₈, SO₂R₈, (CH₂)ₜ-cycloalkyl, (CH₂)ₜ-heterocycloalkyl, (CH₂)ₜ-raryl, (CH₂)ₜ-heteroaryl, (CH₂)ₜ-N(R₈)₂, or (CH₂)ₜ-OR₆ wherein each R₁ alkyl, (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆. In another example of this embodiment, each R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)ₜ-N(R₈)₂, or -(CH₂)ₜ-OR₆ wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆. In another example of this embodiment, each R₁ is alkyl optionally independently substituted by cyano, alkyl, halogen, or OR₆. In another example of this embodiment, each R₁ is independently halogen, OR₆, cyano, trifluoroalkyl, (CH₂)ₜ-cycloalkyl, (CH₂)ₜ-heterocycloalkyl, (CH₂)ₜ-aryl, or (CH₂)ₜ-heteroaryl, wherein each R₁ (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆. In another example of this embodiment, at least one R₁ is (CH₂)ₜ-heterocycloalkyl wherein t is zero and the heterocycloalkyl is pyrrolidinyl, piperidinyl, or morpholinyl, and is optionally independently substituted by cyano, alkyl, halogen, or OR₆. In yet another example of this embodiment, at least one R₁ is COR₈ wherein R₈ is (CH₂)ₜ-heterocycloalkyl and t is zero and the heterocycloalkyl contains a nitrogen wherein the nitrogen is directly bonded to the carbonyl carbon of COR₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano. In another example of this embodiment, at least one R₁ is SO₂R₈ wherein R₈ is (CH₂)ₜ-heterocycloalkyl and t is zero and the heterocycloalkyl contains a nitrogen wherein the nitrogen is directly bonded to the sulfonyl sulfur of SO₂R₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano.

In one embodiment of the invention, B is aryl. In one example of this embodiment, B is substituted with only one R₄ substituent and R₄ is halogen. In another embodiment of this example, B is substituted with two R₄ substituents and each R₄ is halogen.

In another embodiment of the invention, Z is a cycloalkylene moiety.

In another embodiment of the invention, Z is (CH₂)ₙ-Oₚ-(CH₂)_{q}. In one example of this embodiment, p is 0.

In one embodiment of the invention, R₂ is alkyl.

In one embodiment of the invention, R₂ is alkyl optionally substituted with halogen.

In another embodiment of the invention, R_{3A} and R_{3B} are each independently hydrogen or alkyl. In one example of this embodiment, R_{3A} and R_{3B} are each hydrogen.

In another embodiment of the invention, R_{3A} and R_{3B} together with the carbon they are bonded to form a C=O, C=NR₈, a cycloalkylene moiety or a heterocycloalkylene moiety. In one example of this embodiment, R_{3A} and R_{3B} together with the carbon they are bonded to form a C=O. In another example of this embodiment, R_{3A} and R_{3B} together with the carbon they are bonded to form a C=NR₈. In yet another example of this embodiment, R_{3A} and R_{3B} together with the carbon they are bonded to form a cycloalkylene moiety or a heterocycloalkylene moiety.

In one embodiment of the invention, R₄ is COR₈ wherein R₈ is (CH₂)ₜ-heterocycloalkyl and t is zero and the heterocycloalkyl contains a nitrogen wherein the nitrogen is directly bonded to the carbonyl carbon of COR₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano.

In another embodiment of the invention, R₄ is SO₂R₈ wherein R₈ is (CH₂)ₜ-heterocycloalkyl and t is zero and the heterocycloalkyl contains a nitrogen wherein the nitrogen is directly bonded to the sulfonyl sulfur of SO₂R₈ and said heterocycloalkyl is optionally substituted by alkyl, halo, or cyano. In an example of this particular embodiment, the heterocycloalkyl is pyrrolidinyl, piperidinyl, or morpholinyl, and is optionally substituted by alkyl, halo, or cyano.

In yet another embodiment of the invention, R₄ is independently selected from the group consisting of halogen and alkyl.

In one embodiment of the invention, each R₅ is independently alkyl, alkenyl, (CH₂)ₜ-cycloalkyl, (CH₂)ₜ-heterocycloalkyl, (CH₂)ₜ-aryl, or (CH₂)ₜ-heteroaryl and R₅ is substituted with COR₈ wherein R₈ is (CH₂)ₜ-heterocycloalkyl, t is zero, and the heterocycloalkyl contains a nitrogen wherein the nitrogen is directly bonded to the carbonyl carbon of COR₈ and wherein the heterocycloalkyl is optionally substituted by alkyl, halo, or cyano.

In another embodiment of the invention, each R₅ is independently alkyl, alkenyl, (CH₂)ₜ-cycloalkyl, (CH₂)ₜ-heterocycloalkyl, (CH₂)ₜ-aryl, or (CH₂)ₜ-heteroaryl and R₅ is substituted with SO₂R₈, wherein R₈ is (CH₂)ₜ-heterocycloalkyl, t is zero, and the heterocycloalkyl contains a nitrogen wherein the nitrogen is directly bonded to the sulfonyl sulfur of SO₂R₈, and wherein the heterocycloalkyl is optionally substituted by alkyl, halo, or cyano.

In another embodiment of the invention, R₅ is (CH₂)ₜ-heterocycloalkyl wherein t is zero and the heterocycloalkyl is pyrrolidinyl, piperidinyl, or morpholinyl, and is optionally substituted with one or more hydroxyl, aryl, heteroaryl, halogen, alkyl, cycloalkyl, SO₂R₈,-NR₈COR₈, -CON(R₈)₂, COOR₈, - C(O)R₈, -CN, N(R₈)₂, wherein said aryl, alkyl, cycloalkyl and heteroaryl substituent is optionally substituted with one or more halogen, alkyl, hydroxyl, or -O-alkyl.

In yet another embodiment of the invention, R₅ is independently selected from the group consisting of hydrogen and alkyl.

In another embodiment of the invention,
B is aryl;
B is substituted by only one R₄ and R₄ is halogen;
R₂ is alkyl;
Z is (CH₂)ₙ-Oₚ-(CH₂)_{q} wherein n is 1, p is 0;
A is aryl substituted with one R₁ wherein R₁ is OR₆ and R₆ is alkyl; and
R₅, R_{3A} and R_{3B} are each independently H.

In another embodiment of the invention,
B is aryl;
B is substituted by only one R₄ and R₄ is halogen;
R₂ is alkyl;
Z is (CH₂)ₙ-Oₚ-(CH₂)_{q} wherein n is 1, p is 0;
A is aryl substituted with one R₁ wherein R₁ is (CH₂)ₜ-heteroaryl and t is 0;
said R₁ (CH₂)ₜ-heteroaryl is substituted with alkyl; and
R₅, R_{3A} and R_{3B} are each independently H.

In another embodiment of the invention,
B is aryl;
B is substituted by only one R₄ and R₄ is halogen;
R₂ is alkyl;
Z is (CH₂)ₙ-Oₚ-(CH₂)_{q} wherein n is 1, p is 0;
A is aryl substituted with one R₁ wherein R₁ is heteroaryl;
R₅ is alkyl; and
R_{3A} and R_{3B} are each independently H.

In another embodiment of the invention,
B is aryl;
B is substituted by only one R₄ and R₄ is halogen;
R₂ is alkyl;
Z is (CH₂)ₙ-Oₚ-(CH₂)_{q} wherein n is 1, p is 0,;
A is heteroaryl substituted with one R₁ wherein R₁ is OR₆ and R₆ is alkyl; and
R₅, R_{3A} and R_{3B} are each independently H.

Exemplary compounds according to the invention include the compounds:
(5R,7S)-1-(3-fluorophenyl)-8-(3-isopropoxybenzyl)-7-methyl-2-thia-1,3,8-triazaspiro[4.5]decane 2,2-dioxide;
(5R,7S)-1-(3-fluorophenyl)-8-[(6-isopropoxypyridin-2-yl)methyl]-7-methyl-2-thia-1,3,8-triazaspiro[4.5]decane 2,2-dioxide;
(5R,7S)-1-(3-fluorophenyl)-7-methyl-8-[3-(4-methylpyridin-3-yl)benzyl]-2-thia-1,3,8-triazaspiro[4.5]decane 2,2-dioxide;
(5R,7S)-1-(3-fluorophenyl)-8-(3-isopropoxybenzyl)-3,7-dimethyl-2-thia-1,3,8-triazaspiro[4.5]decane 2,2-dioxide;
(5R,7S)-1-(3-fluorophenyl)-8-(3-isopropoxybenzyl)-7-methyl-2-thia-1,3,8-triazaspiro[4.5]decane 2,2-dioxide;
(5*R*,7*S*)-1-(3-fluorophenyl)-8-(3-isopropoxybenzyl)-7-methyl-3-(pyridin-2-ylmethyl)-2-thia-1,3,8-triazaspiro[4.5]decane 2,2-dioxide;
5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol;
(5*R*,7*S*)-1-(3-fluorophenyl)-7-methyl-8-[3-(4-methylpyridin-3-yl)benzyl]-2-thia-1,3,8-triazaspiro[4.5]decane 2,2-dioxide;
2-chloro-4{[(5*R*,7*S*)-1-(3-fluorophenyl)-7-methyl-2,2-dioxido-2-thia-1,3,8-triazaspiro[4.5]dec-8-yl]methyl}phenol;
(5*R*,7*S*)-1-(3-fluorophenyl)-7-methyl-8-[(2'-methylbiphenyl-3-yl)methyl]-2-thia-1,3,8-triazaspiro[4.5]decane 2,2-dioxide;
(5*R*,7*S*)-8-[(2'-chlorobiphenyl-3-yl)methyl]-1-(3-fluorophenyl)-7-methyl-2-thia-1,3,8-triazaspiro[4.5]decane 2,2-dioxide;
2-ethoxy-4-{[(5*R*,7*S*)-1-(3-fluorophenyl)-7-methyl-2,2-dioxido-2-thia-1,3,8-triazaspiro[4.5]dec-8-yl]methyl}phenol;
(5*R*,7*S*)-8-[(2'-ethylbiphenyl-3-yl)methyl]-1-(3-fluorophenyl)-7-methyl-2-thia-1,3,8-triazaspiro[4.5]decane 2,2-dioxide;
(5*R*,7*S*)-1-(3-fluorophenyl)-7-methyl-8-(3-{[(1R)-1-methylpropy]oxy}benzyl)-2-thia-1,3,8-triazaspiro[4.5]decane 2,2-dioxide;
4-{[(5*R*,7*S*)-1-(3-fluorophenyl)-3,7-dimethyl-2,2-dioxido-2-thia-1,3,8-triazaspiro[4.5]dec-8-yl]methyl}-2-isopropoxyphenol;
(5*R*,7*S*)-1-(3-fluorophenyl)-3,7-dimethyl-8-[(2'-methylbiphenyl-3-yl)methyl]-2-thia-1,3,8-triazaspiro[4.5]decane 2,2-dioxide;
(5*R*,7*S*)-8-{[4-(cyclopropylmethyl)-1,3-thiazol-5-yl]methyl}-1-(3-fluorophenyl)-3,7-dimethyl-2-thia-1,3,8-triazaspiro[4.5]decane 2,2-dioxide;
(5*R*,7*S*)-1-(3-fluorophenyl)-8-[(4-isobutyl-1,3-thiazol-5-yl)methyl]-3,7-dimethyl-2-thia-1,3,8-triazaspiro[4.5]decane 2,2-dioxide;
(5*R*,7*S*)-8-{[4-(cyclobutylmethyl)-1,3-thiazol-5-yl]methyl}-1-(3-fluorophenyl)3,7-dimethyl-2-thia-1,3,8-triazaspiro[4.5]decane 2,2-dioxide;
(5*R*,7*S*)1-(3-fluorophenyl)-3,7-dimethyl-8-{3-[(2-methyl-1,3-benzoxazol-6-yl)oxy]benzyl}-2-thia-1,3,8-triazaspiro[4.5]decane 2,2-dioxide;
(5*R*,7*S*)-8-[(3-ethyl-1H-indazol-5-yl)methyl]-1-(3-fluorophenyl)-3,7-dimethyl-2-thia-1,3,8-triazaspiro[4.5]decane 2,2-dioxide;
(5*R*,7*S*)-1-(3-fluorophenyl)-3,7-dimethyl-8-{[3-(trifluoromethyl)-1H-indazol-5-yl]methyl}-2-thia-1,3,8-triazaspiro[4.5]decane 2,2-dioxide;
2'-ethyl-5-{[(5*R*,7*S*)1-(3-fluorophenyl)-3,7-dimethyl-2,2-dioxido-2-thia-1,3,8-triazaspiro[4.5]dec-8-yl]methyl}biphenyl-2-ol;
2'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol;
5'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol;
4'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol;
2'-chloro-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol;
6-{[(5*R*,7*S*)-1-(3-fluorophenyl)-3,7-dimethyl-2,2-dioxido-2-thia-1,3,8-triazaspiro[4.5]dec-8-yl]methyl}-4-isopropoxypyridin-3-ol;
2-cyclopentyl-4-{[(5*R*,7*S*)-1-(3-fluorophenyl)-3,7-dimethyl-2,2-dioxido-2-thia-1,3,8-triazaspiro[4.5]dec-8-yl]methyl}phenol;
4-{[(5*R*,7*S*)-1-(3-fluorophenyl)-3,7-dimethyl-2,2-dioxido-2-thia-1,3,8-triazaspiro[4.5]dec-8-yl]methyl}-2-isobutylphenol;
2-cyclohexyl-4-{[(5*R*,7*S*)-1-(3-fluorophenyl)-3,7-dimethyl-2,2-dioxido-2-thia-1,3,8-triazaspiro[4.5]dec-8-yl]methyl}phenol;
(5*R*,7*S*)-1-(3-fluorophenyl)-8-(3-isopropoxybenzyl)-7-methyl-3-pyrimidin-2-yl-2-thia-1,3,8-triazaspiro[4.5]decane 2,2-dioxide;
(5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide;
(5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide; and
(5*R*,7*S*)-1-(3-fluorophenyl)-8-(3-isopropoxybenzyl)-3-(isoxazol-3-ylmethyl)-7-methyl-2-thia-1,3,8-triazaspiro[4.5]decane 2,2-dioxide; or pharmaceutically acceptable salts thereof.

In one embodiment, the invention also relates to each of the individual compounds described as Examples 1 - 131 in the Examples section of the subject application, (including the free bases or pharmaceutically acceptable salts thereof).

In another embodiment the invention relates to a compound selected from the group consisting of:
4-(cyclopropyloxy)-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
4-(cyclopropyloxy)-2-{[(5R,7S)-1-(3-fluorophenyl)-3,7-dimethyl-2,2-dioxido-2-thia-1,3,8-triazaspiro[4.5]dec-8-yl]methyl}pyrimidin-5-ol;
2-(cyclopropyloxy)-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
6-{[(5R,7S)-1-(3-fluorophenyl)-3,7-dimethyl-2,2-dioxido-2-thia-1,3,8-triazaspiro[4.5]dec-8-yl]methyl}-4-(trifluoromethoxy)pyridin-3-ol;
6-{[(5R,7S)-1-(3-fluorophenyl)-3,7-dimethyl-2,2-dioxido-2-thia-1,3,8-triazaspiro[4.5]dec-8-yl]methyl}-4-(trifluoromethyl)pyridin-3-ol;
6-{[(5R,7S)-1-(3-fluorophenyl)-3,7-dimethyl-2,2-dioxido-2-thia-1,3,8-triazaspiro[4.5]dec-8-yl]methyl}-4-(2-methylphenyl)pyridin-3-ol;
4-(5-chloro-2-thienyl)-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
2-{[(5R,7S)-1-(3-fluorophenyl)-3,7-dimethyl-2,2-dioxido-2-thia-1,3,8-triazaspiro[4.5]dec-8-yl]methyl}-4-(trifluoromethyl)pyrimidin-5-ol;
6-{[(5R,7S)-1-(3-fluorophenyl)-3,7-dimethyl-2,2-dioxido-2-thia-1,3,8-triazaspiro[4.5]dec-8-yl]methyl}-2-(trifluoromethyl)pyridin-3-ol;
2-{[(5R,7S)-1-(3-fluorophenyl)-3,7-dimethyl-2,2-dioxido-2-thia-1,3,8-triazaspiro[4.5]dec-8-yl]methyl}-4-(trifluoromethoxy)pyrimidin-5-ol;
6-{[(5R,7S)-1-(3-fluorophenyl)-3,7-dimethyl-2,2-dioxido-2-thia-1,3,8-triazaspiro[4.5]dec-8-yl]methyl}-2-(trifluoromethoxy)pyridin-3-ol;
4-{[(5R,7S)-1-(3-fluorophenyl)-3-isoxazol-4-yl-7-methyl-2,2-dioxido-2-thia-1,3,8-triazaspiro[4.5]dec-8-yl]methyl}-2-isopropoxyphenol;
4-{[(5R,7S)-1-(3-fluorophenyl)-7-methyl-3-(1,3-oxazol-2-yl)-2,2-dioxido-2-thia-1,3,8-triazaspiro[4.5]dec-8-yl]methyl}-2-isopropoxyphenol;
4-{[(5R,7S)-1-(3-fluorophenyl)-7-methyl-2,2-dioxido-3-(1,3-thiazol-2-yl)-2-thia-1,3,8-triazaspiro[4.5]dec-8-yl]methyl}-2-isopropoxyphenol;
6'-{[(5R,7S)-1-(3-fluorophenyl)-3,7-dimethyl-2,2-dioxido-2-thia-1,3,8-triazaspiro[4.5]dec-8-yl]methyl}-3-methyl-2,4'-bipyridin-3'-ol;
6-{[(5R,7S)-1-(3-fluorophenyl)-3,7-dimethyl-2,2-dioxido-2-thia-1,3,8-triazaspiro[4.5]dec-8-yl]methyl}-4-(3-methyl-2-thienyl)pyridin-3-ol;
4-(2-fluorophenyl)-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
4-(2-chlorophenyl)-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
6'-{[(5R,7S)-1-(3-fluorophenyl)-3,7-dimethyl-2,2-dioxido-2-thia-1,3,8-triazaspiro[4.5]dec-8-yl]methyl}-4-methyl-3,4'-bipyridin-3'-ol;
4-cyclobutyl-6-{[(5R,7S)-1-(3-fluorophenyl)-3,7-dimethyl-2,2-dioxido-2-thia-1,3,8-triazaspiro[4.5]dec-8-yl]methyl}pyridin-3-ol;
6-{[(5R,7S)-1-(3-fluorophenyl)-3,7-dimethyl-2,2-dioxido-2-thia-1,3,8-triazaspiro[4.5]dec-8-yl]methyl}-4-oxetan-3-ylpyridin-3-ol;
4-{[(5R,7S)-1-(3,4-difluorophenyl)-3,7-dimethyl-2,2-dioxido-2-thia-1,3,8 triazaspiro[4.5]dec-8-yl]methyl}-2-isopropoxyphenol;
4-fluoro-2-[(5R,7S)-8-(4-hydroxy-3-isopropoxybenzyl)-3,7-dimethyl-2,2-dioxido-2-thia-1,3,8-triazaspiro[4.5]dec-1-yl]benzonitrile;
5-fluoro-3-[(5R,7S)-8-(4-hydroxy-3-isopropoxybenzyl)-3,7-dimethyl-2,2-dioxido-2-thia-1,3,8-triazaspiro[4.5]dec-1-yl]pyridine-2-carbonitrile;
4-{[(5R,7S)-1-(5-fluoro-6-methylpyridin-3-yl)-3,7-dimethyl-2,2-dioxido-2-thia-1, 3,8-triazaspiro[4.5]dec-8-yl]methyl}-2-isopropoxyphenol;
4-{[(5R,7S)-1-(1,3-dimethyl-1H-pyrazol-4-yl)-3,7-dimethyl-2,2-dioxido-2-thia-1,3,8-triazaspiro[4.5]dec-8-yl]methyl}-2-isopropoxyphenol;
4-{[(5R,7S)-1-(3-ethylisoxazol-4-yl)-3,7-dimethyl-2,2-dioxido-2-thia-1,3,8-triazaspiro[4.5]dec-8-yl]methyl}-2-isopropoxyphenol;
4-{[(5R,7S)-3,7-dimethyl-1-(5-methyl-1,3-thiazol-4-yl)-2,2-dioxido-2-thia-1,3,8-triazaspiro[4.5]dec-8-yl]methyl}-2-isopropoxyphenol;
N-cyclobutyl-5-{[(5R,7S)-1-(3-fluorophenyl)-3,7-dimethyl-2,2-dioxido-2-thia-1,3,8-triazaspiro[4.5]dec-8-yl]methyl}-1,3-thiazol-4-amine;
(5R,7S)-8-{[4-(cyclobutyloxy)-1,3-thiazol-5-yl]methyl}-1-(3-fluorophenyl)-3,7-dimethyl-2-thia-1,3,8-triazaspiro[4.5]decane 2,2-dioxide;
(5R,7S)-1-(3-fluorophenyl)-3,7-dimethyl-8-{[4-(oxetan-3-ylmethyl)-1,3-thiazol-5-yl]methyl}-2-thia-1,3,8-triazaspiro[4.5]decane 2,2-dioxide;
4-{[(5R,7S)-{5-fluoro-2-[(2,2,2-trifluoroethyl)amino]pyridin-3-yl}-3,7-dimethyl-2,2-dioxido-2-thia-1,3,8-triazaspiro[4.5]dec-8-yl]methyl}-2-isopropoxyphenol;
3-{(5R,7S)-3,7-dimethyl-8-[(2-methylcyclobutyl)methyl]-2,2-dioxido-2-thia-1,3,8-triazaspiro[4.5]dec-1-yl}-5-fluoropyridine-2-carbonitrile;
5-fluoro-3-{(5R,7S)-8-[(2-isopropoxycyclobutyl)methyl]-3,7-dimethyl-2,2-dioxido-2-thia-1,3,8-triazaspiro[4.5]dec-1-yl}pyridine-2-carbonitrile; and
(5R,7S)-1-(3-fluoro-4-methylphenyl)-8-[(isopropoxycyclobutyl)methyl]-3,7-dimethyl-2-thia-1,3,8-triazaspiro[4.5]decane 2,2-dioxide, or pharmaceutically acceptable salts thereof.

Also disclosed are methods of treating neurological and psychiatric disorders comprising: administering to a patient in need thereof an amount of a compound of formula I effective in treating such disorders. Neurological and psychiatric disorders, include but are not limited to: acute neurological and psychiatric disorders such as cerebral deficits subsequent to cardiac bypass surgery and grafting, stroke, cerebral ischemia, spinal cord trauma, head trauma, perinatal hypoxia, cardiac arrest, hypoglycemic neuronal damage, dementia, AIDS-induced dementia, vascular dementia, mixed dementias, age associated memory impairment, Alzheimer's disease, Huntington's Chorea, amyotrophic lateral sclerosis, ocular damage, retinopathy, cognitive disorders, including cognitive disorders associated with schizophrenia and bipolar disorders, idiopathic and drug-induced Parkinson's disease, muscular spasms and disorders associated with muscular spasticity including tremors, epilepsy, convulsions, migraine, migraine headache, urinary incontinence, substance tolerance, substance withdrawal, withdrawal from opiates, nicotine, tobacco products, alcohol, benzodiazepines, cocaine, sedatives, and hypnotics, psychosis, mild cognitive impairment, amnestic cognitive impairment, multi-domain cognitive impairment, obesity, schizophrenia, anxiety, generalized anxiety disorder, social anxiety disorder, panic disorder, post-traumatic stress disorder, obsessive compulsive disorder, mood disorders, depression, mania, bipolar disorders, trigeminal neuralgia, hearing loss, tinnitus, macular degeneration of the eye, emesis, brain edema, pain, acute and chronic pain states, severe pain, intractable pain, neuropathic pain, post-traumatic pain, tardive dyskinesia, sleep disorders, narcolepsy, attention deficit/hyperactivity disorder, autism, Asperger's disease, and conduct disorder in a mammal, comprising administering to the mammal an effective amount of compound of formula I or pharmaceutically acceptable salt thereof. Accordingly, in one embodiment, the invention provides a method for treating a condition in a mammal, such as a human, selected from the conditions above, comprising administering a compound of formula I to the mammal. The mammal is preferably a mammal in need of such treatment. As examples, the invention provides a method for treating attention deficit/hyperactivity disorder, schizophrenia and Alzheimer's Disease.

Also disclosed are methods of treating neurological and psychiatric disorders comprising: administering to a patient in need thereof an amount of a compound of formula I effective in treating such disorders. The compound of formula I is optionally used in combination with another active agent. Such an active agent may be, for example, an atypical antipsychotic, a cholinesterase inhibitor, or NMDA receptor antagonist. Such atypical antipsychotics include, but are not limited to, ziprasidone, clozapine, olanzapine, risperidone, quetiapine, aripiprazole, paliperidone; such NMDA receptor antagonists include but are not limited to memantine; and such cholinesterase inhibitors include but are not limited to donepezil and galantamine. Additional active agents may be a P-glycoprotein inhibitor (Pgp-Inhibitor), a Multidrug Resistance Inhibitor (MDR-Inhibitor), or a cytochrome P450 inhibitor (CYP inhibitor) including more specifically cytochrome P450 3A4 inhibitor.

The invention is also directed to a pharmaceutical composition comprising a compound of formula I, and a pharmaceutically acceptable carrier. The composition may be, for example, a composition for treating a condition selected from the group consisting of neurological and psychiatric disorders, including but not limited to: acute neurological and psychiatric disorders such as cerebral deficits subsequent to cardiac bypass surgery and grafting, stroke, cerebral ischemia, spinal cord trauma, head trauma, perinatal hypoxia, cardiac arrest, hypoglycemic neuronal damage, dementia, AIDS-induced dementia, vascular dementia, mixed dementias, age associated memory impairment, Alzheimer's disease, Huntington's Chorea, amyotrophic lateral sclerosis, ocular damage, retinopathy, cognitive disorders, including cognitive disorders associated with schizophrenia and bipolar disorders, idiopathic and drug-induced Parkinson's disease, muscular spasms and disorders associated with muscular spasticity including tremors, epilepsy, convulsions, migraine, migraine headache, urinary incontinence, substance tolerance, substance withdrawal, withdrawal from opiates, nicotine, tobacco products, alcohol, benzodiazepines, cocaine, sedatives, and hypnotics, psychosis, mild cognitive impairment, amnestic cognitive impairment, multi-domain cognitive impairment, obesity, schizophrenia, anxiety, generalized anxiety disorder, social anxiety disorder, panic disorder, post-traumatic stress disorder, obsessive compulsive disorder, mood disorders, depression, mania, bipolar disorders, trigeminal neuralgia, hearing loss, tinnitus, macular degeneration of the eye, emesis, brain edema, pain, acute and chronic pain states, severe pain, intractable pain, neuropathic pain, post-traumatic pain, tardive dyskinesia, sleep disorders, narcolepsy, attention deficit/hyperactivity disorder, autism, Asperger's disease, and conduct disorder in a mammal, comprising administering an effective amount of compound of formula I or pharmaceutically acceptable salt thereof, and a pharmaceutically acceptable carrier. The composition optionally further comprises an atypical antipsychotic, a cholinesterase inhibitor, or NMDA receptor antagonist. Such atypical antipsychotics include, but are not limited to, ziprasidone, clozapine, olanzapine, risperidone, quetiapine, aripiprazole, paliperidone; such NMDA receptor antagonists include but are not limited to memantine; and such cholinesterase inhibitors include but are not limited to donepezil and galantamine.

### Abbreviations and Definitions

**TABLE A - Abbreviations**

| Ac | Acetyl |
|---|---|
| APCI | Atmospheric pressure chemical ionization (in mass spectrometry) |
| Boc | *tert*-Butoxycarbonyl |
| br | Broad |
| CD₃OD | Deuterated methanol |
| CDCl₃ | Deuterated chloroform |
| d | Doublet |
| dba | Dibenzylidene acetone |
| DCM | Dichloromethane |
| DMF | *N,N*-Dimethylformamide |
| dd | Doublet of doublets |
| DMSO | Dimethyl sulfoxide |
| ES | Electrospray Ionization (in mass spectrometry) |
| Et₃N | Triethylamine |
| EtOAc | Ethyl acetate |
| g | Gram(s) |
| h | Hour(s) |
| HPLC | High performance liquid chromatography |
| J | Coupling constant (in NMR) |
| LCMS | Liquid Chromatography - Mass Spectrometry |
| LDA | Lithium diisopropylamide |
| LRMS (ES+) | Low Resolution Mass Spectrometry (electrospray or thermospray ionization positive scan) |
| LRMS (ES⁻) | Low Resolution Mass Spectrometry (electrospray ionization negative scan) |
| m | Multiplet (spectral), meters(s), milli |
| m/z | Mass to charge ratio (in mass spectrometry) |
| MeOH | Methanol |
| MHz | Megahertz |
| MS | Mass spectrometry |
| NMR | Nuclear Magnetic Resonance |
| ppm | Parts per million (in NMR) |
| psi | Pounds per square inch |
| q | Quartet |
| s | Singlet |
| t | Triplet |
| Tf | Trifluoromethanesulfonyl (triflyl) |
| TFA | Trifluoroacetic acid |
| THF | Tetrahydrofuran |
| TLC | Thin layer chromatography |
| TMHD | 2,2,6,6-Tetramethyl-3,5-heptanedione |
| Vol. | Volume |
| δ | Chemical shift |

The term "alkyl" refers to a linear or branched-chain saturated hydrocarbyl substituent (i.e., a substituent obtained from a hydrocarbon by removal of a hydrogen) containing from one to twenty carbon atoms; in one embodiment from one to twelve carbon atoms; in another embodiment, from one to ten carbon atoms; in another embodiment, from one to six carbon atoms; and in another embodiment, from one to four carbon atoms. Examples of such substituents include methyl, ethyl, propyl (including n-propyl and isopropyl), butyl (including n-butyl, isobutyl, sec-butyl and tert-butyl), pentyl, iso-amyl, hexyl and the like.

The term "benzyl" refers to methyl radical substituted with phenyl, i.e., the following structure:

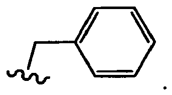

The term "cycloalkyl" refers to a carbocyclic substituent obtained by removing a hydrogen from a saturated carbocyclic molecule and having three to fourteen carbon atoms. In one embodiment, a cycloalkyl substituent has three to ten carbon atoms. Examples of cycloalkyl include cyclopropyl, cyclobutyl, cyclopentyl and cyclohexyl.

The term "cycloalkylene moiety" refers to a carbocyclic substituent obtained by removing two hydrogen atoms from a saturated carbocyclic molecule and having three to fourteen carbon atoms. In one embodiment, a cycloalkylene substituent has three to ten carbon atoms. Examples of cycloalkylene include the following:

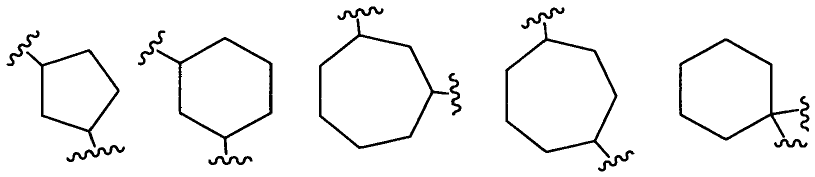

The term "cycloalkyl" also includes substituents that are fused to a C₆-C₁₀ aromatic ring or to a 5-10-membered heteroaromatic ring, wherein a group having such a fused cycloalkyl group as a substituent is bound to a carbon atom of the cycloalkyl group. When such a fused cycloalkyl group is substituted with one or more substituents, the one or more substituents, unless otherwise specified, are each bound to a carbon atom of the cycloalkyl group. The fused C₆-C₁₀ aromatic ring or 5-10-membered heteroaromatic ring may be optionally substituted with halogen, C₁-C₆ alkyl, C₃-C₁₀ cycloalkyl, or =O.

A cycloalkyl may be a single ring, which typically contains from 3 to 6 ring atoms. Examples include cyclopropyl, cyclobutyl, cyclopentyl, and cyclohexyl. Alternatively, 2 or 3 rings may be fused together, such as bicyclodecanyl and decalinyl.

The term "aryl" refers to an aromatic substituent containing one ring or two or three fused rings. The aryl substituent may have six to eighteen carbon atoms. As an example, the aryl substituent may have six to fourteen carbon atoms. The term "aryl" may refer to substituents such as phenyl, naphthyl and anthracenyl. The term "aryl" also includes substituents such as phenyl, naphthyl and anthracenyl that are fused to a C₄-C₁₀ carbocyclic ring, such as a C₅ or a C₆ carbocyclic ring, or to a 4-10 membered heterocyclic ring, wherein a group having such a fused aryl group as a substituent is bound to an aromatic carbon of the aryl group. When such a fused aryl group is substituted with one more substituents, the one or more substituents, unless otherwise specified, are each bound to an aromatic carbon of the fused aryl group. The fused C₄-C₁₀ carbocyclic or 4-10 membered heterocyclic ring may be optionally substituted with halogen, C₁-C₆ alkyl, C₃-C₁₀ cycloalkyl, or =O. Examples of aryl groups include accordingly phenyl, naphthalenyl, tetrahydronaphthalenyl (also known as "tetralinyl"), indenyl, isoindenyl, indanyl, anthracenyl, phenanthrenyl, benzonaphthenyl (also known as "phenalenyl"), and fluorenyl.

In some instances, the number of carbon atoms in a hydrocarbyl substituent (i.e., alkyl, alkenyl, cycloalkyl, cycloalkenyl, aryl, etc.) is indicated by the prefix "Cₓ-C_{y}," wherein x is the minimum and y is the maximum number of carbon atoms in the substituent. Thus, for example, "C₁-C₆ alkyl" refers to an alkyl substituent containing from 1 to 6 carbon atoms. Illustrating further, C₃-C₆ cycloalkyl refers to saturated cycloalkyl containing from 3 to 6 carbon ring atoms.

In some instances, the number of atoms in a cyclic substituent containing one or more heteroatoms (i.e., heteroaryl or heterocycloalkyl) is indicated by the prefix "X-Y membered", wherein wherein x is the minimum and y is the maximum number of atoms forming the cyclic moiety of the substituent. Thus, for example, 5-8 membered heterocycloalkyl refers to a heterocycloalkyl containing from 5 to 8 atoms, including one or more heteroatoms, in the cyclic moiety of the heterocycloalkyl.

The term "hydrogen" refers to hydrogen substituent, and may be depicted as -H.

The term "hydroxy" or "hydroxyl" refers to -OH. When used in combination with another term(s), the prefix "hydroxy" indicates that the substituent to which the prefix is attached is substituted with one or more hydroxy substituents. Compounds bearing a carbon to which a hydroxy substituent is attached include, for example, alcohols, enols and phenol.

The term "hydroxyalkyl" refers to an alkyl that is substituted with at least one hydroxy substituent. Examples of hydroxyalkyl include hydroxymethyl, hydroxyethyl, hydroxypropyl and hydroxybutyl.

The term "cyano" (also referred to as "nitrile") means -CN, which also may be depicted:

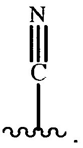

The term "carbonyl" means -C(O)-, which also may be depicted as:

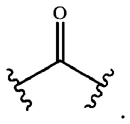

The term "amino" refers to -NH₂.

The term "alkylamino" refers to an amino group, wherein at least one alkyl chain is bonded to the amino nitrogen in place of a hydrogen atom. Examples of alkylamino substituents include monoalkylamino such as methylamino (exemplified by the formula -NH(CH₃)), which may also be depicted: 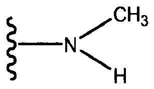 and dialkylamino such as dimethylamino, (exemplified by the formula -N(CH₃)₂, which may also be depicted:

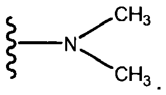

The term "halogen" refers to fluorine (which may be depicted as -F), chlorine (which may be depicted as -Cl), bromine (which may be depicted as -Br), or iodine (which may be depicted as -I). In one embodiment, the halogen is chlorine. In another embodiment, the halogen is a fluorine.

The prefix "halo" indicates that the substituent to which the prefix is attached is substituted with one or more independently selected halogen substituents. For example, haloalkyl refers to an alkyl that is substituted with at least one halogen substituent. Where more than one hydrogen is replaced with halogens, the halogens may be the identical or different. Examples of haloalkyls include chloromethyl, dichloromethyl, difluorochloromethyl, dichlorofluoromethyl, trichloromethyl, 1-bromoethyl, fluoromethyl, difluoromethyl, trifluoromethyl, 2,2,2-trifluoroethyl, difluoroethyl, pentafluoroethyl, difluoropropyl, dichloropropyl, and heptafluoropropyl. Illustrating further, "haloalkoxy" refers to an alkoxy that is substituted with at least one halogen substituent. Examples of haloalkoxy substituents include chloromethoxy, 1-bromoethoxy, fluoromethoxy, difluoromethoxy, trifluoromethoxy (also known as "perfluoromethyloxy"), and 2,2,2-trifluoroethoxy. It should be recognized that if a substituent is substituted by more than one halogen substituent, those halogen substituents may be identical or different (unless otherwise stated).

The term "oxo" refers to =O.

The term "oxy" refers to an ether substituent, and may be depicted as -O-.

The term "alkoxy" refers to an alkyl linked to an oxygen, which may also be represented as
-O-R, wherein the R represents the alkyl group. Examples of alkoxy include methoxy, ethoxy, propoxy and butoxy.

The term "heterocycloalkyl" refers to a substituent obtained by removing a hydrogen from a saturated or partially saturated ring structure containing a total of 3 to 14 ring atoms. At least one of the ring atoms is a heteroatom (i.e., oxygen, nitrogen, or sulfur), with the remaining ring atoms being independently selected from the group consisting of carbon, oxygen, nitrogen, and sulfur. A heterocycloalkyl alternatively may comprise 2 or 3 rings fused together, wherein at least one such ring contains a heteroatom as a ring atom (i.e., nitrogen, oxygen, or sulfur). In a group that has a heterocycloalkyl substituent, the ring atom of the heterocycloalkyl substituent that is bound to the group may be the at least one heteroatom, or it may be a ring carbon atom, where the ring carbon atom may be in the same ring as the at least one heteroatom or where the ring carbon atom may be in a different ring from the at least one heteroatom. Similarly, if the heterocycloalkyl substituent is in turn substituted with a group or substituent, the group or substituent may be bound to the at least one heteroatom, or it may be bound to a ring carbon atom, where the ring carbon atom may be in the same ring as the at least one heteroatom or where the ring carbon atom may be in a different ring from the at least one heteroatom.

The term "heterocycloalkyl" also includes substituents that are fused to a C₆-C₁₀ aromatic ring or to a 5-10-membered heteroaromatic ring, wherein a group having such a fused heterocycloalkyl group as a substituent is bound to a heteroatom of the heterocycloalkyl group or to a carbon atom of the heterocycloalkyl group. When such a fused heterocycloalkyl group is substituted with one or more substituents, the one or more substituents, unless otherwise specified, are each bound to a heteroatom of the heterocycloalkyl group or to a carbon atom of the heterocycloalkyl group. The fused C₆-C₁₀ aromatic ring or 5-10-membered heteroaromatic ring may be optionally substituted with halogen, C₁-C₆ alkyl, C₃-C₁₀ cycloalkyl, C₁-C₆ alkoxy, or =O.

The term "heterocycloalkylene moiety" refers to a substituent obtained by removing two hydrogen atoms from a saturated or partially saturated ring structure containing a total of 3 to 14 ring atoms, where at least one of the ring atoms is a heteroatom. In one embodiment, a heterocycloalkylene substituent has three to ten ring atoms. Examples of heterocycloalkylene include the following:

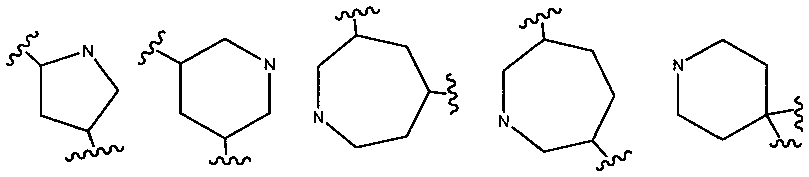

The term "heteroaryl" refers to an aromatic ring structure containing from 5 to 14 ring atoms in which at least one of the ring atoms is a heteroatom (i.e., oxygen, nitrogen, or sulfur), with the remaining ring atoms being independently selected from the group consisting of carbon, oxygen, nitrogen, and sulfur. A heteroaryl may be a single ring or 2 or 3 fused rings. Examples of heteroaryl substituents include 6-membered ring substituents such as pyridyl, pyrazyl, pyrimidinyl, and pyridazinyl; 5-membered ring substituents such as triazolyl, imidazolyl, furanyl, thiophenyl, pyrazolyl, oxazolyl, isoxazolyl, thiazolyl, 1,2,3-, 1,2,4-, 1,2,5-, or 1,3,4-oxadiazolyl and isothiazolyl; 6/5-membered fused ring substituents such as benzothiofuranyl, isobenzothiofuranyl, benzisoxazolyl, benzoxazolyl, purinyl, and anthranilyl; and 6/6-membered fused rings such as quinolinyl, isoquinolinyl, cinnolinyl, quinazolinyl, and 1,4-benzoxazinyl. In a group that has a heteroaryl substituent, the ring atom of the heteroaryl substituent that is bound to the group may be the at least one heteroatom, or it may be a ring carbon atom, where the ring carbon atom may be in the same ring as the at least one heteroatom or where the ring carbon atom may be in a different ring from the at least one heteroatom. Similarly, if the heteroaryl substituent is in turn substituted with a group or substituent, the group or substituent may be bound to the at least one heteroatom, or it may be bound to a ring carbon atom, where the ring carbon atom may be in the same ring as the at least one heteroatom or where the ring carbon atom may be in a different ring from the at least one heteroatom. The term "heteroaryl" also includes pyridyl N-oxides and groups containing a pyridine N-oxide ring.

Examples of single-ring heteroaryls and heterocycloalkyls include furanyl, dihydrofuranyl, tetradydrofuranyl, thiophenyl (also known as "thiofuranyl"), dihydrothiophenyl, tetrahydrothiophenyl, pyrrolyl, isopyrrolyl, pyrrolinyl, pyrrolidinyl, imidazolyl, isoimidazolyl, imidazolinyl, imidazolidinyl, pyrazolyl, pyrazolinyl, pyrazolidinyl, triazolyl, tetrazolyl, dithiolyl, oxathiolyl, oxazolyl, isoxazolyl, thiazolyl, isothiazolyl, thiazolinyl, isothiazolinyl, thiazolidinyl, isothiazolidinyl, thiadiazolyl, oxathiazolyl, oxadiazolyl (including oxadiazolyl, 1,2,4-oxadiazolyl (also known as "azoximyl"), 1,2,5-oxadiazolyl (also known as "furazanyl"), or 1,3,4-oxadiazolyl), oxatriazolyl (including 1,2,3,4-oxatriazolyl or 1,2,3,5-oxatriazolyl), dioxazolyl (including 1,2,3-dioxazolyl, 1,2,4-dioxazolyl, 1,3,2-dioxazolyl, or 1,3,4-dioxazolyl), oxathiazolyl, oxathiolyl, oxathiolanyl, pyranyl (including 1,2-pyranyl or 1,4-pyranyl), dihydropyranyl, pyridinyl (also known as "azinyl"), piperidinyl, diazinyl (including pyridazinyl (also known as "1,2-diazinyl"), pyrimidinyl (also known as "1,3-diazinyl" or "pyrimidyl"), or pyrazinyl (also known as "1,4-diazinyl")), piperazinyl, triazinyl (including s-triazinyl (also known as "1,3,5-triazinyl"), as-triazinyl (also known 1,2,4-triazinyl), and v-triazinyl (also known as "1,2,3-triazinyl")), oxazinyl (including 1,2,3-oxazinyl, 1,3,2-oxazinyl, 1,3,6-oxazinyl (also known as "pentoxazolyl"), 1,2,6-oxazinyl, or 1,4-oxazinyl), isoxazinyl (including o-isoxazinyl or p-isoxazinyl), oxazolidinyl, isoxazolidinyl, oxathiazinyl (including 1,2,5-oxathiazinyl or 1,2,6-oxathiazinyl), oxadiazinyl (including 1,4,2-oxadiazinyl or 1,3,5,2-oxadiazinyl), morpholinyl, azepinyl, oxepinyl, thiepinyl, and diazepinyl.

Examples of 2-fused-ring heteroaryls and heterocycloalkyls include, indolizinyl, pyrindinyl, pyranopyrrolyl, 4H-quinolizinyl, purinyl, naphthyridinyl, pyridopyridinyl (including pyrido[3,4-b]-pyridinyl, pyrido[3,2-b]-pyridinyl, or pyrido[4,3-b]-pyridinyl), and pteridinyl, indolyl, isoindolyl, indoleninyl, isoindazolyl, benzazinyl, phthalazinyl, quinoxalinyl, quinazolinyl, benzodiazinyl, benzopyranyl, benzothiopyranyl, benzoxazolyl, indoxazinyl, anthranilyl, benzodioxolyl, benzodioxanyl, benzoxadiazolyl, benzofuranyl, isobenzofuranyl, benzothienyl, isobenzothienyl, benzothiazolyl, benzothiadiazolyl, benzimidazolyl, benzotriazolyl, benzoxazinyl, benzisoxazinyl, and tetrahydroisoquinolinyl.

Examples of 3-fused-ring heteroaryls or heterocycloalkyls include 5,6-dihydro-4H-imidazo[4,5,1-ij]quinoline, 4,5-dihydroimidazo[4,5,1-hi]indole, 4,5,6,7-tetrahydroimidazo[4,5,1-jk][1]benzazepine, and dibenzofuranyl.

Other examples of fused-ring heteroaryls or heterocycloalkyls include benzo-fused heteroaryls such as indolyl, isoindolyl (also known as "isobenzazolyl" or "pseudoisoindolyl"), indoleninyl (also known as "pseudoindolyl"), isoindazolyl (also known as "benzpyrazolyl"), benzazinyl (including quinolinyl (also known as "1-benzazinyl") or isoquinolinyl (also known as "2-benzazinyl")), phthalazinyl, quinoxalinyl, quinazolinyl, benzodiazinyl (including cinnolinyl (also known as "1,2-benzodiazinyl") or quinazolinyl (also known as "1,3-benzodiazinyl")), benzopyranyl (including "chromanyl" or "isochromanyl"), benzothiopyranyl (also known as "thiochromanyl"), benzoxazolyl, indoxazinyl (also known as "benzisoxazolyl"), anthranilyl, benzodioxolyl, benzodioxanyl, benzoxadiazolyl, benzofuranyl (also known as "coumaronyl"), isobenzofuranyl, benzothienyl (also known as "benzothiophenyl," "thionaphthenyl," or "benzothiofuranyl"), isobenzothienyl (also known as "isobenzothiophenyl," "isothionaphthenyl," or "isobenzothiofuranyl"), benzothiazolyl, benzothiadiazolyl, benzimidazolyl, benzotriazolyl, benzoxazinyl (including 1,3,2-benzoxazinyl, 1,4,2-benzoxazinyl, 2,3,1-benzoxazinyl, or 3,1,4-benzoxazinyl), benzisoxazinyl (including 1,2-benzisoxazinyl or 1,4-benzisoxazinyl), tetrahydroisoquinolinyl , carbazolyl, xanthenyl, and acridinyl.

The term "heteroaryl" also includes substituents such as pyridyl and quinolinyl that are fused to a C₄-C₁₀ carbocyclic ring, such as a C₅ or a C₆ carbocyclic ring, or to a 4-10-membered heterocyclic ring, wherein a group having such a fused aryl group as a substituent is bound to an aromatic carbon of the heteroaryl group or to a heteroatom of the heteroaryl group. When such a fused heteroaryl group is substituted with one or more substituents, the one or more substitutents, unless otherwise specified, are each bound to an aromatic carbon of the heteroaryl group or to a heteroatom of the heteroaryl group. The fused C₄-C₁₀ carbocyclic or 4-10-membered heterocyclic ring may be optionally substituted with halogen, C₁-C₆ alkyl, C₃-C₁₀ cycloalkyl, or =O.

Additional examples of heteroaryls and heterocycloalkyls include: 3-1 H-benzimidazol-2-one, (1-substituted)-2-oxo-benzimidazol-3-yl, 2-tetrahydrofuranyl, 3-tetrahydrofuranyl, 2-tetrahydropyranyl, 3-tetrahydropyranyl, 4-tetrahydropyranyl, [1,3]-dioxalanyl, [1,3]-dithiolanyl, [1,3]-dioxanyl, 2- tetrahydrothiophenyl, 3-tetrahydrothiophenyl, 2-morpholinyl, 3-morpholinyl, 4-morpholinyl, 2-thiomorpholinyl, 3-thiomorpholinyl, 4-thiomorpholinyl, 1-pyrrolidinyl, 2-pyrrolidinyl, 3-pyrrolidinyl, 1-piperazinyl, 2-piperazinyl, 1-piperidinyl, 2-piperidinyl, 3-piperidinyl, 4-piperidinyl, 4-thiazolidinyl, diazolonyl, N-substituted diazolonyl, 1-phthalimidinyl, benzoxanyl, benzo[1,3]dioxine, benzo[1,4]dioxine, benzopyrrolidinyl, benzopiperidinyl, benzoxolanyl, benzothiolanyl, 4,5,6,7-tetrahydropyrazol[1,5-alpha]pyridine, benzothianyl, pyrrolidinyl, tetrahydrofuranyl, dihydrofuranyl, tetrahydrothienyl, tetrahydropyranyl, dihydropyranyl, tetrahydrothiopyranyl, piperidino, morpholino, thiomorpholino, thioxanyl, piperazinyl, azetidinyl, oxetanyl, thietanyl, homopiperidinyl, oxepanyl, thiepanyl, oxazepinyl, diazepinyl, thiazepinyl, 1,2,3,6-tetrahydropyridinyl, 2-pyrrolinyl, 3-pyrrolinyl, indolinyl, 2H-pyranyl, 4H-pyranyl, dioxanyl, 1,3-dioxolanyl, pyrazolinyl, dithianyl, dithiolanyl, dihydropyranyl, dihydrothienyl, dihydrofuranyl, pyrazolidinyl, imidazolinyl, imidazolidinyl, 3-azabicyclo[3.1.0]hexanyl, 3-azabicyclo[4.1.0]heptanyl, 3H-indolyl, quinolizinyl, pyridinyl, imidazolyl, pyrimidinyl, pyrazolyl, triazolyl, pyrazinyl, tetrazolyl, furyl, thienyl, isoxazolyl, thiazolyl, oxazolyl, isothiazolyl, pyrrolyl, quinolinyl, isoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, thiadiazolyl, furazanyl, benzofurazanyl, benzothiophenyl, benzothiazolyl, benzoxazolyl, quinazolinyl, quinoxalinyl, naphthyridinyl, and furopyridinyl. The foregoing groups, as derived from the groups listed above, may be C-attached or N-attached where such is possible. For instance, a group derived from pyrrole may be pyrrol-1-yl (N-attached) or pyrrol-3-yl (C-attached). Further, a group derived from imidazole may be imidazol-1-yl (N-attached) or imidazol-2-yl (C-attached).

A substituent is "substitutable" if it comprises at least one carbon, sulfur, oxygen or nitrogen atom that is bonded to one or more hydrogen atoms. Thus, for example, hydrogen, halogen, and cyano do not fall within this definition.

If a substituent is described as being "substituted," a non-hydrogen substituent is in the place of a hydrogen substituent on a carbon, oxygen, sulfur or nitrogen of the substituent. Thus, for example, a substituted alkyl substituent is an alkyl substituent wherein at least one non-hydrogen substituent is in the place of a hydrogen substituent on the alkyl substituent. To illustrate, monofluoroalkyl is alkyl substituted with a fluoro substituent, and difluoroalkyl is alkyl substituted with two fluoro substituents. It should be recognized that if there is more than one substitution on a substituent, each non-hydrogen substituent may be identical or different (unless otherwise stated).

If a substituent is described as being "optionally substituted," the substituent may be either (1) not substituted, or (2) substituted. If a carbon of a substituent is described as being optionally substituted with one or more of a list of substituents, one or more of the hydrogens on the carbon (to the extent there are any) may separately and/or together be replaced with an independently selected optional substituent. If a nitrogen of a substituent is described as being optionally substituted with one or more of a list of substituents, one or more of the hydrogens on the nitrogen (to the extent there are any) may each be replaced with an independently selected optional substituent. One exemplary substituent may be depicted as -NR'R," wherein R' and R" together with the nitrogen atom to which they are attached, may form a heterocyclic ring. The heterocyclic ring formed from R' and R" together with the nitrogen atom to which they are attached may be partially or fully saturated. In one embodiment, the heterocyclic ring consists of 4 to 7 atoms. In another embodiment, the heterocyclic ring is selected from the group consisting of pyrrolyl, imidazolyl, pyrazolyl, triazolyl, tetrazolyl, isoxazolyl, pyridyl and thiazolyl.

This specification uses the terms "substituent," "radical," and "group" interchangeably.

If a group of substituents are collectively described as being optionally substituted by one or more of a list of substituents, the group may include: (1) unsubstitutable substituents, (2) substitutable substituents that are not substituted by the optional substituents, and/or (3) substitutable substituents that are substituted by one or more of the optional substituents.

If a substituent is described as being optionally substituted with up to a particular number of non-hydrogen substituents, that substituent may be either (1) not substituted; or (2) substituted by up to that particular number of non-hydrogen substituents or by up to the maximum number of substitutable positions on the substituent, whichever is less. Thus, for example, if a substituent is described as a heteroaryl optionally substituted with up to 3 non-hydrogen substituents, then any heteroaryl with less than 3 substitutable positions would be optionally substituted by up to only as many non-hydrogen substituents as the heteroaryl has substitutable positions. To illustrate, tetrazolyl (which has only one substitutable position) would be optionally substituted with up to one non-hydrogen substituent. To illustrate further, if an amino nitrogen is described as being optionally substituted with up to 2 non-hydrogen substituents, then the nitrogen will be optionally substituted with up to 2 non-hydrogen substituents if the amino nitrogen is a primary nitrogen, whereas the amino nitrogen will be optionally substituted with up to only 1 non-hydrogen substituent if the amino nitrogen is a secondary nitrogen.

A prefix attached to a multi-moiety substituent only applies to the first moiety. To illustrate, the term "alkylcycloalkyl" contains two moieties: alkyl and cycloalkyl. Thus, a C₁-C₆ prefix on C₁-C₆ alkylcycloalkyl means that the alkyl moiety of the alkylcycloalkyl contains from 1 to 6 carbon atoms; the C₁-C₆ prefix does not describe the cycloalkyl moiety. To illustrate further, the prefix "halo" on haloalkoxyalkyl indicates that *only* the alkoxy moiety of the alkoxyalkyl substituent is substituted with one or more halogen substituents. If the halogen substitution may *only* occur on the alkyl moiety, the substituent would be described as "alkoxyhaloalkyl." If the halogen substitution may occur on both the alkyl moiety and the alkoxy moeity, the substituent would be described as "haloalkoxyhaloalkyl."

When a substituent is comprised of multiple moieties, unless otherwise indicated, it is the intention for the final moiety to serve as the point of attachment to the remainder of the molecule. For example, in a substituent A-B-C, moiety C is attached to the remainder of the molecule. In a substituent A-B-C-D, moiety D is attached to the remainder of the molecule. Similarly, in a substituent aminocarbonylmethyl, the methyl moiety is attached to the remainder of the molecule, where the substituent may also be be depicted as 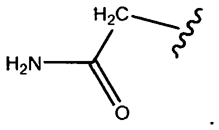 In a substituent trifluoromethylaminocarbonyl, the carbonyl moiety is attached to the remainder of the molecule, where the substituent may also be depicted as

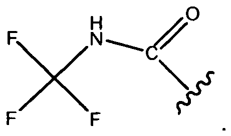

If substituents are described as being "independently selected" from a group, each substituent is selected independent of the other. Each substituent therefore may be identical to or different from the other substituent(s).

### Isomers

When an asymmetric center is present in a compound of formula I, hereinafter referred to as the compound of the invention, the compound may exist in the form of optical isomers (enantiomers). In one embodiment, the present invention comprises enantiomers and mixtures, including racemic mixtures of the compounds of formula I. In another embodiment, for compounds of formulae I that contain more than one asymmetric center, the present invention comprises diastereomeric forms (individual diastereomers and mixtures thereof) of compounds. When a compound of formula I contains an alkenyl group or moiety, geometric isomers may arise.

### Tautomeric Forms

The present invention comprises the tautomeric forms of compounds of formula I. Where structural isomers are interconvertible *via* a low energy barrier, tautomeric isomerism ('tautomerism') can occur. This can take the form of proton tautomerism in compounds of formula I containing, for example, an imino, keto, or oxime group, or so-called valence tautomerism in compounds which contain an aromatic moiety. It follows that a single compound may exhibit more than one type of isomerism. The various ratios of the tautomers in solid and liquid form is dependent on the various substituents on the molecule as well as the particular crystallization technique used to isolate a compound.

### Salts

The compounds of this invention may be used in the form of salts derived from inorganic or organic acids. Depending on the particular compound, a salt of the compound may be advantageous due to one or more of the salt's physical properties, such as enhanced pharmaceutical stability in differing temperatures and humidities, or a desirable solubility in water or oil. In some instances, a salt of a compound also may be used as an aid in the isolation, purification, and/or resolution of the compound.

Where a salt is intended to be administered to a patient (as opposed to, for example, being used in an in vitro context), the salt preferably is pharmaceutically acceptable. The term "pharmaceutically acceptable salt" refers to a salt prepared by combining a compound of formula I with an acid whose anion, or a base whose cation, is generally considered suitable for human consumption. Pharmaceutically acceptable salts are particularly useful as products of the methods of the present invention because of their greater aqueous solubility relative to the parent compound. For use in medicine, the salts of the compounds of this invention are non-toxic "pharmaceutically acceptable salts." Salts encompassed within the term "pharmaceutically acceptable salts" refer to non-toxic salts of the compounds of this invention which are generally prepared by reacting the free base with a suitable organic or inorganic acid.

Suitable pharmaceutically acceptable acid addition salts of the compounds of the present invention when possible include those derived from inorganic acids, such as hydrochloric, hydrobromic, hydrofluoric, boric, fluoroboric, phosphoric, metaphosphoric, nitric, carbonic, sulfonic, and sulfuric acids, and organic acids such as acetic, benzenesulfonic, benzoic, citric, ethanesulfonic, fumaric, gluconic, glycolic, isothionic, lactic, lactobionic, maleic, malic, methanesulfonic, trifluoromethanesulfonic, succinic, toluenesulfonic, tartaric, and trifluoroacetic acids. Suitable organic acids generally include, for example, aliphatic, cycloaliphatic, aromatic, araliphatic, heterocyclic, carboxylic, and sulfonic classes of organic acids.

Specific examples of suitable organic acids include acetate, trifluoroacetate, formate, propionate, succinate, glycolate, gluconate, digluconate, lactate, malate, tartaric acid, citrate, ascorbate, glucuronate, maleate, fumarate, pyruvate, aspartate, glutamate, benzoate, anthranilic acid, mesylate, stearate, salicylate, p-hydroxybenzoate, phenylacetate, mandelate, embonate (pamoate), methanesulfonate, ethanesulfonate, benzenesulfonate, pantothenate, toluenesulfonate, 2-hydroxyethanesulfonate, sulfanilate, cyclohexylaminosulfonate, algenic acid, β-hydroxybutyric acid, galactarate, galacturonate, adipate, alginate, butyrate, camphorate, camphorsulfonate, cyclopentanepropionate, dodecylsulfate, glycoheptanoate, glycerophosphate, heptanoate, hexanoate, nicotinate, 2-naphthalenesulfonate, oxalate, palmoate, pectinate, 3-phenylpropionate, picrate, pivalate, thiocyanate, tosylate, and undecanoate.

Furthermore, where the compounds of the invention carry an acidic moiety, suitable pharmaceutically acceptable salts thereof may include alkali metal salts, i.e., sodium or potassium salts; alkaline earth metal salts, e.g., calcium or magnesium salts; and salts formed with suitable organic ligands, e.g., quaternary ammonium salts. In another embodiment, base salts are formed from bases which form non-toxic salts, including aluminum, arginine, benzathine, choline, diethylamine, diolamine, glycine, lysine, meglumine, olamine, tromethamine and zinc salts.

Organic salts may be made from secondary, tertiary or quaternary amine salts, such as tromethamine, diethylamine, N,N'-dibenzylethylenediamine, chloroprocaine, choline, diethanolamine, ethylenediamine, meglumine (N-methylglucamine), and procaine. Basic nitrogen-containing groups may be quaternized with agents such as lower alkyl (C₁-C₆) halides (e.g., methyl, ethyl, propyl, and butyl chlorides, bromides, and iodides), dialkyl sulfates (i.e., dimethyl, diethyl, dibutyl, and diamyl sulfates), long chain halides (i.e., decyl, lauryl, myristyl, and stearyl chlorides, bromides, and iodides), arylalkyl halides (i.e., benzyl and phenethyl bromides), and others.

In one embodiment, hemisalts of acids and bases may also be formed, for example, hemisulphate and hemicalcium salts.

### Prodrugs

Also disclosed are so-called "prodrugs" of the compound of the invention. Thus, certain derivatives of the compound of the invention which may have little or no pharmacological activity themselves can, when administered into or onto the body, be converted into the compound of the invention having the desired activity, for example, by hydrolytic cleavage. Such derivatives are referred to as "prodrugs." Further information on the use of prodrugs may be found in "Pro-drugs as Novel Delivery Systems, Vol. 14, ACS Symposium Series (T Higuchi and W Stella) and "Bioreversible Carriers in Drug Design," Pergamon Press, 1987 (ed. E B Roche, American Pharmaceutical Association). Prodrugs in accordance with the invention can, for example, be produced by replacing appropriate functionalities present in the compounds of any of formula I with certain moieties known to those skilled in the art as "pro-moieties" as described, for example, in "Design of Prodrugs" by H. Bundgaard (Elsevier, 1985).

### Isotopes

Also disclosed are labelled compounds, which are identical to those recited in formula I, but for the fact that one or more atoms are replaced by an atom having an atomic mass or mass number different from the atomic mass or mass number usually found in nature. Examples of isotopes that can be incorporated into compounds of the present invention include isotopes of hydrogen, carbon, nitrogen, oxygen, phosphorous, sulfur, fluorine and chlorine, such as ²H, ³H, ¹³C, ¹¹C, ¹⁴C, ¹⁵N, ¹⁸O, ¹⁷O, ³¹P, ³²P, ³⁵S, ¹⁸F, and ³⁶Cl, respectively. Compounds of the present invention, prodrugs thereof, and pharmaceutically acceptable salts of said compounds or of said prodrugs which contain the aforementioned isotopes and/or other isotopes of other atoms are within the scope of this invention. Certain isotopically labelled compounds of the present invention, for example those into which radioactive isotopes such as ³H and ¹⁴C are incorporated, are useful in drug and/or substrate tissue distribution assays. Tritiated, i.e., ³H, and carbon-14, i.e., ¹⁴C, isotopes are particularly preferred for their ease of preparation and detectability. Further, substitution with heavier isotopes such as deuterium, i.e., ²H, can afford certain therapeutic advantages resulting from greater metabolic stability, for example increased in vivo half-life or reduced dosage requirements and, hence, may be preferred in some circumstances. Isotopically labelled compounds of formula I of this invention and prodrugs thereof can generally be prepared by carrying out the procedures disclosed in the Schemes and/or in the Examples and Preparations below, by substituting a readily available isotopically labelled reagent for a non-isotopically labelled reagent.

### Administration and Dosing

Typically, a compound of the invention is administered in an amount effective to treat a condition as described herein. The compounds of the invention are administered by any suitable route in the form of a pharmaceutical composition adapted to such a route, and in a dose effective for the treatment intended. Therapeutically effective doses of the compounds required to treat the progress of the medical condition are readily ascertained by one of ordinary skill in the art using preclinical and clinical approaches familiar to the medicinal arts.

The compounds of the invention may be administered orally. Oral administration may involve swallowing, so that the compound enters the gastrointestinal tract, or buccal or sublingual administration may be employed by which the compound enters the blood stream directly from the mouth.

In another embodiment, the compounds of the invention may also be administered directly into the blood stream, into muscle, or into an internal organ. Suitable means for parenteral administration include intravenous, intraarterial, intraperitoneal, intrathecal, intraventricular, intraurethral, intrasternal, intracranial, intramuscular and subcutaneous. Suitable devices for parenteral administration include needle (including microneedle) injectors, needle-free injectors and infusion techniques.

In another embodiment, the compounds of the invention may also be administered topically to the skin or mucosa, that is, dermally or transdermally. In another embodiment, the compounds of the invention can also be administered intranasally or by inhalation. In another embodiment, the compounds of the invention may be administered rectally or vaginally. In another embodiment, the compounds of the invention may also be administered directly to the eye or ear.

The dosage regimen for the compounds and/or compositions containing the compounds is based on a variety of factors, including the type, age, weight, sex and medical condition of the patient; the severity of the condition; the route of administration; and the activity of the particular compound employed. Thus the dosage regimen may vary widely. Dosage levels of the order from about 0.01 mg to about 100 mg per kilogram of body weight per day are useful in the treatment of the above-indicated conditions. In one embodiment, the total daily dose of a compound of the invention (administered in single or divided doses) is typically from about 0.01 to about 100 mg/kg. In another embodiment, total daily dose of the compound of the invention is from about 0.1 to about 50 mg/kg, and in another embodiment, from about 0.5 to about 30 mg/kg (i.e., mg compound of the invention per kg body weight). In one embodiment, dosing is from 0.01 to 10 mg/kg/day. In another embodiment, dosing is from 0.1 to 1.0 mg/kg/day. Dosage unit compositions may contain such amounts or submultiples thereof to make up the daily dose. In many instances, the administration of the compound will be repeated a plurality of times in a day (typically no greater than 4 times). Multiple doses per day typically may be used to increase the total daily dose, if desired.

For oral administration, the compositions may be provided in the form of tablets containing 0.01, 0.05, 0.1, 0.5, 1.0, 2.5, 5.0, 10.0, 15.0, 25.0, 50.0, 75.0, 100, 125, 150, 175, 200, 250 and 500 milligrams of the active ingredient for the symptomatic adjustment of the dosage to the patient. A medicament typically contains from about 0.01 mg to about 500 mg of the active ingredient, or in another embodiment, from about 1 mg to about 100 mg of active ingredient. Intravenously, doses may range from about 0.1 to about 10 mg/kg/minute during a constant rate infusion.

Suitable subjects according to the present invention include mammalian subjects. Mammals according to the present invention include, but are not limited to, canine, feline, bovine, caprine, equine, ovine, porcine, rodents, lagomorphs, primates, and the like, and encompass mammals in utero. In one embodiment, humans are suitable subjects. Human subjects may be of either gender and at any stage of development.

### Use in the Preparation of a Medicament

In another embodiment, the invention comprises the use of one or more compounds of the invention for the preparation of a medicament for the treatment of the conditions recited herein.

### Pharmaceutical Compositions

For the treatment of the conditions referred to above, the compound of the invention can be administered as compound per se. Alternatively, pharmaceutically acceptable salts are suitable for medical applications because of their greater aqueous solubility relative to the parent compound.

In another embodiment, the present invention comprises pharmaceutical compositions. Such pharmaceutical compositions comprise a compound of the invention presented with a pharmaceutically-acceptable carrier. The carrier can be a solid, a liquid, or both, and may be formulated with the compound as a unit-dose composition, for example, a tablet, which can contain from 0.05% to 95% by weight of the active compounds. A compound of the invention may be coupled with suitable polymers as targetable drug carriers. Other pharmacologically active substances can also be present.

The compounds of the present invention may be administered by any suitable route, preferably in the form of a pharmaceutical composition adapted to such a route, and in a dose effective for the treatment intended. The active compounds and compositions, for example, may be administered orally, rectally, parenterally, or topically.

Oral administration of a solid dose form may be, for example, presented in discrete units, such as hard or soft capsules, pills, cachets, lozenges, or tablets, each containing a predetermined amount of at least one compound of the present invention. In another embodiment, the oral administration may be in a powder or granule form. In another embodiment, the oral dose form is sub-lingual, such as, for example, a lozenge. In such solid dosage forms, the compounds of formula I are ordinarily combined with one or more adjuvants. Such capsules or tablets may contain a controlled-release formulation. In the case of capsules, tablets, and pills, the dosage forms also may comprise buffering agents or may be prepared with enteric coatings.

In another embodiment, oral administration may be in a liquid dose form. Liquid dosage forms for oral administration include, for example, pharmaceutically acceptable emulsions, solutions, suspensions, syrups, and elixirs containing inert diluents commonly used in the art (i.e., water). Such compositions also may comprise adjuvants, such as wetting, emulsifying, suspending, flavoring (e.g., sweetening), and/or perfuming agents.

In another embodiment, the present invention comprises a parenteral dose form. "Parenteral administration" includes, for example, subcutaneous injections, intravenous injections, intraperitoneally, intramuscular injections, intrasternal injections, and infusion. Injectable preparations (i.e., sterile injectable aqueous or oleaginous suspensions) may be formulated according to the known art using suitable dispersing or wetting agents, and/or suspending agents.

In another embodiment, the present invention comprises a topical dose form. "Topical administration" includes, for example, transdermal administration, such as via transdermal patches or iontophoresis devices, intraocular administration, or intranasal or inhalation administration. Compositions for topical administration also include, for example, topical gels, sprays, ointments, and creams. A topical formulation may include a compound which enhances absorption or penetration of the active ingredient through the skin or other affected areas. When the compounds of this invention are administered by a transdermal device, administration will be accomplished using a patch either of the reservoir and porous membrane type or of a solid matrix variety. Typical formulations for this purpose include gels, hydrogels, lotions, solutions, creams, ointments, dusting powders, dressings, foams, films, skin patches, wafers, implants, sponges, fibres, bandages and microemulsions. Liposomes may also be used. Typical carriers include alcohol, water, mineral oil, liquid petrolatum, white petrolatum, glycerin, polyethylene glycol and propylene glycol. Penetration enhancers may be incorporated - see, for example, J Pharm Sci, 88 (10), 955-958, by Finnin and Morgan (October 1999).

Formulations suitable for topical administration to the eye include, for example, eye drops wherein the compound of this invention is dissolved or suspended in suitable carrier. A typical formulation suitable for ocular or aural administration may be in the form of drops of a micronised suspension or solution in isotonic, pH-adjusted, sterile saline. Other formulations suitable for ocular and aural administration include ointments, biodegradable (i.e., absorbable gel sponges, collagen) and non-biodegradable (i.e., silicone) implants, wafers, lenses and particulate or vesicular systems, such as niosomes or liposomes. A polymer such as crossed-linked polyacrylic acid, polyvinylalcohol, hyaluronic acid, a cellulosic polymer, for example, hydroxypropylmethylcellulose, hydroxyethylcellulose, or methyl cellulose, or a heteropolysaccharide polymer, for example, gelan gum, may be incorporated together with a preservative, such as benzalkonium chloride. Such formulations may also be delivered by iontophoresis.

For intranasal administration or administration by inhalation, the active compounds of the invention are conveniently delivered in the form of a solution or suspension from a pump spray container that is squeezed or pumped by the patient or as an aerosol spray presentation from a pressurized container or a nebulizer, with the use of a suitable propellant. Formulations suitable for intranasal administration are typically administered in the form of a dry powder (either alone, as a mixture, for example, in a dry blend with lactose, or as a mixed component particle, for example, mixed with phospholipids, such as phosphatidylcholine) from a dry powder inhaler or as an aerosol spray from a pressurised container, pump, spray, atomiser (preferably an atomiser using electrohydrodynamics to produce a fine mist), or nebuliser, with or without the use of a suitable propellant, such as 1,1,1,2-tetrafluoroethane or 1,1,1,2,3,3,3-heptafluoropropane. For intranasal use, the powder may comprise a bioadhesive agent, for example, chitosan or cyclodextrin.

In another embodiment, the present invention comprises a rectal dose form. Such rectal dose form may be in the form of, for example, a suppository. Cocoa butter is a traditional suppository base, but various alternatives may be used as appropriate.

Other carrier materials and modes of administration known in the pharmaceutical art may also be used. Pharmaceutical compositions of the invention may be prepared by any of the well-known techniques of pharmacy, such as effective formulation and administration procedures. The above considerations in regard to effective formulations and administration procedures are well known in the art and are described in standard textbooks. Formulation of drugs is discussed in, for example, Hoover, John E., Remington's Pharmaceutical Sciences, Mack Publishing Co., Easton, Pennsylvania, 1975; Liberman et al., Eds., Pharmaceutical Dosage Forms, Marcel Decker, New York, N.Y., 1980; and Kibbe et al., Eds., Handbook of Pharmaceutical Excipients (3rd Ed.), American Pharmaceutical Association, Washington, 1999.

### Co-administration

The compounds of the present invention can be used, alone or in combination with other therapeutic agents, in the treatment of various conditions or disease states. The compound(s) of the present invention and other therapeutic agent(s) may be may be administered simultaneously (either in the same dosage form or in separate dosage forms) or sequentially. An exemplary therapeutic agent may be, for example, a metabotropic glutamate receptor agonist.

The administration of two or more compounds "in combination" means that the two compounds are administered closely enough in time that the presence of one alters the biological effects of the other. The two or more compounds may be administered simultaneously, concurrently or sequentially. Additionally, simultaneous administration may be carried out by mixing the compounds prior to administration or by administering the compounds at the same point in time but at different anatomic sites or using different routes of administration.

The phrases "concurrent administration," "co-administration," "simultaneous administration," and "administered simultaneously" mean that the compounds are administered in combination.

### Kits

The present invention further comprises kits that are suitable for use in performing the methods of treatment described above. In one embodiment, the kit contains a first dosage form comprising one or more of the compounds of the present invention and a container for the dosage, in quantities sufficient to carry out the methods of the present invention.

In another embodiment, the kit of the present invention comprises one or more compounds of the invention.

### Intermediates

In another embodiment, the invention relates to the novel intermediates useful for preparing the compounds of the invention.

### General Synthetic Schemes

The compounds of the formula I may be prepared by the methods described below, together with synthetic methods known in the art of organic chemistry, or modifications and derivatisations that are familiar to those of ordinary skill in the art. The starting materials used herein are commercially available or may be prepared by routine methods known in the art (such as those methods disclosed in standard reference books such as the COMPENDIUM OF ORGANIC SYNTHETIC METHODS, Vol. I-VI (published by Wiley-Interscience)). Preferred methods include, but are not limited to, those described below.

During any of the following synthetic sequences it may be necessary and/or desirable to protect sensitive or reactive groups on any of the molecules concerned. This can be achieved by means of conventional protecting groups, such as those described in T. W. Greene, Protective Groups in Organic Chemistry, John Wiley & Sons, 1981; T. W. Greene and P. G. M. Wuts, Protective Groups in Organic Chemistry, John Wiley & Sons, 1991, and T. W. Greene and P. G. M. Wuts, Protective Groups in Organic Chemistry, John Wiley & Sons, 1999, which are hereby incorporated by reference.

Compounds of formula I, or their pharmaceutically acceptable salts, can be prepared according to the reaction Schemes discussed herein below. Unless otherwise indicated, the substituents in the Schemes are defined as above. Isolation and purification of the products is accomplished by standard procedures, which are known to a chemist of ordinary skill.

It will be understood by one skilled in the art that the various symbols, superscripts and subscripts used in the schemes, methods and examples are used for convenience of representation and/or to reflect the order in which they are introduced in the schemes, and are not intended to necessarily correspond to the symbols, superscripts or subscripts in the appended claims. The schemes are representative of methods useful in synthesizing the compounds of the present invention. They are not to constrain the scope of the invention in any way.

### Working Examples

The following illustrate the synthesis of various compounds of the present invention. Additional compounds within the scope of this invention may be prepared using the methods illustrated in these Examples, either alone or in combination with techniques generally known in the art.

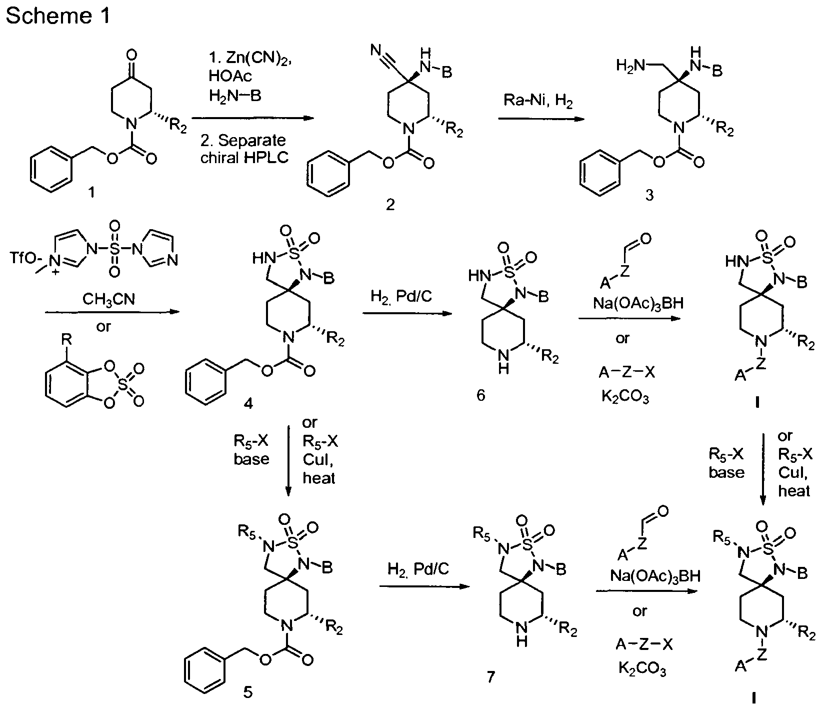

Scheme 1 illustrates the synthesis of cyclic sulfamide derivatives depicted by Formula I employing methods well known to one skilled in the art. The starting piperidinone is prepared following methods analogous to those described in the literature (Bioorganic & Medicinal Chemistry Letters, 2006, 16, 6241-6245) and separated using chiral HPLC. Referring to scheme 1, Strecker reaction of an appropriately protected piperidinone and an amine NH₂-B with zinc cyanide in acetic acid provides compound **2** after separation via chiral HPLC. Reduction of the nitrile group with Raney Nickel and a hydrogen source provides intermediate **3.** Formation of the cyclic sulfamide can be accomplished by treatment of intermediate **3** with an appropriate reagent such as 3-(imidazole-1-sulfonyl)-1-methyl-3*H*-imidazol-1-ium triflate (S. Beaudoin, J. Org Chem. 2003, 68, 115-119) in acetonitrile or sulfamide in pyridine or an appropriate catechol sulfate in DMF to provide **4.** Treatment of **4** with an appropriate alkyl halide and a base such as sodium hydride or cesium carbonate can provide intermediate **5;** alternatively, treatment of **4** with an aryl/heteroaryl halide or triflate, Cul, and heating provides intermediate **5.** Deprotection of **4** or **5** using hydrogenation with an appropriate catalyst such as palladium on carbon provides intermediate **6** or **7,** respectively. Reductive amination of **6** or **7** with an aldehyde and sodium triacetoxyborohydride or alkylation of **6** or **7** with A-Z-X (where X = I, Br, Cl, OTf) and base such as potassium or cesium carbonate provides compounds of formula **I.** Chiral compounds of formula **I** (R₅ defined above) can be prepared by alkylation of **1** (where R₅ = H) using methods known to one skilled in the art such as NaH in DMF with an appropriate group R₅-X where X is defined as above or treatment with an aryl/heteroaryl halide or triflate, Cul, and heating, to provide compounds of formula I (R₅ defined above).

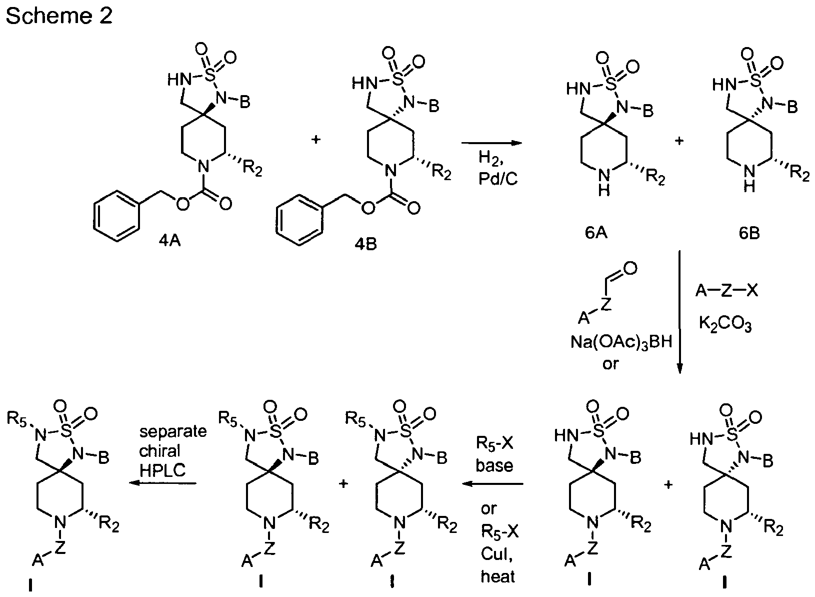

Scheme 2 illustrates the synthesis of cyclic sulfamide derivatives depicted by formula **I** employing methods well known to one skilled in the art. Intermediates **4A** and **4B** are prepared in similar manner to that described in Scheme 1 where chiral separation is conducted at a different step. Chiral compounds depicted by formula I are prepared in an analogous manner to those described in Scheme 1.

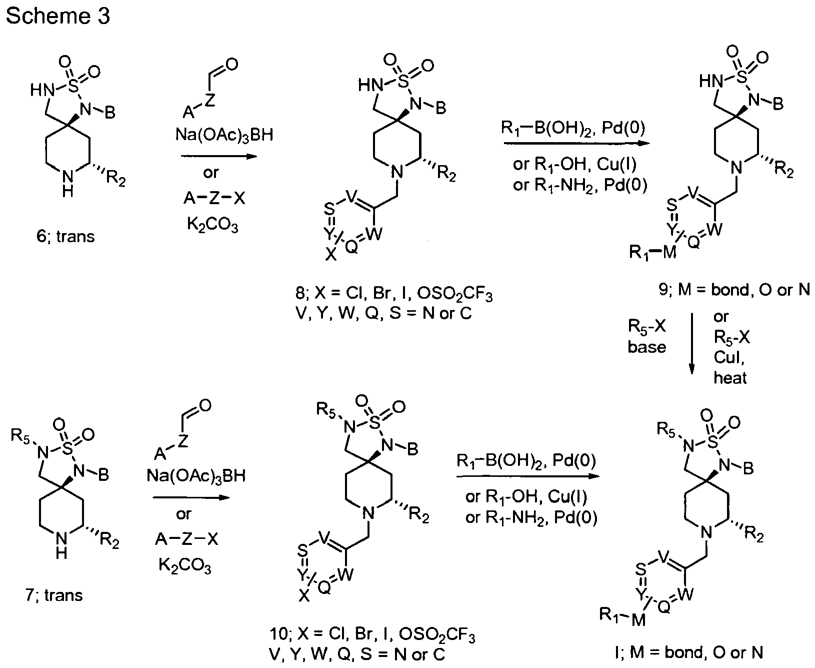

Scheme 3 illustrates the synthesis of cyclic sulfamide derivatives depicted by formula I employing methods well known to one skilled in the art. Reductive amination of **6** or **7** with an aldehyde and sodium triacetoxyborohydride or alkylation of **6** or **7** with A-Z-X (where X is defined above) and base such as potassium or cesium carbonate provides intermediates **8** or **10** (where X = halogen or triflate and V, W, Y, Q, S are defined as a nitrogen or a carbon atom). Derivatization of **8** or **10** by Suzuki coupling may be accomplished using an aryl or heteroaryl boronic acid, palladium (0) source such as Pd(OAc)₂, and a base such as cesium carbonate in DMF. Alternatively, derivatization of **8** or **10** by Buchwald coupling is accomplished with an appropriate amine, palladium (0) source such as Pd₂(dba)₃, a ligand such as BINAP, and a base such as cesium carbonate in toluene/DMA. Alternatively, conversion of **8** or **10** to form an ether is accomplished by treatment with an appropriate alcohol, CuCl, NMP, a base such as cesium carbonate, and 2, 2, 6, 6-tetramethyl-heptane-3,5-dione (TMHD). Conversion of **9** to formula I is accomplished by methods analogous to those described above.

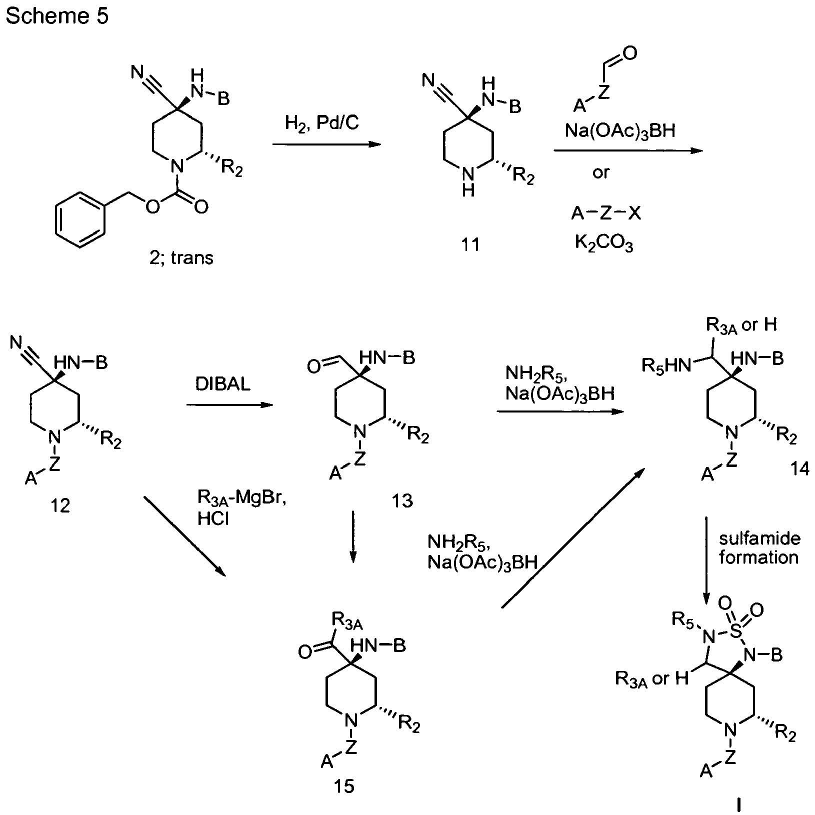

Scheme 5 illustrates the synthesis of cyclic sulfamide derivatives depicted by formula I employing methods well known to one skilled in the art. Intermediate **12** is prepared from intermediate **2** using methods analogous to those described above. Reduction of **12** using DIBAL in toluene provides intermediate **13.** Alternatively, intermediate **12** can be converted to ketone **15** by treatment with an appropriate Grignard reagent followed by acid hydrolysis. Reductive amination of **13** or **15** provides compound **14.** Formation of the cyclic sulfamide can be accomplished by treatment in a manner analogous to those described above to provide compounds depicted by formula **I.**

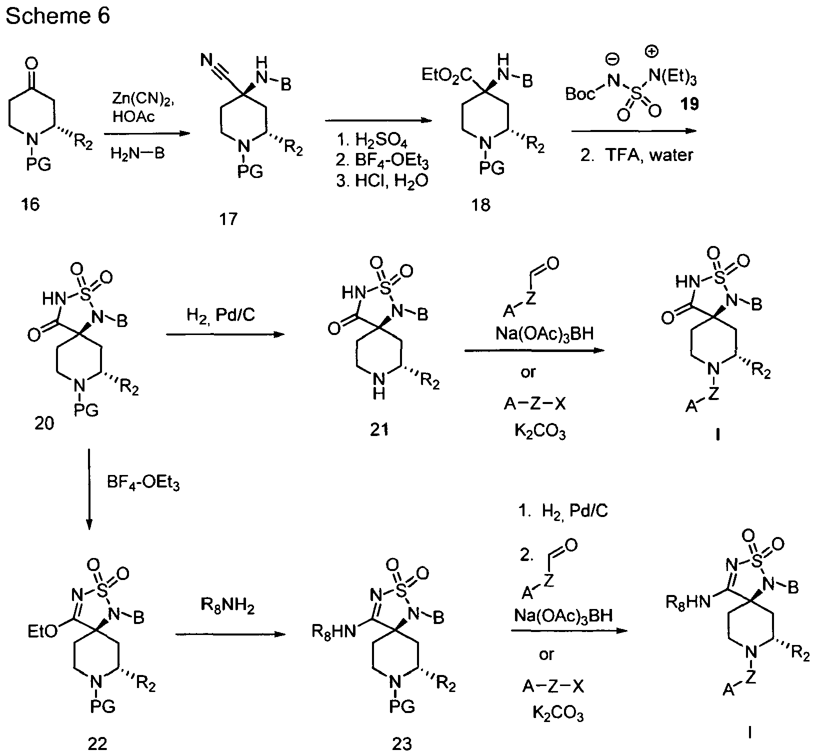

Scheme 6 illustrates the synthesis of cyclic sulfamide derivatives depicted by formula I employing methods well known to one skilled in the art. The starting appropriately protected piperidinone **16** (PG = Protecting Group) is prepared following methods analogous to those described above. Conversion of the nitrile **17** to ester **18** is accomplished by treatment with acid followed by treatment with triethyloxonium tetrafluoroborate in phosphate buffer. Treatment of **18** with an appropriate reagent such as Burgess reagent **19** provides cyclic sulfamide **20.** Deprotection using hydrogenation with an appropriate catalyst such as palladium on carbon provides compounds of formula **21.** Reductive amination of **21** with an aldehyde and sodium triacetoxyborohydride or alkylation of **21** with R-Z-X where X is defined above and base such as potassium or cesium carbonate provides compounds of formula I. Alternatively, cyclic sulfamide **20** is converted to **22** using an appropriate alkylating agent such as triethyloxonium tetrafluoroborate in phosphate buffer. Treatment of **22** with an appropriate amine provides **23,** which is converted to compounds of formula I following methods analogous to those described above.

### Experimental Section

### Preparations

### Preparation 1: (5R,7S)-1-(3-Fluorophenyl)-3,7-dimethyl-2-thia-1,3,8-triazaspiro[4.5]decane 2,2-dioxide (P1)

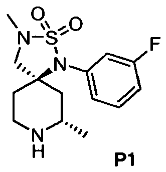

### Benzyl (2S,4R)-4-cyano-4-[(3-fluorophenyl)amino]-2-methylpiperidine-1-carboxylate

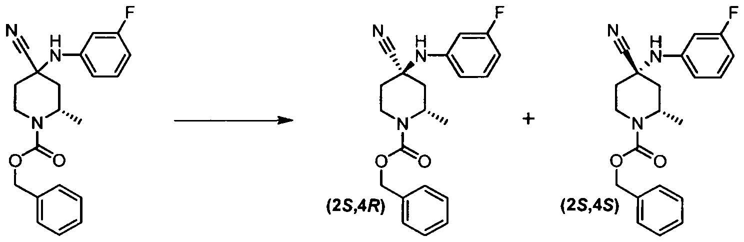

The diastereomeric mixture benzyl (2*S*)-4-cyano-4-[(3-fluorophenyl)amino]-2-methylpiperidine-1-carboxylate, prepared as in Example 1 (36 g) was subjected to chromatography to separate the diastereomeric products (Column: Chiralcel OJ-H; Eluent: 80: 20 CO₂: MeOH). The (2S,4S) isomer eluted first, followed by the title product (17 g, 47%). LCMS *m*/*z* 368.1 (M+1). ¹H NMR (400 MHz, CHLOROFORM-*d*) δ ppm 1.49 (d, *J*=7.2 Hz, 3H), 1.70 (ddd, *J*=13.1, 13.2, 4.4 Hz, 1H), 1.89 (dd, *J*=13.9, 6.5 Hz, 1H), 2.42-2.49 (m, 2H), 3.35 (ddd, *J*=14.6, 13.0, 2.4 Hz, 1 H), 3.74 (s, 1H), 4.27 (m, 1 H), 4.63 (m, 1 H), 5.14 (d, half of AB quartet, *J*=12.3 Hz, 1 H), 5.18 (d, half of AB quartet, *J*=12.3 Hz, 1 H), 6.60-6.68 (m, 3H), 7.21 (m, 1H), 7.32-7.41 (m, 5H). The relative stereochemistry of the substituents in these products was established by examination of the NOESY NMR spectra of the two diastereomers, in particular the NOE correlations between the methyl group and other hydrogens.

### Benzyl (2S,4R)-4-(aminomethyl)-4-[(3-fluorophenyl)amino]-2-methylpiperidine-1-carboxylate

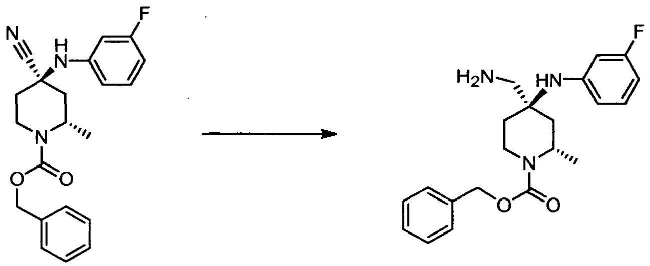

Benzyl (2*S*,4*R*)-4-cyano-4-[(3-fluorophenyl)amino]-2-methylpiperidine-1-carboxylate was converted to the title product according to the general procedure for the synthesis of benzyl (2*S*)-4-(aminomethyl)-4-[(3-fluorophenyl)amino]-2-methylpiperidine-1-carboxylate from benzyl (2S)-4-cyano-4-[(3-fluorophenyl)amino]-2-methylpiperidine-1-carboxylate in Example 1. Purification by silica gel chromatography (Gradient: 1% to 4% MeOH in dichloromethane) provided the product (7.8 g, 77%). LCMS *m*/*z* 372.1 (M+1). ¹H NMR (400 MHz, CHLOROFORM-*d*) δ ppm 1.17 (d, *J*=6.5 Hz, 3H), 1.56 (dd, *J*=14.4, 8.3 Hz, 1H), 1.70 (m, 1 H), 1.85 (ddd, *J*=13.9, 11.3, 6.2 Hz, 1 H), 2.04 (dd, *J*=14.4, 6.3 Hz, 1 H), 2.87 (d, half of AB quartet, *J*=14 Hz, 1H), 2.91 (d, half of AB quartet, *J=*14 Hz, 1 H), 3.05 (ddd, *J*=13.9, 11.5, 4.7 Hz, 1 H), 3.93 (ddd, *J*=14.0, 6.0, 2.8 Hz, 1 H), 4.12 (m, 1 H), 5.08 (s, 2H), 6.31-6.40 (m, 3H), 6.99 (m, 1 H), 7.23-7.31 (m, 5H).

### Benzyl (2S,4R)-4-[(3-fluorophenyl)amino]-4-({[(2-hydroxy-3-methoxyphenoxy)sulfonyl]amino}methyl)-2-methylpiperidine-1-carboxylate

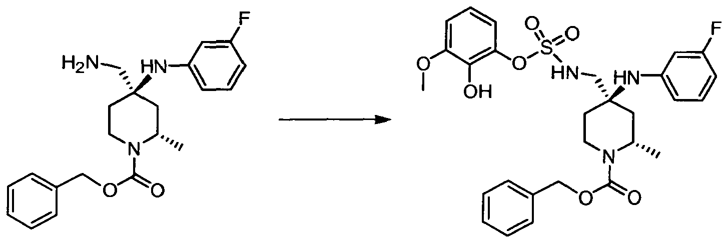

A solution of 5-methoxy-1,3,2-benzodioxathiole 2,2-dioxide (prepared according to the method of A.M. Tickner et al., Synthetic Communications 1994, 24, 1631-7; 599 mg, 2.96 mmol) in dichloromethane (0.8 mL) was added drop-wise to an ice-cooled solution of benzyl (2*S*,4*R*)-4-(aminomethyl)-4-[(3-fluorophenyl)amino]-2-methylpiperidine-1-carboxylate (1.00 g, 2.69 mmol) and triethylamine (0.20 mL, 1.4 mmol) in DMF (13.5 mL), and the resulting solution was stirred overnight at room temperature. The reaction was poured into aqueous HCl (1%, 20 mL) and extracted with dichloromethane (3x10 mL). The combined organic layers were washed with brine (30 mL), dried over sodium sulfate, filtered and concentrated *in vacuo* to provide the title product as an oil (1.50 g, 97%), which was used in the next step without purification. LCMS *m*/*z* 574.0 (M+1).

### Benzyl (5R,7S)-1-(3-fluorophenyl)-7-methyl-2-thia-1,3,8-triazaspiro[4.5]decane-8-carboxylate 2,2-dioxide (Compound 1)

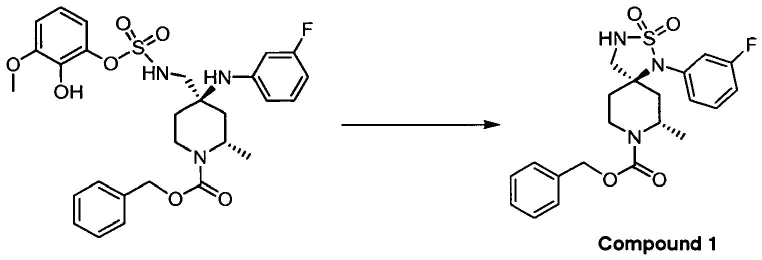

A solution of benzyl (2*S*,4*R*)-4-[(3-fluorophenyl)amino]-4-({[(2-hydroxy-3-methoxyphenoxy)sulfonyl]amino}methyl)-2-methylpiperidine-1-carboxylate (1.50 g, from the previous step, 2.61 mmol) in dioxane (26.2 mL) was heated at reflux for 66 hours. The reaction mixture was cooled to room temperature and poured into aqueous HCl (0.5M, 20 mL), then extracted with ethyl acetate (3 x 20 mL), dried over sodium sulfate and filtered. The filtrate was concentrated under reduced pressure and purified via silica gel chromatography (Gradient: 0% to 35% ethyl acetate in heptane) to provide the product as a white solid (440 mg, 39%). LCMS *m*/*z* 434.4 (M+1). ¹H NMR (400 MHz, CHLOROFORM-*d*) δ ppm 1.16 (d, *J*=7.2 Hz, 3H), 1.58 (m, 1H), 1.66-1.75 (m, 2H), 2.02 (br d, *J*=12 Hz, 1 H), 2.94 (m, 1 H), 3.60 (dd, *J*=11.6, 9.2 Hz, 1 H), 3.72 (dd, *J*=12.0, 6.9 Hz, 1 H), 4.08 (v br s, 1H), 4.50 (v br s, 1 H), 5.05 (d, half of AB quartet, *J*=12.5 Hz, 1 H), 5.09 (d, half of AB quartet, *J*=12.8 Hz, 1H), 5.56 (dd, *J*=8.7, 7.2 Hz, 1H), 7.07-7.18 (m, 3H), 7.29-7.37 (m, 5H), 7.39 (ddd, *J*=8.2, 8.2, 6.5 Hz, 1 H).

### Benzyl (5R,7S)-1-(3-fluorophenyl)-3,7-dimethyl-2-thia-1,3,8-triazaspiro[4.5]decane-8-carboxylate 2,2-dioxide

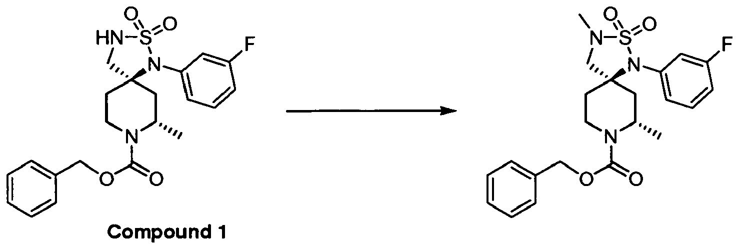

Benzyl (5*R*,7*S*)-1-(3-fluorophenyl)-7-methyl-2-thia-1,3,8-triazaspiro[4.5]decane-8-carboxylate 2,2-dioxide **(Compound 1)** was converted to the title product according to the general procedure for the synthesis of (5*R*,7*S*)-1-(3-fluorophenyl)-8-(3-isopropoxybenzyl)-3,7-dimethyl-2-thia-1,3,8-triazaspiro[4.5]decane 2,2-dioxide from (5*R*,7*S*)-1(3-fluorophenyl)-8-(3-isopropoxybenzyl)-7-methyl-2-thia-1,3,8 triazaspiro[4.5]decane 2,2-dioxide in Example 2. Purification by silica gel chromatography (Gradient: 0% to 100% ethyl acetate in heptane) provided the product as a white solid (560.4 mg, 89%).
LCMS *m*/*z* 448.0 (M+1). ¹H NMR (400 MHz, CHLOROFORM-*d*) δ ppm 1.21 (d, *J*=7.2 Hz, 3H), 1.66 (m, 1 H), 1.73-1.82 (m, 2H), 2.10 (br d, *J*=13 Hz, 1 H), 2.85 (s, 3H), 3.03 (v br dd, *J*=14, 14 Hz, 1 H), 3.38 (br d, *J*=9.3 Hz, 1 H), 3.62 (d, *J*=9.3 Hz, 1 H), 4.16 (v br s, 1 H), 4.53 (v br s, 1 H), 5.06 (br s, 2H), 7.10 (ddd, *J*=9.3, 2.2, 2.2 Hz, 1H), 7.15-7.21 (m, 2H), 7.28-7.37 (m, 5H), 7.41 (ddd, *J*=8.2, 8.2, 6.4 Hz, 1 H).

### (5R,7S)-1-(3-Fluorophenyl)-3,7-dimethyl-2-thia-1,3,8-triazaspiro[4.5]decane 2,2-dioxide (P1)

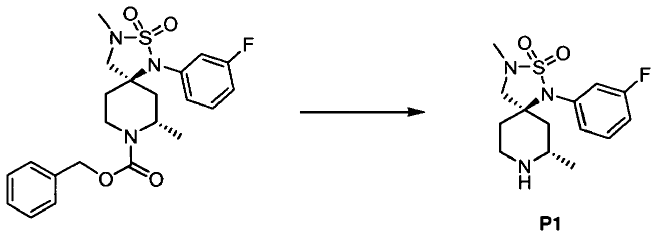

Benzyl (5*R*,7*S*)-1-(3-fluorophenyl)-3,7-dimethyl-2-thia-1,3,8-triazaspiro[4.5]decane-8-carboxylate 2,2-dioxide was converted to **P1** according to the general procedure for the synthesis of (7*S*)-1-(3-fluorophenyl)-7-methyl-2-thia-1,3,8-triazaspiro[4.5]decane 2,2-dioxide from benzyl (7*S*)-1-(3-fluorophenyl)-7-methyl-2-thia-1,3,8-triazaspiro[4.5]decane-8-carboxylate 2,2-dioxide in Example 1. The title product was obtained as a white foam (405 mg, quantitative). LCMS *m*/*z* 314.0 (M+1). ¹H NMR (400 MHz, CHLOROFORM-*d*) δ ppm 1.00 (d, *J*=6.4 Hz, 3H), 1.41 (dd, *J*=14.0, 10.0 Hz, 1H), 1.76 (ddd, *J*=14.1, 11.2, 4.6 Hz, 1H), 2.22-2.31 (m, 2H), 2.55 (ddd, *J*=12.8, 11.3, 3.0 Hz, 1 H), 2.66 (m, 1 H), 2.80-2.86 (m, 1 H), 2.83 (s, 3H), 3.21 (d, half of AB quartet, *J*=9.3 Hz, 1H), 3.28 (d, half of AB quartet, *J*=9.3 Hz, 1H), 7.13-7.22 (m, 2H), 7.27 (ddd, *J*=8.0, 1.9, 1.1 Hz, 1H), 7.40 (ddd, *J*=8.1, 8.1, 6.4 Hz, 1H).

### Preparation 2: (5R,7S)-1-(3-Fluorophenyl)-7-methyl-2-thia-1,3,8-triazaspiro[4.5]decane 2,2-dioxide (P2)

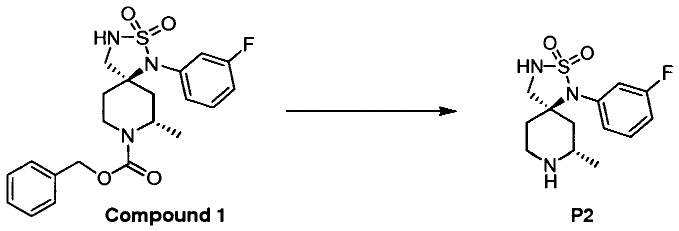

Benzyl (5*R,* 7*S*)-1-(3-fluorophenyl)-7-methyl-2-thia-1,3,8-triazaspiro[4.5]decane-8-carboxylate 2,2-dioxide (**Compound 1**) was converted to **P2** according to the general procedure for the synthesis of (7*S*)-1-(3-fluorophenyl)-7-methyl-2-thia-1,3,8-triazaspiro[4.5]decane 2,2-dioxide from benzyl (7*S*)-1-(3-fluorophenyl)-7-methyl-2-thia-1,3,8-triazaspiro[4.5]decane-8-carboxylate 2,2-dioxide in Example 1. The title product was obtained as a colorless oil (1.055 g, 99%). LCMS *m*/*z* 299.37 (M+1). ¹H NMR (400 MHz, CHLOROFORM-*d*) δ ppm 0.98 (d, *J*=6.3 Hz, 3H), 1.37 (dd, *J*=14.3, 10.9 Hz, 1H), 1.69 (ddd, *J*=14.4, 12.2, 4.7 Hz, 1H), 2.17-2.22 (m, 2H), 2.46 (ddd, *J*=12.5, 12.5, 2.8 Hz, 1H), 2.56 (m, 1H), 2.80 (ddd, *J*=12.8, 4.6, 3.5 Hz, 1H), 3.29 (d, half of AB quartet, *J*=11.9 Hz, 1H), 3.34 (d, half of AB quartet, *J*=12.0 Hz, 1H), 7.08-7.14 (m, 2H), 7.19 (ddd, *J*=8.0, 2.0, 1.0 Hz, 1 H), 7.36 (dddd, *J*=8.0, 8.0, 6.4, 0.8 Hz, 1H).

### Preparation 3: (5R,7S)-1-(3-Fluorophenyl)-8-(3-iodobenzyl)-7-methyl-2-thia-1,3,8-triazaspiro[4.5]decane 2,2-dioxide (P3)

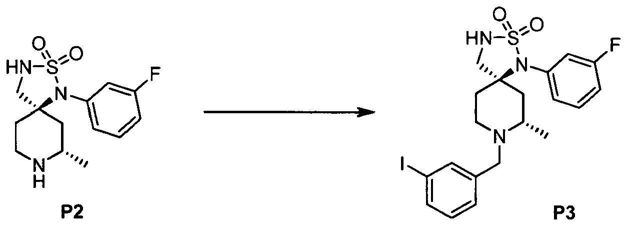

(5*R*,7*S*]-1-(3-Fluorophenyl)-7-methyl-2-thia-1,3,8-triazaspiro[4.5]decane 2,2-dioxide (**P2**) was converted to **P3** according to the general procedure for the synthesis of (5*R*,7*S*)-1-(3-fluorophenyl-8-(3-isopropoxybenzyl-7-methyl-2-thia-1,3,8 triazaspiro[4.5]decane 2,2-dioxide (**1a**) from (7*S*)-1-(3-fluorophenyl)-7-methyl-2-thia-1,3,8-triazaspiro[4.5]decane 2,2-dioxide in Example 1. Purification by silica gel chromatography (Gradient: 0% to 5% MeOH in dichloromethane) provided **P3** as a solid (230 mg, 48%). LCMS *m*/*z* 516.1 (M+1). ¹H NMR (400 MHz, CHLOROFORM-*d*) δ ppm 1.01 (d, *J*=6.7 Hz, 3H), 1.60 (ddd, *J*=13.6, 6.1, 1.0 Hz, 1H), 1.81 (m, 1H), 1.92 (m, 1H), 1.99 (dd, *J*=13.6, 4.6 Hz, 1H), 2.19 (ddd, *J*=12.6, 7.0, 3.8 Hz, 1H), 2.46 (ddd, *J*=12.6, 8.1, 3.5 Hz, 1H), 2.67 (m, 1H), 3.23 (d, *J*=13.8, 1H), 3.43-3.53 (m, 2H), 3.54 (d, *J*=13.7 Hz, 1 H), 5.40 (br s, 1H), 6.99 (dd, *J*=8.2, 7.7 Hz, 1 H), 7.11-7.19 (m, 4H), 7.37 (m, 1H), 7.53-7.56 (m, 2H).

### Preparation 4: (5R,7S)-1-(3,5-difluorophenyl)-7-methyl-2-thia-1,3,8-triazaspiro[4.5]decane 2,2-dioxide (P4)

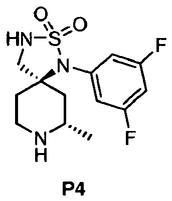

The title compound was prepared in a manner similar to that employed for (5*R*,7*S*)-1-(3-fluorophenyl)-7-methyl-2-thia-1,3,8-triazaspiro[4.5]decane 2,2-dioxide (**P2**), except that 3,5-difluoroaniline was used in place of 3-fluoroaniline. Purification was carried out via silica gel chromatography (Gradient: 1% to 15% MeOH in dichloromethane) to provide **P4** as an oil. LCMS *m*/*z* 318.0 (M+1). ¹H NMR (400 MHz, CHLOROFORM-*d*) δ ppm 1.01 (d, *J*=6.4 Hz, 3H), 1.34 (dd, *J*=14.0, 10.4 Hz, 1H), 1.69 (ddd, *J*=14.0, 11.7, 4.8 Hz, 1 H), 2.17-2.25 (m, 2H), 2.56 (m, 1 H), 2.64 (m, 1 H), 2.86 (ddd, *J*=12.8, 4, 4 Hz, 1H), 3.38 (d, half of AB quartet, *J*=12.1 Hz, 1H), 3.43 (d, half of AB quartet, *J*=12.1 Hz, 1 H), 6.93 (tt, *J*=8.7, 2.3 Hz, 1H), 6.99-7.05 (m, 2H).

### Examples

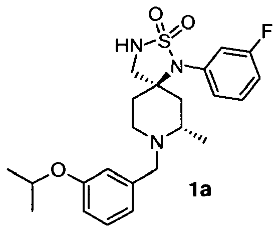

### Example 1: (5R,7S)-1-(3-Fluorophenyl)-8-(3-isopropoxybenzyl)-7-methyl-2-thia-1,3,8-triazaspiro[4.5]decane 2,2-dioxide (1a)

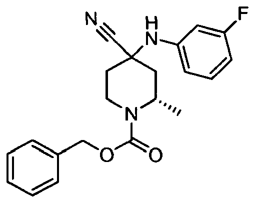

### Benzyl (2S)-4-cyano-4-[(3-fluorophenyl)amino]-2-methylpiperidine-1-carboxylate

To a solution of benzyl (2*S*)-2-methyl-4-oxopiperidine-1-carboxylate (see C. Coburn *et al.*,PCT Int. Appl. 2007, WO 2007011810; 20.0 g, 80.9 mmol) in acetic acid (162 mL) was added 3-fluoroaniline (18.0 g, 162 mmol) and zinc cyanide (23.7 g, 202 mmol). The reaction was allowed to stir overnight at room temperature. The reaction was cooled to 0°C and quenched with ammonium hydroxide until the mixture was basic. The reaction was filtered to provide a yellow solid which was combined with further extractions with methylene chloride, dried with sodium sulfate, and concentrated. The material was purified using silica gel chromatography to afford the title compound (29.7 g, 72%). MS *m*/*z* 368.1 (M+1).

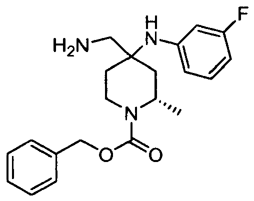

### Benzyl (2S)-4-(aminomethyl)-4-[(3-fluorophenyl)amino]-2-methylpiperidine-1-carboxylate

To a solution of benzyl (2*S*)-4-cyano-4-[(3-fluorophenyl)amino]-2-methylpiperidine-1-carboxylate (8.0 g, 21.8 mmol) in NH₃ (109 mL, 218 mmol, 1M in MeOH) was added Raney Nickel in water (1.87 g, 21.8 mmol). The reaction was hydrogenated (50 psi) for 12 h, filtered over Celite, concentrated, and purified by silica gel chromatography to provide the title compound (8.0 g, 72%). MS *m*/*z* 372.1 (M+1).

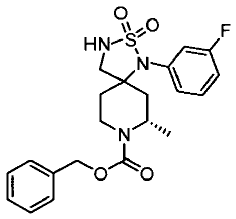

### Benzyl (7S)-1-(3-fluorophenyl)-7-methyl-2-thia-1,3,8-triazaspiro[4.5]decane-8-carboxylate 2,2-dioxide

To a solution of 3-(imidazole-1-sulfonyl)-1-methyl-3*H*-imidazol-1-ium triflate (S. Beaudoin, J. Org Chem. 2003, 68, 115-119) (1.47 g, 4.04 mmol) in acetonitrile (3 mL) was added benzyl (2*S*)-4-(aminomethyl)-4-[(3-fluorophenyl)amino]-2-methylpiperidine-1-carboxylate (1.0 g, 2.69 mmol) in acetonitrile (2 mL) and the reaction was stirred overnight at room temperature. The reaction was concentrated and purification by silica gel chromatography provided the title compound (750 mg, 64%). MS *m*/*z* 434.1 (M+1).

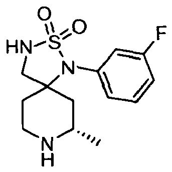

### (7S)-1-(3-Fluorophenyl)-7-methyl-2-thia-1,3,8triazaspiro[4.5]decane 2,2-dioxide

To a solution of benzyl (7*S*)-1-(3-fluorophenyl)-7-methyl-2-thia-1,3,8-triazaspiro[4.5]decane-8-carboxylate 2,2-dioxide (90 mg, 0.2 mmol) in MeOH (2 mL) was added palladium hydroxide (15 mg, 0.1 mmol). The mixture was hydrogenated at 50 psi overnight. The reaction was filtered and concentrated to afford the title compound (60 mg, 96%). MS *m*/*z* 300.1 (M+1).

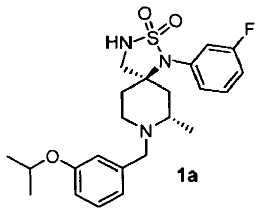

### (5R,7S)-1-(3-Fluorophenyl)-8-(3-isopropoxybenzyl)-7-methyl-2-thia-1,3,8-triazaspiro[4.5]decane 2,2-dioxide (1a)

To a solution of (7*S*)-1-(3-fluorophenyl)-7-methyl-2-thia-1,3,8-triazaspiro[4.5]decane 2,2-dioxide (60 mg, 0.2 mmol) and 3-isopropoxybenzaldehyde (3 mg, 0.2 mmol) in dichloroethane (2.0 mL) was added acetic acid (12 mg, 0.2 mmol). The reaction was allowed to stir at room temperature for 5 h and charged with sodium triacetoxyborohydride (85 mg, 0.4 mmol) and stirred overnight. The reaction was quenched with aqueous sodium bicarbonate, extracted with methylene chloride, dried over sodium sulfate, and concentrated. Purification by silica gel chromatography using 20-50% ethyl acetate/hexane gradient provided the first eluent (9.7 mg, 11%) of (5*S*,7*S*)-1-(3-fluorophenyl)-8-(3-isopropoxybenzyl)-7-methyl-2-thia-1,3,8-triazaspiro[4.5]decane 2,2-dioxide (**1b**). ¹H NMR (400 MHz, CHLOROFORM-*d*) δ ppm 1.17 (d, *J*=6.22 Hz, 3H) 1.30 (dd, *J*=6.01, 3.52 Hz, 6H) 1.41 - 1.54 (m, 1H) 1.61 (dt, *J*=12.86, 4.15 Hz, 1H) 1.69 - 1.77 (m, 1H) 1.82 - 1.98 (m, 2H) 2.24 - 2.36 (m, 1 H) 2.77 (td, *J*=12.34, 4.46, 3.11 Hz, 1 H) 2.97 (d, *J*=13.27 Hz, 1 H) 3.49 - 3.61 (m, 2H) 4.00 (d, *J*=13.27 Hz, 1 H) 4.45 - 4.56 (m, 1H) 4.67 (t, *J*=7.46 Hz, 1H) 6.71 - 6.80 (m, 3H) 7.07 - 7.20 (m, 4H) 7.40 (ddd, *J*=8.09, 6.22 Hz, 1 H), MS *m*/*z* 448.1 (M+1) and the second eluent (14.5 mg, 16%) of (5*R*,7*S*)-1-(3-fluorophenyl)-8-(3-isopropoxybenzyl)-7-methyl-2-thia-1,3,8-triazaspiro[4.5]decane 2,2-dioxide (**1a**). ¹H NMR (400 MHz, CHLOROFORM-*d*) δ ppm 1.03 (d, *J*=6.64 Hz, 3H) 1.30 (d, *J*=6.22 Hz, 6H) 1.60 (dd, *J*=14.10, 5.39 Hz, 1 H) 1.78 - 1.86 (m, 1 H) 1.92 - 2.05 (m, 2H) 2.22 - 2.30 (m, 1H) 2.47 - 2.56 (m, 1 H) 2.67 - 2.75 (m, 1H) 3.26 (d, *J*=13.69 Hz, 1 H) 3.44 - 3.56 (m, 2H) 3.59 (d, *J*=13.69 Hz, 1H) 4.44 - 4.55 (m, 1 H) 4.85 (t, *J*=7.67 Hz, 1 H) 6.74 (d, 2H) 6.73 (d, *J*=4.98 Hz, 1H) 7.07 - 7.19 (m, 4H) 7.33 - 7.41 (m, 1 H), MS *m*/*z* 448.1 (M+1).

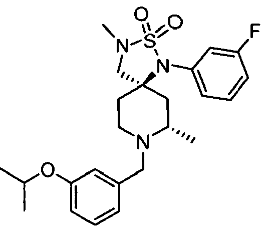

### Example 2: (5R,7S)-1-(3-Fluorophenyl)-8-(3-isopropoxybenzyl)-3,7-dimethyl-2-thia-1,3,8-triazaspiro[4.5]decane 2,2-dioxide

A solution of (5*R*,7*S*)-1-(3-fluorophenyl)-8-(3-isopropoxybenzyl)-7-methyl-2-thia-1,3,8 triazaspiro[4.5]decane 2,2-dioxide (**1a**) (20 mg, 0.045 mmol) in DMF was added to a slurry of NaH (3.6 mg, 0.09 mmol) in DMF (0.6 mL) at 0°C. The reaction was stirred at 0°C for 2 hours then methyl iodide (8.7 mg, 0.061 mmol) in DMF (0.2 mL) was added. The reaction was allowed to gradually warm to room temperature and stirred overnight. The reaction was diluted with water and extracted with ethyl acetate (3 x 10 mL). The combined organics were dried, concentrated, and purified by silica gel chromatography to yield the title compound (15 mg, 67%). ¹H NMR (400 MHz, CHLOROFORM-*d*) δ ppm 1.04 (d, *J*=6.64 Hz, 3H) 1.30 (d, J=5.81 Hz, 6H) 1.64 (dd, *J*=13.69, 4.98 Hz, 1H) 1.89 - 1.96 (m, 2H) 2.04 (dd, *J*=13.48, 4.77 Hz, 1 H) 2.28 - 2.37 (m, 1 H) 2.49 - 2.58 (m, 1 H) 2.75 - 2.80 (m, 1 H) 2.82 (s, 3H) 3.26 (d, *J*=9.13 Hz, 1 H) 3.31 (d, *J*=13.69 Hz, 1 H) 3.44 (d, *J*=9.13 Hz, 1 H) 3.56 (d, *J*=13.27 Hz, 1 H) 4.44 - 4.54 (m, 1 H) 6.73 (d, *J*=2.07 Hz, 1 H) 6.75 (s, 2H) 7.09 - 7.21 (m, 4H) 7.34 - 7.43 (m, 1 H); MS *m*/*z* 462.3 (M+1).

### Example 3: (5R,7S)-1-(3-fluorophenyl)-8-(3-isopropoxybenzyl)-7-methyl-3-(pyridin-2-ylmethyl)-2-thia-1,3,8-triazaspiro[4.5]decane 2,2-dioxide (3)

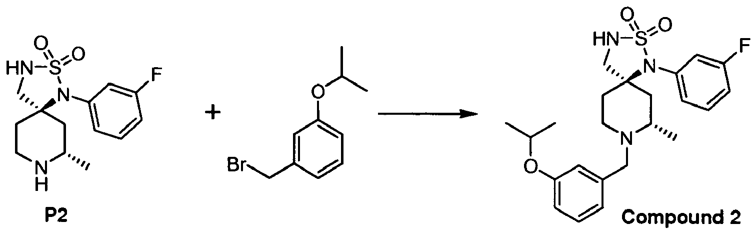

### (5R,7S)-1-(3-Fluorophenyl)-8-(3-isopropoxybenzyl)-7-methyl-2-thia-1,3,8-triazaspiro[4.5]decane 2,2-dioxide (Compound 2; also described as Example 1a)

A solution of 1-(bromomethyl)-3-isopropoxybenzene (239 mg, 1.04 mmol) in DMF (0.5 mL) was added drop-wise over 5 min to a mixture of (5*R*,7*S*)-1-(3-fluorophenyl)-7-methyl-2-thia-1,3,8-triazaspiro[4.5]decane 2,2-dioxide (**P2,** 312 mg, 1.04 mmol) and cesium carbonate (680 mg, 2.09 mmol) in DMF (3 mL), and the resulting suspension was stirred overnight at room temperature. The reaction mixture was then diluted with water (20 mL) and extracted with ethyl acetate (3 x 30 mL); the combined organic extracts were dried over sodium sulfate, filtered, and concentrated *in vacuo.* The residue was purified by silica gel chromatography (Gradient: 0% to 5% MeOH in dichloromethane) to provide the product as a white solid (185 mg, 40%). LCMS *m*/*z* 448.1 (M+1). ¹H NMR (400 MHz, CHLOROFORM-*d*) δ ppm 1.06 (br s, 3H), 1.32 (d, *J*=6.1 Hz, 6H), 1.61 (br m, 1 H), 1.85 (br s, 1 H), 1.96-2.09 (br m, 2H), 2.29 (m, 1 H), 2.55 (br s, 1H), 2.73 (m, 1 H), 3.28 (br d, *J*=13 Hz, 1 H), 3.50 (dd, half of ABX system, *J*=12.0, 9.0 Hz, 1H), 3.56 (dd, half of ABX system, *J*=12.1, 7.4 Hz, 1 H), 3.62 (br d, *J*=13 Hz, 1 H), 4.51 (septet, *J*=6.0 Hz, 1 H), 4.68 (br dd, *J*=8, 8 Hz, 1 H), 6.74-6.77 (m, 3H), 7.10-7.19 (m, 4H), 7.38 (m, 1 H).

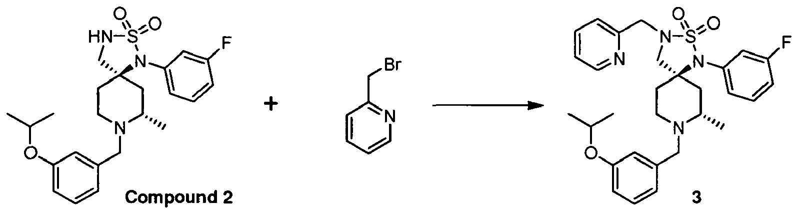

### (5R,7S')-1-(3-Fluorophenyl)-8-(3-isopropoxybenzyl)-7-methyl-3-(pyridin-2-ylmethyl)-2-thia-1,3,8-triazaspiro[4.5]decane 2,2-dioxide (3)

2-(Bromomethyl)pyridine hydrobromide (40.0 mg, 0.158 mmol) was added to a mixture of (5*R*,7*S*)-1-(3-fluorophenyl)-8-(3-isopropoxybenzyl)-7-methyl-2-thia-1,3,8-triazaspiro[4.5]decane 2,2-dioxide (**Compound 2,** 28.3 mg, 0.0632 mmol) and cesium carbonate (103 mg, 0.316 mmol) in DMF (1.5 mL), and the resulting suspension was stirred overnight at room temperature. The reaction mixture was then diluted with water and extracted with ethyl acetate (3 x 20 mL). The combined organic extracts were dried over sodium sulfate, filtered and concentrated *in vacuo* to provide a residue, which was purified by silica gel chromatography (Gradient 0% to 5% MeOH in dichloromethane). The title product was obtained as a white solid (22 mg, 65%). LCMS *m*/*z* 539.0 (M+1). ¹H NMR (500 MHz, CHLOROFORM-*d*) δ 0.97 (br s, 3H), 1.30 (br d, *J*=6 Hz, 6H), 1.63 (m, 1 H), 1.85 (m, 1 H), 1.93-2.06 (m, 2H), 2.29 (m, 1H), 2.45 (m, 1 H), 2.77 (m, 1 H), 3.27 (br d, *J*=13 Hz, 1 H), 3.40 (d, *J*=9.6 Hz, 1H), 3.49-3.56 (m, 2H), 4.36 (d, *J*=14.9 Hz, 1 H), 4.47-4.54 (m, 2H), 6.72-6.75 (m, 3H), 7.13-7.24 (m, 4H), 7.28 (m, 1 H, assumed, partially obscured by solvent peak), 7.40 (m, 1 H), 7.56 (br d, *J*= 7 Hz, 1 H), 7.75 (ddd, *J*=7.7, 7.7, 1.8 Hz, 1 H), 8.60 (m, 1H).

### Example 4: (5R, 7S)-1-(3-Fluorophenyl)-8-(3-isopropoxybenzyl)-7-methyl-3-(5-phenyl-4,5-dihydroisoxazol-3-yl)-2-thia-1,3,8-triazaspiro[4.5]decane 2,2-dioxide (4)

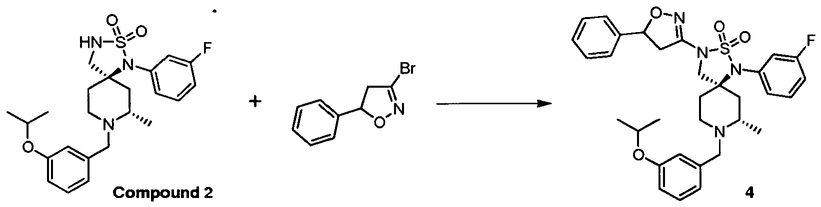

(5*R*,7*S*)-1-(3-Fluorophenyl)-8-(3-isopropoxybenzyl)-7-methyl-2-thia-1,3,8-triazaspiro[4.5]decane 2,2-dioxide (**Compound 2,** 33 mg, 0.074 mmol), dioxane (2 mL), 3-bromo-5-phenyl-4,5-dihydroisoxazole (prepared according to P. Caldirola et al., Tetrahedron Letters 1986, 27, 4647-4650; 50.2 mg, 0.222 mmol), copper(I) iodide (42.5 mg, 0.222 mmol) and potassium carbonate (30.7 mg, 0.222 mmol) were combined in a dry sealable flask, and treated with N,N'-dimethylethane-1,2-diamine (32 µL, 0.30 mmol). The mixture was heated overnight at 105 °C, then cooled to room temperature and filtered through Celite. The filter pad was washed with ethyl acetate (20 mL), and the combined filtrates were washed with saturated aqueous sodium bicarbonate solution (20 mL), dried over sodium sulfate, filtered and concentrated *in vacuo.* The residue was purified by chromatography on silica gel (Gradient: 0% to 100% ethyl acetate in heptane) to provide the title product as an off-white solid (21.8 mg, 50%). LCMS *m*/*z* 593.1 (M+1). ¹H NMR (500 MHz, CHLOROFORM-*d*) δ 1.09 (m, 3H), 1.32 (br d, *J*=6 Hz, 6H), 1.70 (m, 1H), 1.95-2.10 (m, 3H), 2.38 (m, 1H), 2.61 (m, 1 H), 2.83 (m, 1H), 3.34 (br d, *J*=13 Hz, 1 H), 3.45 (m, 1 H), 3.62 (m, 1 H), 3.83 (m, 1 H), 3.90 (m, 1 H), 4.10 (m, 1H), 4.51 (m, 1 H), 5.72 (m, 1 H), 6.75-6.78 (m, 3H), 7.12-7.23 (m, 4H), 7.33-7.45 (m, 6H).

The structures of additional Examples are shown in Tables 1, 2 and 3. Tables 1-3 give physical data and preparative information for these additional Examples, and Table 4 contains relevant biological data for all Examples. Data was obtained either on the compound as a free base or on a pharmaceutically acceptable salt of the compound.

### Methods

### Method A: Preparation of 8-substituted (5R,7S)-1-(3-fluorophenyl)-7-methyl-2-thia-1,3,8-triazaspiro[4.5]decane 2,2-dioxide analogues by reductive amination

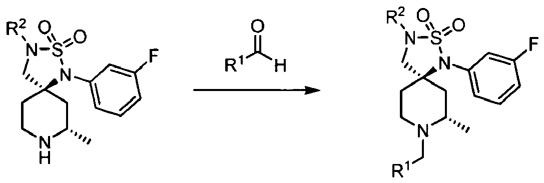

To a solution of (5*R*,7*S*)-1-(3-fluorophenyl)-7-methyl-2-thia-1,3,8-triazaspiro[4.5]decane 2,2-dioxide, optionally substituted at the 3-position (1 equivalent) and the appropriate aldehyde (1-2 equivalents) in dichloroethane (generally 0.1M in substrate) is added acetic acid (2 equivalents). The reaction is allowed to stir at room temperature for 5 h, then charged with sodium triacetoxyborohydride (2 equivalents) and stirred overnight. The reaction is quenched with aqueous sodium bicarbonate solution and extracted with methylene chloride; the combined organic layers are dried over sodium sulfate, filtered and concentrated. The product can be purified by silica gel chromatography. If the required aldehyde is not commercially available, or easily derived from a similar commercially available compound, preparative information is given in a footnote in Table 1.

### Method B: Preparation of 3-substituted (5R,7S)-1-aryl-7-methyl-2-thia-1,3,8triazaspiro[4.5]decane 2,2-dioxide analogues by alkylation

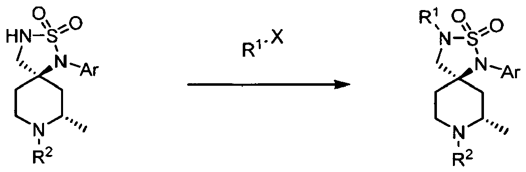

For Methods B and C, if the required alkylating agent or a related compound (alcohol, aldehyde, carboxylic acid derivative) is not commercially available, preparative information is given in a footnote in Tables 1 - 3. A solution of 8-substituted (5*R*,7*S*)-1-aryl-7-methyl-2-thia-1,3,8-triazaspiro[4.5]decane 2,2-dioxide (1 equivalent) in DMF (generally 0.1M in substrate) is added to a slurry of NaH (2 equivalents) in a small amount of DMF at 0°C. The reaction is stirred at 0 °C for 2 hours and then treated with a solution of the alkylating agent (1-1.4 equivalents) in a small amount of DMF. The reaction is allowed to gradually warm to room temperature. Alternatively, the substrate (1 equivalent) and alkylating agent (1-3 equivalents) can be combined in DMF (0.1M in substrate) and treated with cesium carbonate (1.5-5 equivalents) at room temperature. The reaction is stirred overnight, then diluted with water and extracted with ethyl acetate. The combined organic layers are dried, concentrated, and purified by silica gel chromatography to provide the title product.

### Method C: Preparation of 8-substituted (5R,7S)-1-aryl-7-methyl-2-thia-1,3,8-triazaspiro[4.5]decane 2,2-dioxide analogues via alkylation

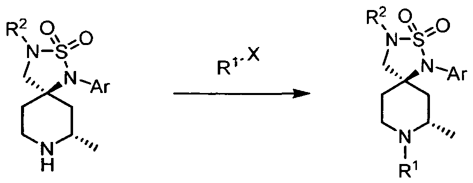

(5*R*,7*S*)-1-Aryl-7-methyl-2-thia-1,3,8-triazaspiro[4.5]decane 2,2-dioxide, preferably substituted at the 3-position (1 equivalent) and the alkylating agent (1-2 equivalents) are combined in DMF (generally 0.1M in substrate) and treated with cesium carbonate (1.5-3 equivalents) at room temperature. The reaction is stirred overnight, then diluted with water and extracted with ethyl acetate. The combined organic layers are dried, concentrated, and purified by silica gel chromatography to provide the title product.

### Method D: Preparation of 1-substituted-1H-pyrazole-5-carbaldehydes

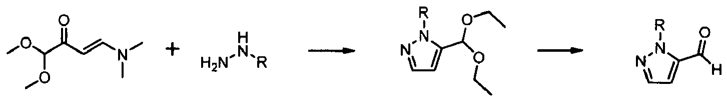

### Step 1. Preparation of 1-substituted 5-(diethoxymethyl)-1H-pyrazole

(3*E*)-4-(Dimethylamino)-1,1-dimethoxybut-3-en-2-one (2.5 mmol) and the appropriate substituted hydrazine (2.5 mmol) are combined in ethanol (12 mL) and heated to 85 °C until the reaction has proceeded to completion. Removal of solvent *in vacuo* provides the crude product, which can be taken on without purification or subjected to silica gel chromatography.

### Step 2. Preparation of 1-substituted-1H-pyrazole-5-carbaldehyde.

The 1-substituted 5-(diethoxymethyl)-1*H*-pyrazole from the previous step is dissolved in aqueous HCl (0.4 - 1.5N, 10 mL). Ethanol can be added to enhance solubility. The reaction mixture is reacted at room temperature or heated to 60 °C until the reaction is complete. Neutralization with sodium bicarbonate is followed by extraction with dichloromethane. Removal of solvent provides a residue which can be purified by silica gel chromatography to provide the desired pyrazole aldehyde.

### Method E: Preparation of 8-(3-arylbenzyl)-substituted (5R,7S)-1-(3-fluorophenyl)-7-methyl-2-thia-1,3,8-triazaspiro[4.5]decane 2,2-dioxide analogues via Suzuki reaction

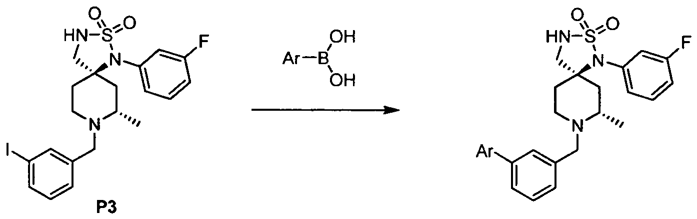

(5*R*,7*S*)-1-(3-Fluorophenyl)-8-(3-iodobenzyl)-7-methyl-2-thia-1,3,8-triazaspiro[4.5]decane 2,2-dioxide (**P3**) (1 equivalent), the appropriate boronic acid (2-3 equivalents), sodium carbonate (5 equivalents) and water (100 equivalents) are combined in ethanol (0.1M in substrate) and degassed with nitrogen. Tetrakis(triphenylphosphine)palladium(0) (0.1 equivalent) is added, and the reaction mixture is heated at 40 °C overnight. The reaction is poured into dilute aqueous sodium bicarbonate and extracted with dichloromethane. The combined organic layers are dried over sodium sulfate, filtered, and concentrated *in vacuo.* Purification of the title product is carried out via silica gel chromatography.

**Table 1**

| | | | | | |
|---|---|---|---|---|---|
| 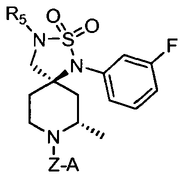 | | | | | |

| Ex # | R₅ | Z-A | Prep Meth od; Starti ng Mate rial | IUPAC Name | ¹H NMR (400 MHz, CDCl₃), ¹³C NMR (100 MHz, CDCl₃) (unless otherwise indicated): observed peaks, δ (ppm); Mass spectrum: LCMS, observed ion *m*/*z* (unless otherwise indicated) |
|---|---|---|---|---|---|
| 5 | Me | 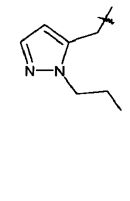 | A¹ ; P1 | (5*R*,7*S*)-1-(3-fluorophenyl)-3,7-dimethyl-8-[(1-propyl-1H-pyrazol-5-yl)methyl]-2-thia-1,3,8-triazaspiro[4.5]deca ne 2,2-dioxide | 0.69 (t, *J*=7 6Hz, 3H), 1.02 (d, *J*=6.8 Hz, 3H), 159-1.72 (m, 3H), 1.84-1.89 (m, 2H), 194 (s, 2H), 2.26-2.33 (m, 1H), 2.47-2.55 (m, 1H), 2.74-2.78 (m, 1H), 2.79 (s, 3H), 3.23 (d, *J*=9.4 Hz, 1 H), 3.30 (d, *J*=13.9 Hz, 1H), 3.42 (d, *J*=9.2 Hz, 1H), 3.57 (d, *J*=13.9 Hz, 1H), 3.87-3.93 (m, 2H), 5.97 (d, *J*=1.8 Hz, 1H), 7.08-7.18 (m, 3H), 7.32-7.40 (m, 2H); 11.27, 23.94, 33.25, 33.51, 34.29, 42.05, 43.86, 48.77, 51 10, 51.62, 60.41, 61.45, 106.72, 116.80, 117.06, 120.68, 120.94, 129.22, 130.25, 130.51, 138.01; APCI 436.0 (M+1). |
| 6 | Me | 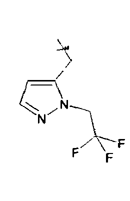 | A, D; P1 | (5*R*,7*S*)-1-(3-fluorophenyl)-3,7-dimethyl-8-{[1-(2,2,2-trifluoroethyl)-1 H-pyrazol-5-yl]methyl}-2-thia-1,3,8-triazaspiro[4.5]deca ne 2,2-dioxide | 1.03 (d, *J*=6.8 Hz, 3H), 1.60-1.66 (m, 1H), 1.85-1 93 (m, 2H), 2.17-2.28 (m, 1H), 2.45-2.52 (m, 1H), 2.64-2.70 (m, 1H), 2.8 (s, 2H), 3.23 (d, *J*=9.4 Hz, 1 H), 3.3 (d, *J*=14 2 Hz, 1H), 3.39 (d, *J*=9.2 Hz, 1H), 3.70(d, *J*=14.2 Hz, 1H), 4.06-4.13 (q, 1H), 4.68-4.78 (m, 1 H), 6.08 (d, *J*=1.8 Hz, 1H), 7.1-7.2 (m, 1H), 7.36-7.45 (m, 1H); APCI 476.3 (M+1). |
| 7 | Me | 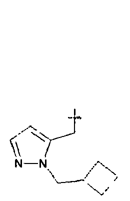 | A, D; P1 | (5R,7S)-8-{[1-(cyclobutylmethyl)-1 H-pyrazol-5-yl]methyl}-1-(3-fluorophenyl)-3,7-dimethyl-2-thia-1,3,8-triazaspiro[4.5]deca ne 2,2-dioxide | 1.04 (d, *J*=6.6 Hz, 3H), 1.26 (t, *J*=7.4 Hz, 3H), 1.60-1.72 (m, 4H), 1.79-1.92 (m, 3H), 2.3-2.37 (m, 1H), 2.49-2.56 (m, 1H), 2.64-2.72 (m, 1H), 2.81 (s, 3H), 3.25 (d, *J*=9.6 Hz, 2H), 3.32 (d, *J*=13.9 Hz, 1H), 3.44 (d, *J*=9.2 Hz), 3.56 (d, *J*= 13.9 Hz), 3.97 (dd, *J*=7.2, 2.0 Hz, 2H), 5.98 (d, *J*=1.8 Hz, 1H), 7.09-7.19 (m, 3H), 7.32 (d, *J*=1.8 Hz, 1H), 7.35-7.41 (m, 1H); 33.24, 33.50, 34.01, 36.08, 42.03, 43.85, 45.66, 48.50, 48.76, 51.61, 51.87, 54.20, 60.15, 60.40, 61.44, 61.96, 99.99, 106.72, 116.81, 117.07, 120.69, 120.95, 129.23, 129.49, 130.26, 130.52, 138.02, 138.28; APCI 462.4 (M+1). |
| 8 | Me | 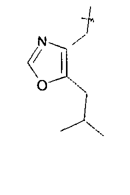 | A², P1 | (5*R*,7*S*)-1-(3-fluorophenyl)-8-[(5-isobutyl-1,3-oxazol-4-yl)methyl]-3,7-dimethyl-2-thia-1,3,8-triazaspiro[4.5]deca ne 2,2-dioxide | 0 83 (dd, J = 4.7, 6 6 Hz, 6H), 1.09 (d, J=6.6 Hz, 3H), 1.65 (d, J=13.1, 5.1 Hz, 1 H), 1 85-1.95 (m, 3H), 1.97-2 08 (m, 2H), 2.11 (d, J=13.5, 4.1 Hz, 1H), 2.38 (d, J=7.0 Hz, 1H), 2 30-2.36 (m, 2H), 2.50-2.59 (m, 1H), 2.80 (s, 3H), 3.22 (d, J=9.2 Hz, 1H), 3.38 (d, J=9.2 Hz, 2H), 7.06-7.17 (m, 3H), 7.30-7.39 (m, 1H), 7.76 (s, 1H); 22.37, 22.63, 28.06, 33.24, 33 50, 33.76, 42.55, 51.35, 60.92, 116.81, 117.07, 120.69, 120.95, 129.23, 129.49, 130.26, 149.15, 149.41, |
| 9 | Me | 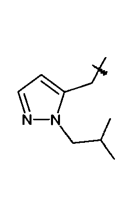 | A¹, P1 | (5*R*,7*S*)-1-(3-fluorophenyl)-8-[(1-isobutyl-1H-pyrazol-5-yl)methyl]-3,7-dimethyl-2-thia-1,3,8-triazaspiro[4.5]deca ne 2,2-dioxide | 0 74 (dd, *J*=6.6, 3.7 Hz, 6H), 1.03 (d, J 6.8 Hz, 2H), 1.35 (t, *J*=7.2 Hz, 3H), 1.62 (dd, *J*=5.1 Hz, 13.5Hz, 1 H), 1.89 (t, *J*=5.5 Hz, 1H), 1.95-2.01 (m, 1H), 2.09-2.18 (m, 1H), 2.26-2.33 ( m, 1H), 2.48-2.56 (m, 1 H), 2.73-2.79 (m, 1H), 2.80 (s, 3H), 3.25 (t, *J*=9.2 Hz, 1H), 3.40-3.49 (m, 3H), 3.78 (t, *J*=7.0 Hz, 2H), 5.99 (d, *J*=1.8 Hz, 1H), 7.09-7.19 (m, 3H), 7.34-7.42 (m, 2H); 20.30, 29.62, 48.76, 51.87, 61.44, 106.72, 174.50; APCI 450.43 (M+1). |
| 10 | Me | 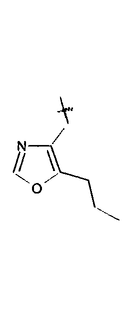 | A; P1 | (5*R*,7*S*)-1-(3-fluorophenyl)-3,7-dimethyl-8-[(5-propyl-1,3-oxazol-4-yl)methyl]-2-thia-1,3,8-triazaspiro[4.5]deca ne 2,2-dioxide | 0.84 (t, *J*=7.4 Hz, 3H), 1.07 (d, *J*=6 6Hz, 3H), 1.23 (t, *J*=7.2 Hz, 1H), 1.51-1.58 (q, 1H), 1.62 (dd, *J*=6 5, 14 3 Hz, 1H), 1.87-1.95 (m, 1 H), 198-206 (m, 1 H), 2.09 (dd, *J*=4.3, 13 7 Hz, 1H), 2.28-2.35 (m, 1 H), 2.48 (t, *J*=7.6 Hz, 1 H), 2.51-2.56 (m, 2H), 2.71-2.78 (m, 1H), 2.79 (s, 3H), 3.21 (d, *J*=9.4 Hz, 1 H), 3 37 (d, *J*=9.4 Hz, 2H), 343 (d, *J*=12.3 Hz, 1H), 7.04-7.16 (m, 3H), 7.28-7.35 (m, 1H), 7.64 (s, 1H); 13.83, 21.34, 21.60, 21.85, 26.77, 33.24, 33.50, 34.27, 34.23, 42.55, 42.81, 44.88, 48.24, 51.09, 51.35, 60.92, 61.70, 116.55, 116.81, 117.07, 120.69, 120.95, 129.23, 129.49, 130 26, 149.15, 149.41, 150.18, 161.57; APCI 437 3 (M+1). |
| 11 | Me | 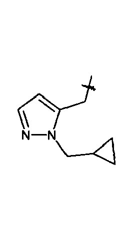 | A¹; P1 | (5*R*,7*S*)-8-{[1-(cyclopropylmethyl) -1H-pyrazol-5-yl]methyl}-1-(3-fluorophenyl)-3,7-dimethyl-2-thia-1,3,8-triazaspiro[4.5]deca ne 2,2-dioxide | 1.03 (d, *J*=6.8 Hz, 3H), 1.28-1.34 (m, 2H), 1.63 (dd, *J*=12.7, 5.1 Hz, 1H), 1.86-1.91 (m, 2H), 1.96 (d, *J*=11.3 Hz, 2H), 2.28-2.36 (m, 2H), 2.50-2 57 (m, 2H), 2.77-2.83 (m, 2H), 2.92-2 98 (q, 1H), 3.24 (d, *J*=9.0 Hz, 1H), 3.37 (t, *J*=7.6 Hz, 1H), 3.43 (d, *J*=9.2 Hz, 1H), 3.47 (s, 3H), 3.85 (dd, *J*=8.6, 7.0 Hz, 1H), 6.01 (d, *J*=1.8 Hz, 1H), 7.09-7.18 (m, 3H), 7.35-7.41(m, 2H); 3.75, 14 36, 14.62, 21.35, 43.85, 48.77, 51.61, 53.68, 60.67, 106.97, 171.65; APCI 448.4 (M+1). |
| 12 | Me | 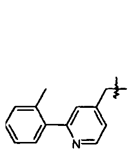 | A³, P1 | (5*R*,7*S*)-1-(3-fluorophenyl)-3,7-dimethyl-8-{[2-(2-methylphenyl)pyridi n-4-yl]methyl}-2-thia-1,3,8-triazaspiro[4.5]deca ne 2,2-dioxide | 1.04 (d, *J*=6.6Hz, 3H), 1.35 (t, *J*=7.2 Hz, 1 H), 1.65 (dd,*J*=4 5, 13.5 Hz, 1H), 1.92-1.98 (m, 2H), 1.77 (s, 3H), 2.30-2.35 (m, 1H), 2.53-2.61 (m, 1H), 2.81 (s, 3H), 3.25 (d, *J*=9.2Hz, 1H), 3.37 (d, *J*=14.7 Hz, 1H), 343 (d, *J*=9.2 Hz, 1H), 3.64 (d, *J*=14.5 Hz, 1H), 7.01-7.21 (m, 4H), 7.22-7.31 (m, 4H), 7.32-7.41 (m, 1H), 8.55 (d, *J*=4.9 Hz, 1H); 14.35, 15.64, 33.24, 33.50, 34.53, 42.29, 44.88, 51.87, 52.13, 57.30, 60.66, 61.70, 117.32, 120.95,121.72, 124 05, 126.12, 128.45, 129.74, 131.04, 140.61, 148.37, 149.31; APCI 495.4 (M+1). |
| 13 | H | 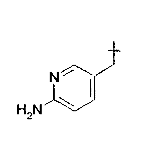 | A⁴; P2 | 5-{[(5*R*,7*S*)-1-(3-fluorophenyl)-7-methyl-2,2-dioxido-2-thia-1,3,8-triazaspiro[4.5]dec-8-yl]methyl}pyridin-2-amine | 1.10 (d, *J*=6.8 Hz, 3H), 1.78-1.93 (m, 2H), 1 94-2.03 (m, 1 H), 2.4-2.51 (m, 1H), 2.71-2.82 (m, 1H), 3.25 (d, *J*=12.9 Hz, 1 H), 3 41-3.5 (m, 2H), 3.54-3 67 (m, 1 H), 6.42 (d, *J*=8 3Hz, 1H), 7 13 (d, 1H), 7.15-7.21 (m, 2H), 7.32 (d, *J*=9.1 Hz, 1H), 7.35-7.42 (m, 1H), 7.8 (br s, 1 H); APCI 406.0 (M+1) |
| 14 | H | 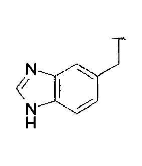 | A; P2 | (5*R*,7*S*)-8-(1H-benzimidazol-5-ylmethyl)-1-(3-fluorophenyl)-7-methyl-2-thia-1,3,8-triazaspiro[4.5]deca ne 2,2-dioxide | (400 MHz, METHANOL-d4) d ppm 1.10 -1 19 (m, 4H) 1.54 - 1.67 (m, 1H) 2.00 -2 13 (m, 2H) 2.17 - 2.30 (m, 1H) 2.45 -2.53 (m, 1H) 2.56 - 2.66 (m, 1H) 3.32 -3 36 (m, 1H) 3.40 - 3.48 (m, 1H) 3.55 -3.68 (m, 1H) 3.73 - 3.83 (m, 1H) 6.92 -7.22 (m, 6H) 7.34 - 7.56 (m, 2H) 8 14 (s, 1 H); 430.2 (M+1) |
| 15 | Me | 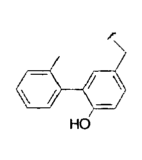 | A⁵; P1 | 5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol | 1.03 (d, *J*=6.6 Hz, 3H) 1.49 - 1.69 (m, 2H) 1.84 - 2.07 (m, 2H) 2 12 (s, 3H) 2.25 - 2.38 (m, 1H) 2.47 - 2.62 (m, 1H) 2.70 -2.85 (m, 4H) 3.16 - 3.33 (m, 2H) 3.43 (d, *J*=9.1 Hz, 1H) 3.55 (d, *J*=13.2 Hz, 1H) 4.72 (br. s., 1H) 6.87 (d, *J*=8.3 Hz, 1H) 6.94 (d, *J*=2.1 Hz, 1 H) 7.04 - 7.43 (m, 10 H); 510 3 (M+1). |
| 16 | Me | 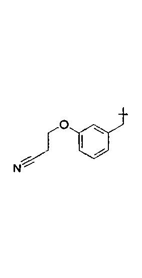 | A⁶, P1 | 3-(3-{[(5*R*,7*S*)-1-(3-fluorophenyl)-3,7-dimethyl-2,2-dioxido-2-thia-1.3,8-triazaspiro[4.5]dec-8-yl]methyl}phenoxy) propanenitrile | 1.08 (d, *J*=6.6Hz, 3H) 1.63 (dd, *J*=13.8, 7.1 Hz, 1H) 1.89 (d, *J*=8.6Hz, 1H) 2.00 -2.10 (m, 2H) 2.20 - 2.28 (m, 1H) 2.54 -2.64 (m, 2H) 2.75 (s, 3H) 2.88 (t, *J*=6.0 Hz, 2H) 3.22 - 3.30 (m, 1H) 3.39 (d, *J*=13.3 Hz, 1H) 3.49 (d, *J*=9.6Hz, 1H) 3.59 (d, *J*=13.5 Hz, 1H) 4.12 (t, *J*=6.1 Hz, 2H) 6.75 - 6.84 (m, 3H) 7.08 - 7.22 (m, 4H) 7.35 - 7.43 (m, 1H); 17.72, 31.95, 34 02, 34.28, 42 04, 51 10, 57.05, 60.67, 61.70, 62.74, 63.00, 113.18, 113.44, 115.25, 115.51, 116.29, 116.55, 120.17, 122.24, 122.50, 129.22, 129.74, 158.20; 473.3 (M+1). |
| 17 | H | 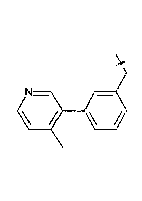 | A⁷, P2 | (5*R*,7*S*)-1-(3-fluorophenyl)-7-methyl-8-[3-(4-methylpyridin-3-yl)benzyl]-2-thia-1,3,8-triazaspiro[4.5]deca ne 2,2-dioxide | 1.04 (d, *J*=6.64 Hz, 3 H) 1.61 (dd, *J*=13.27, 5.81 Hz, 1 H) 1.79 - 1.88 (m, 1 H) 1.95 (dd, *J*=8.50, 3.94 Hz, 1 H) 1.99 (d, *J*=4.56 Hz, 1 H) 2.02 (d, *J*=4.15 Hz, 1 H) 2.23 (s, 3 H) 2.25 - 2.32 (m, 1 H) 2.49 - 2.59 (m, 1 H) 2.68 - 2.75 (m, 1 H) 3.32 (d, *J*=13.69 Hz, 1 H) 3.51 (dd, 2 H) 3.69 (d, *J*=13.69 Hz, 1 H) 7.08 - 7.27 (m, 7 H) 7.31 - 7.41 (m, 2 H) 8.38 (s, 1 H) 8.42 (d, *J*=4.98 Hz, 1 H); MS m/z 481.2 (M+1). |
| 18 | H | 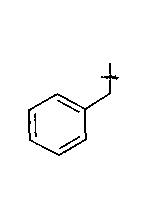 | A; P2 | (5*R*,7*S*)-8-benzyl-1-(3-fluorophenyl)-7-methyl-2-thia-1,3,8-triazaspiro[4.5]deca ne 2,2-dioxide | 1.04 (d, *J*=6.2 Hz, 3H), 1.21 (t, *J*=7.1 Hz, 1 H), 1.60 (dd, *J*=5.8. 13.7 Hz, 1H), 1.77-1.86 (m, 1H), 1.90-2.05 (m, 2H), 2.22-2.30 (m, 1H), 2.46-2.54 (m, 1H), 2.65-2.75 (m, 1 H), 3.30 (d, *J*=13.3 Hz, 1 H), 3.51 (d, *J*=14.5 Hz, 1H), 3.63 (d, *J*=13.7 Hz, 1H), 4.96 (br s, 1H), 7.08-7.28 (m, 8H), 7.33-7.40 (q, 1H); 16.09, 34.05, 41.97, 44.61, 51.21, 51.48, 53.59, 53.86, 58.08, 116.71, 116.98, 120.41, 127.28, 128.33, 128.86, 129.13, 130 45; 390.1 (M+1). |
| 19 | H | 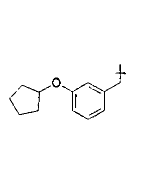 | A; P2 | (5*R*,7*S*)-8-[3-(cyclopentyloxy)be nzyl]-1-(3-fluorophenyl)-7-methyl-2-thia-1,3,8-triazaspiro[4.5]deca ne 2,2-dioxide | 1.04 (d, *J*=6.6 Hz, 3H) 1.54 - 1 66 (m, 3H) 1 71 - 1.91 (m, 7 H) 1.92 - 2.06 (m, 2H) 2 21 - 2.32 (m, 1 H) 2.46 - 2 57 (m, 1H) 2 66 - 2.77 (m, 1H) 3.28 (d, *J*=13.7 Hz, 1H) 3.44 - 3.57 (m, 2H) 3 59 (d, *J*=13.3 Hz, 1 H) 4.65 - 4.73 (m, 1 H) 4.80 (br s., 1H) 6.73 (d, *J*=5.81Hz, 3H) 7.07 -7.19 (m, 4H) 7.32 - 7.42 (m, 1 H); 474 2 (M+1). |
| 20 | H | 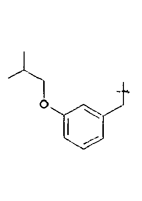 | A; P2 | (5*R*,7*S*)-1-(3-fluorophenyl)-8-(3-isobutoxybenzyl)-7-methyl-2-thia-1,3,8-triazaspiro[4.5]deca ne 2,2-dioxide | 1.00 (d, *J*=6 6 Hz, 6H) 1.04 (d, *J*=6 6 Hz, 3H) 1.61 (dd, *J*=13.3, 6.2 Hz, 1H) 1.77 -1.86 (m, 1H) 1.93 - 2.11 (m, 3H) 2.22 -2.31 (m, 1 H) 2 47 - 2.56 (m, 1 H) 2.66 -2.75 (m, 1H) 3.26 (d, J*=*13.7 Hz, 1H) 3 45 - 3.58 (m, 2H) 3.61 (d, *J*=13.3 Hz, 1H) 3.66 (d, *J*=6.6Hz, 2H) 4.78 (t, *J*=7.5 Hz, 1 H) 6 74 (s, 2H) 6.76 (s, 1H) 7.08 - 7.15 (m, 2H) 7.17 (d, *J*=8.3 Hz, 2H) 7.33 -7.41 (m, 1H); 462.6 (M+1). |
| 21 | H | 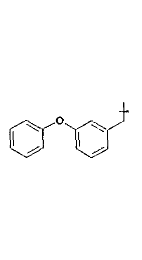 | A; P2 | (5*R*,7*S*)-1-(3-fluorophenyl)-7-methyl-8-(3-phenoxybenzyl)-2-thia-1,3,8-triazaspiro[4.5]deca ne 2,2-dioxide | 1.02 (d, *J*=6.6 Hz, 3H) 1.55 - 1.65 (m, 1 H) 1.77 - 1.88 (m, 1 H) 1.93 (dd, *J*=8 5, 3.9 Hz, 1H) 1.97 (d, *J*=1.6 Hz, 1H) 2.00 (d, *J*=4.6Hz, 1 H) 2.22 - 2 33 (m, 1 H) 2.46 - 2.56 (m, 1H) 2.67 - 2.77 (m, 1H) 3.29 (d, *J*=13.7 Hz, 1H) 3.44 - 3.58 (m, 2H) 3.61 (d, *J*=13.69 Hz, 1 H) 4.76 (t, *J*=7.9 Hz, 1 H) 6 85 (dd, *J*=7.9, 2.1 Hz, 1 H) 6 90 (s, 1H) 6 93 (d, *J*=7.5 Hz, 1H) 6.96 (d, *J*=7.5 Hz, 1H) 7.05 - 7.14 (m, 2H) 7 16 (dd, *J*=8.3. 2.1 Hz, 2H) 7.21 (t, *J*=7.9 Hz, 1H) 7.28 - 7.34 (m, 2H) 7.35 - 7.43 (m, 1H); 482.5 (M+1) 480.5 (M-1). |
| 22 | H | 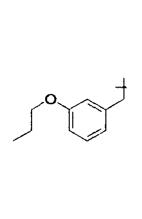 | A; P2 | (5*R*,7*S*)-1-(3-fluorophenyl)-7-methyl-8-(3-propoxybenzyl)-2-thia-1,3,8-triazaspiro[4.5]deca ne 2,2-dioxide | 1.03 (d, *J*=5.4 Hz, 3H) 1.04 (d, *J*=5.0 Hz, 3H) 1 60 (dd, *J*=13.7, 5.8 Hz, 1H) 1.73 -1.86 (m, 3H) 1.95 (dd, *J*=8.5, 3.9 Hz, 1H) 2.01 (dd, *J*=13.3, 4.6Hz, 1H) 2.22 - 2.30 (m, 1H) 2.47 - 2.56 (m, 1H) 2.66 - 2.74 (m, 1H) 3.27 (d, *J*=13.3 Hz, 1H) 3.43 -3.57 (m, 2H) 3.60 (d, *J*=13.7 Hz, 1H) 3 87 (t, *J*=6.6Hz, 2H) 4.80 (t, *J*=7.5 Hz, 1H) 6.73 - 6.77 (m, 3H) 7.08 - 7.13 (m, 1H) 7.13 - 7.18 (m, 3H) 7.33 - 7.40 (m, 1H); 448.5 (M+1) 446.5 (M-1). |
| 23 | H | 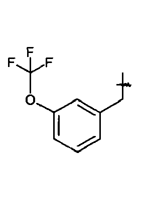 | A; P2 | (5*R*,7*S*)-1-(3-fluorophenyl)-7-methyl-8-[3-(trifluoromethoxy)b enzyl]-2-thia-1,3,8-triazaspiro[4.5]deca ne 2,2-dioxide | 1.03 (d, *J*=6.6 Hz, 3H) 1.57 - 1.61 (m, 1H) 1 62 (dd, *J*=5.8, 1.3 Hz, 1H) 1.78 -1.86 (m, 1H) 1.95 (dd, *J*=8.3, 4.2 Hz, 1H) 1.98 - 2.05 (m, 1H) 2.19 - 2.26 (m, 1 H) 2.46 - 2.54 (m, 1H) 2.64 - 2.73 (m, 1H) 3.30 (d, *J*=14.1 Hz, 1H) 3.44 - 3.57 (m, 2H) 3.64 (d, *J*=14.1 Hz, 1H) 4.82 (t, *J*=8.1 Hz, 1H) 7.03 - 7.19 (m, 5 H) 7.26 - 7.30 (m, 1H) 7.33 - 7.41 (m, 1H); 474.5 (M+1) 472.4 (M-1). |
| 24 | H | 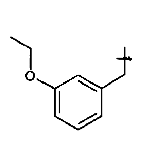 | A; P2 | (5*R*,7*S*)-8-(3-ethoxybenzyl)-1-(3-fluorophenyl)-7-methyl-2-thia-1,3,8-triazaspiro[4.5]deca ne 2,2-dioxide | 1.04 (d, *J*=6.6 Hz, 3H) 1.39 (t, *J*=7.1 Hz, 3H) 1.56 - 1.64 (m, 2H) 1.77 - 1.87 (m, 1H) 1.95 (dd, *J*=8.5, 3.9Hz, 1H) 2.01 (dd, *J*=13.5, 4.8 Hz, 1 H) 2.22 - 2.31 (m, 1H) 2.47 - 2.56 (m, 1H) 2.65 - 2.75 (m, 1H) 3.27 (d, *J*=13.7 Hz, 1H) 3 44 - 3.57 (m, 2H) 3.61 (d, *J*=13.7 Hz, 1H) 3.98 (q, *J*=7.1 Hz, 2H) 4 75 (t, *J*=8.1 Hz, 1H) 6.72- 6.78 (m, 3H) 7.11 (dd, *J*=9.8, 1 9 Hz, 1H) 713 - 7.19 (m, 2H) 7.33 - 7.42 (m, 1H); 434.5 (M+1). |
| 25 | H | 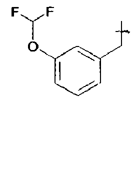 | A, P2 | (5*R*,7*S*)-8-[3-(difluoromethoxy)b enzyl]-1-(3-fluorophenyl)-7-methyl-2-thia-1,3,8-triazaspiro[4.5]deca ne 2,2-dioxide | 1.04 (d, *J*=6 6 Hz, 3H) 1.61 (dd, *J*=13.3, 5.8 Hz, 2H) 1.77 - 1.88 (m, 1H) 1.96 (dd, *J*=8.1, 3.9 Hz, 1H) 1.99 - 206 (m, 1H) 2.18 - 2.29 (m, 1H) 2 46 - 2 56 (m, 1H) 2.63 - 2.74 (m, 1 H) 3 30 (d, *J*=13.7 Hz, 1H) 3.45 - 3.58 (m, 2H) 3.64 (d, *J*=13.7 Hz, 1H) 4.76 (t, *J*=8.1 Hz, 1H) 648 (t, *J*=7.4 Hz, 1H) 6.93 - 7.00 (m, 2H) 7.04 (d, *J*=7.9 Hz, 1H) 7.11 (dd, *J*=9.5, 2.1 Hz, 1H) 7 14 - 7.20 (m, 1H) 7.21 - 7.29 (m, 1H) 7 33 - 7.43 (m, 1H); 456.5 (M+1) 454 (M-1). |
| 26 | H | 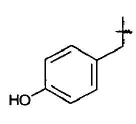 | A; P2 | 4-{[(5*R*,7*S*)-1-(3-fluorophenyl)-7-methyl-2,2-dioxido-2-thia-1,3,8-triazaspiro[4.5]dec-8-yl]methyl}phenol | 406 1 (M+1) 404.1 (M-1) |
| 27 | H | 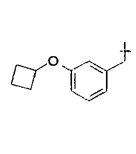 | A⁸, P2 | (5*R*,7*S*)-8-[3-(cyclobutyloxy)benz yl]-1-(3-fluorophenyl)-7-methyl-2-thia-1,3,8-triazaspiro[4.5]deca ne 2,2-dioxide | 1.04 (d, *J*=6.6 Hz, 3H) 1.56 - 1 65 (m, 2H) 1.77 - 1.88 (m, 2H) 1.92 - 2.00 (m, 2H) 2.06 - 2.16 (m, 2H) 2.24 - 2.31 (m, 1 H) 2.33 - 2.43 (m, 2H) 2.47 - 2.55 (m, 1H) 2.68 - 2.76 (m, 1H) 3.28 (d, *J*=13.7 Hz, 1H) 3.45 - 3 50 (m, 1H) 3.51 - 3.56 (m, 1H) 3.59 (d, *J*=13.7 Hz, 1H) 4.53 -4 62 (m, 1H) 4.71 (t, *J*=8.1 Hz, 1H) 6 65 (dd, *J*=8.3, 2.49 Hz, 1 H) 6.68 (s, 1H) 6 74 (d, *J*=7.5 Hz, 1H) 7.07 - 7 18 (m, 4H) 7 33 - 7.41 (m, 1H); 460.1 (M+1). |
| 28 | H | 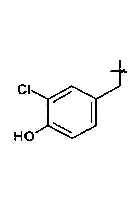 | A, P2 | 2-chloro-4-{(5*R*,7*S*)-1-(3-fluorophenyl)-7-methyl-2,2-dioxido-2-thia-1,3,8-triazaspiro[4.5]dec-8-yl]methyl}phenol | 1.07 (d, *J*=6.6 Hz, 3H), 1.66 (dd, =7.05, 13.7 Hz, 1H), 1.82-1.90 (m, 1H), 1.99-2.04 (m, 1 H), 2.05-2.07 (m, 2H), 2.19-2.27 (m, 1 H), 2.50-2.57 (m, 1 H), 2.58-2.66 (m, 1H), 3.30 (d, *J*=13.7 Hz, 1H), 3.46-3.49 (m, 2H), 3.57 (d, *J*=136 Hz, 1H), 6.90 (d, J =16.0, 8.2 Hz, 1H), 6.97 (d, *J* = 8 3 Hz, 1 H), 7.04-7.09 (m, 2H), 7.10-7.17 (m, 2H), 7.31-7.38 (m, 1H); 440 0 (M+1) |
| 29 | H | 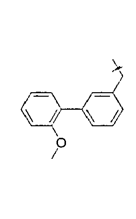 | E; P3 | (5*R*,7*S*)-1-(3-fluorophenyl)-8-[(2'-methoxybiphenyl-3-yl)methyl]-7-methyl-2-thia-1,3,8-triazaspiro[4.5]deca ne 2,2-dioxide | 1.05 (d, *J*=6.6 Hz, 3H) 1.59 (dd, *J*=13.3, 6.3 Hz, 2H) 1.77 - 1.87 (m, 1H) 1.92 -2.05 (m, 1H) 2.27 - 2.38 (m, 1H) 2.51 -2.60 (m, 1H) 2.67 - 2.77 (m, 1H) 3.37 (d, *J*=13.5 Hz, 1H) 3.47 - 3.57 (m, 2H) 3.67 (d, *J*=13.5 Hz, 1H) 3.75 (s, 3H) 4.66 (br. s., 1H) 6.93 - 6.97 (m, 1H) 7.00 (dt, *J*=7.5, 1.07 Hz, 1H) 7.08 (dd, *J*=8.6, 1.4 Hz, 2H) 7.10 - 7 15 (m, 2H) 7.25 (dd, *J*=7.4, 1.56 Hz, 2H) 7.27 - 7.33 (m, 2H) 7.33 - 7.40 (m, 2H); 496.1 (M+1). |
| 30 | H | 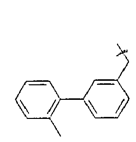 | E; P3 | (5*R*,7*S*)-1-(3-fluorophenyl)-7-methyl-8-[(2'-methylbiphenyl-3-yl)methyl]-2-thia-1,3,8-triazaspiro[4.5]deca ne 2,2-dioxide | 1.06 (d, *J*=6 6 Hz, 3H) 1.61 (dd, *J*=13.5, 5.6 Hz, 2H) 1.86 (br. s., 1 H) 1.94 - 2.06 (m, 2H) 2.22 (s, 3H) 2.27 - 2.34 (m, 1H) 2.52 - 2.61 (m, 1 H) 2.69 - 2.78 (m, 1H) 3.34 (d, *J*=13.3 Hz, 1H) 3.45 - 3.60 (m, 2H) 3.70 (d, *J*=13.3 Hz, 1H) 4.68 (t, *J*=8.1 Hz, 1 H) 7.08 - 7 15 (m, 2H) 7.18 (t, *J*=7.3 Hz, 5 H) 7.25 (d, *J*=2.1 Hz, 2H) 7.28 -7.34 (m, 1H) 733 - 7.42 (m, 1H); 480.1 (M+1). |
| 31 | H | 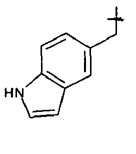 | A; P2 | (5*R*,7*S*)-1-(3-fluorophenyl)-8-(1H-indol-5-ylmethyl)-7-methyl-2-thia-1,3,8-triazaspiro[4.5]deca ne 2,2-dioxide | 1.24 - 1.31 (m, 3H) 1 87 - 1.96 (m, 1H) 2.07 - 2.16 (m, 2H) 2.24 (s, 1H) 2.32 -2.42 (m, 1H) 2.63 - 2.72 (m, 1H) 2.76 -2.88 (m, 1H) 3.41 - 3.48 (m, 2H) 3 74 -3.83 (m, 1H) 3.87 - 3 96 (m, 1H) 5.17 (br. s., 1H) 6.50 (br. s , 1H) 6.85 (t, *J*=7.7 Hz, 1 H) 6.89 - 7.01 (m, 3H) 7.13 - 7.24 (m, 1 H) 7 32 (d, *J*=8.3 Hz, 1H) 7.36 (s, 1H) 8.33 (br. s., 1H); 429.1 (M+1). |
| 32 | H | 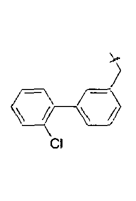 | E; P3 | (5*R*,7*S*)-8-[(2'-chlorobiphenyl-3-yl)methyl]-1-(3-fluorophenyl)-7-methyl-2-thia-1,3,8-triazaspiro[4.5]deca ne 2,2-dioxide | 1.07 (d, *J*=6.6 Hz, 3H) 1.64 (d, *J*=6.6 Hz, 1H) 1.79 - 1.90 (m, 1H) 1.96 - 2.07 (m, 1H) 2.26 - 2.37 (m, 1H) 2.54 - 2.62 (m, 1H) 2.69 - 2.78 (m, 2H) 3.36 (d, *J*=13.3 Hz, 1H) 3.45 - 3.61 (m, 2H) 3.72 (d, *J*=13.3 Hz, 1H) 4.62 (t, *J*=8.3 Hz, 1H) 7.09 - 7.15 (m, 2H) 7.18 (d, *J*=8.3 Hz, 1H) 7.21 (d, *J*=7.1 Hz, 1 H) 7.27 - 7.33 (m, 5 H) 7.33 - 7.42 (m, 2H) 7.46 (d, *J*=7.1 Hz, 1H); 500.2 (M+1). |
| 33 | H | 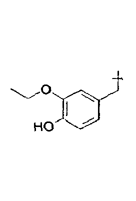 | A, P2 | 2-ethoxy-4-{[(5*R*,7*S*)-1-(3-fluorophenyl)-7-methyl-2,2-dioxido-2-thia-1,3,8-triazaspiro[4.5]dec-8-yl]methyl}phenol | 1.15 (d, *J*=6.6 Hz, 3H) 1.43 (t, *J*=7.1 Hz, 3H) 1.77 (dd, *J*=14.1, 7.5 Hz, 1H) 1.92 -2.02 (m, 1H) 2.10 (d, *J*=4.15 Hz, 1H) 2.29 (dt, *J*=12 4, 8.9, 3.5 Hz, 1H) 2.59 - 2.73 (m, 2H) 3 38 - 3.57 (m, 3H) 3.67 (d, *J*=13.3 Hz, 1H) 4.03 (q, *J*=7.1 Hz, 2H) 5.52 (br. s., 1H) 6 62 (d, *J*=7.9 Hz, 1H) 6.67 (d, *J*=2.1 Hz, 1H) 6.81 (d, *J*=8.3 Hz, 1H) 7.01 - 7.16 (m, 3H) 7.27 - 7.35 (m, 1H); 449.9 (M+1). |
| 34 | H | 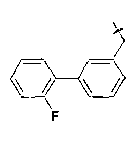 | E; P3 | (5*R*,7*S*)-8-[(2'-fluorobiphenyl-3-yl)methyl]-1-(3-fluorophenyl)-7-methyl-2-thia-1,3,8-triazaspiro[4.5]deca ne 2,2-dioxide | 484.3 (M+1) |
| 35 | H | 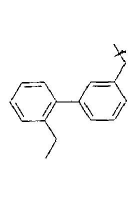 | E, P3 | (5*R*,7*S*)-8-[(2'-ethylbiphenyl-3-yl)methyl]-1-(3-fluorophenyl)-7-methyl-2-thia-1,3,8-triazaspiro[4 5]deca ne 2,2-dioxide | 1.10 (t, *J*=7.5 Hz, 3H) 1.55 (d, *J*=5.0 Hz, 3H) 2.35 - 2.46 (m, 3H) 2.46 - 2.60 (m, 4H) 2.80 (br. s., 1H) 3.27 (d, *J*=9.5 Hz, 1H) 3.51 (br. s., 2H) 4.11 (d, *J*=14.5 Hz, 1H) 4.34 (d, *J*=13.7 Hz, 1H) 6.92 (d, *J*=8.3 Hz, 1H) 6.97 (d, *J*=7.9 Hz, 1H) 7.06 (t, *J*=7.9 Hz, 1 H) 7.12 (d, *J*=7 5 Hz, 2H) 7.17 (br. s., 2H) 7.20 - 7.24 (m, 1H) 7.30 (d, *J*=7.1 Hz, 1H) 7.33 - 7.39 (m, 2H) 7.44 - 7.51 (m, 2H); 494.2 (M+1). |
| 36 | H | 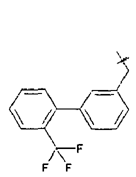 | E, P3 | (5*R*,7*S*)-1-(3-fluorophenyl)-7-methyl-8-{[2'-(trifluoromethyl)bip henyl-3-yl]methyl}-2-thia-1,3,8-triazaspiro[4.5]deca ne 2,2-dioxide | 1.06 (d, *J*=6.6 Hz, 3H) 1.63 (d, *J*=5.8 Hz, 1H) 1.79 - 1.89 (m, 1H) 1.94 - 2.07 (m, 2H) 2.24 - 2.33 (m, 1H) 2.50 - 2.60 (m, 1H) 2.67 - 2.77 (m, 1H) 3.32 (d, *J*=13 3 Hz, 1H) 3.44 - 3.60 (m, 2H) 3.71 (d, *J*=13.3 Hz, 1H) 4.70 (t, *J*=8.1 Hz, 1H) 7.10 - 7.15 (m, 2H) 7.15 - 7.24 (m, 4H) 7.28 - 7.34 (m, 2H) 7.38 (q, *J*=7.1 Hz, 1 H) 7.47 (t, *J*=7.7 Hz, 1 H) 7.55 (t, *J*=7.5 Hz, 1H) 7.74 (d, *J*=7.5 Hz, 1H); 534.2 (M+1). |
| 37 | H | 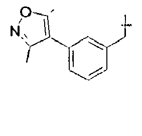 | E; P3 | (5*R*,7*S*)-8-[3-(3.5-dimethylisoxazol-4-yl)benzyl]-1-(3-fluorophenyl)-7-methyl-2-thia-1,3,8-triazaspiro[4.5]deca ne 2,2-dioxide | 1.55 (d, *J*=3.7 Hz, 3H) 2.27 (s, 3H) 2.41 (s, 3H) 2.43 - 2.56 (m, 4H) 2.74 (s, 1H) 2.84 (br s., 1H) 3.27 (br. s., 1H) 3.52 (br. s., 2H) 3.98 (d, *J*=14.1 Hz, 1H) 4.46 (d, *J*=14.1 Hz, 1H) 509 (br. s., 1 H) 6.93 (d, *J*=8.3 Hz, 1H) 7.05 (d, *J*=7.5 Hz, 1H) 7.07 - 7.14 (m, 1H) 7.14 - 7.23 (m, 2H) 7.30 (d, *J*=9.1 Hz, 1H) 7.42 (d, *J*=9.1 Hz, 1H) 7.52 (t, *J*=8.5 Hz, 1H); 485.2 (M+1). |
| 38 | H | 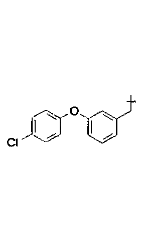 | A, P2 | (5*R*,7*S*)-8-[3-(4-chlorophenoxy)ben zyl]-1-(3-fluorophenyl)-7-methyl-2-thia-1,3,8-triazaspiro[4.5]deca ne 2,2-dioxide | 1.03 (d, *J*=6.6Hz, 3H), 1.62 (dd, *J*=13.7, 5.8 Hz, 1H), 1.78-1.88 (m, 1H), 1.90-1.97 (m, 1H), 1.97-2.03 (m, 1H), 2.23-2.32 (m, 1H), 2.47-2.57 (m, 1H), 2.67-2.77 (m, 1H), 3.29 (d, *J*=13.7 Hz, 1H), 3.43-3.58 (m, 2H), 3.62 (d, *J*=13.7 Hz, 1H), 4.89 (t, *J*=8.1 Hz, 1H) 6.84 (dd, *J*=8.1, 1.9 Hz, 1H), 6.86-6.93 (m, 3H), 6.96 (d, *J*=7.5 Hz, 1H), 7.08-7.15 (m, 1H), 7.17 (dd, *J*=8.3, 2.1 Hz, 2H), 7.20-7.24 (m, 1H), 7.24-7.29 (m, 2H), 7.35-7.43 (m, 1H), 551.6 (M+1); 516.2 (M+1), 514.2 (M-1). |
| 39 | H | 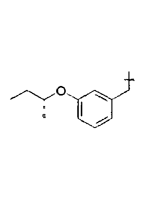 | A⁹, P2 | (5*R*,7*S*)-1-(3-fluorophenyl)-7-methyl-8-(3-{[(1R)-1-methylpropyl]oxy}b enzyl)-2-thia-1,3,8-triazaspiro[4.5]deca ne 2,2-dioxide | 0.96 (t, *J*=7.5 Hz, 3H) 1.10 (d, *J*=6.6 Hz, 3H) 1.27 (d, J*=*5.8 Hz, 3H) 1.55 - 1.64 (m, 1H) 1.65 - 1.78 (m, 2H) 1.85 - 1.94 (m, 1H) 1.96 - 2.10 (m, 2H) 2.25 - 2.37 (m, 1 H) 2.53 - 2.65 (m, 1H) 2.70 - 2.80 (m, 1H) 3.35 (d, *J*=13.3 Hz, 1H) 3.45 -3.60 (m, 2H) 3 66 (d, *J*=13.7 Hz, 1H) 4.19 - 4.32 (m, 1H) 4.99 (br. s., 1H) 6.71 -6.81 (m, 3H) 7.05 - 7.22 (m, 4H) 7.29 -7 43 (m, 1H)/462.2 (M+1). |
| 40 | Me | 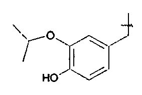 | A¹⁰; P1 | 4-{[(5*R*,7*S*)-1-(3-fluorophenyl)-3,7-dimethyl-2,2-dioxido-2-thia-1,3,8-triazaspiro[4.5]dec-8-yl]methyl}-2-isopropoxyphenol | 1.04 (d, *J*=6.6 Hz, 3H) 1.32 (d, *J*=5.8 Hz, 6H) 1.65 (dd, *J*=13.5, 5.2 Hz, 1 H) 1.93 (t, *J*=4.2 Hz, 2H) 2.03 (dd, *J*=13.5. 4.8 Hz, 1H) 2.29 - 2.37 (m, 1H) 2.48 - 2.58 (m, 1 H) 2.77 - 2.81 (m, 1 H) 2.83 (s, 3H) 3.28 (d, *J*=22.8 Hz, 1 H) 3.26 (d, *J*=9.3 Hz, 1H) 3.29 (d, *J*=13.5 Hz, 1 H) 3.28 (s, 1 H) 3.45 (d, *J*=9.1 Hz, 1H) 3.51 (d, J*=*13.3 Hz, 1H) 4.46 - 4.55 (m, 1 H) 5.64 (br. s., 1 H) 6.65 (d, *J*=7.9 Hz, 1H) 6.73 (s, 1H) 6.80 (d, *J*=7.9 Hz, 1H) 7.10 - 7.17 (m, 2H) (d, *J*=8.3 Hz, 1H) 7.34 - 7 42 (m, 1 H); 478.2 (M+1), 476.3 (M-1). |
| 41 | Me | 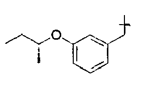 | A⁹, P1 | (5R*,*7*S*)-1-(3-fluorophenyl)-3,7-dimethyl-8-(3-{[(1R)-1-methylpropyl]oxy}b enzyl)-2-thia-1,3,8-triazaspiro[4.5]deca ne 2,2-dioxide | 0.96 (t, *J*=7.5 Hz, 3H) 1.05 (d, *J*=7.1 Hz, 3H) 1.26 (d, *J*=6.2 Hz, 3H) 1.53 - 1.79 (m, 4H) 1.91 - 1.97 (m, 2H) 2.05 (dd, *J*=13.5, 4.8 Hz, 1 H) 2 30 - 2.40 (m, 1H) 2.54 (dd, J*=*7.9, 4.6 Hz, 1H) 2.83 (s, 3H) 3.28 (t, *J*=8.5 Hz, 1 H) 3.34 (s, 1H) 3.46 (d, *J*=9.1 Hz, 1H) 3.57 (d, *J*=13.3 Hz, 1H) 4.20 - 4.29 (m, 1H) 6 74 (d, J=1.7 Hz, 1 H) 6.76 (s, 2H) 7 10 - 7.23 (m, 4H) 7.35 - 7.44 (m, 1H); 476.3 (M+1). |
| 42 | Me | 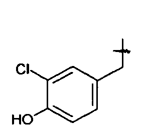 | A; P1 | 2-chloro-4-{[(5R,7S)-1-(3-fluorophenyl)-3,7-dimethyl-2,2-dioxido-2-thia-1,3,8-triazaspiro[4.5]dec-8-yl]methyl}phenol | 1.04 (d, *J*=6.6 Hz, 3H) 1.24 - 1.32 (m, 2H) 1.64 (dd, *J*=13.7, 5.4 Hz, 1H) 1.89 -1.97 (m, 1H) 2.05 (dd, *J*=13.5, 4.8 Hz, 1 H) 2.22 - 2.33 (m, 1H) 2 44 - 2.56 (m, 1H) 2.71 - 2.79 (m, 1H) 2.82 (s, 3H) 3.22 - 3.31 (m, 2H) 3.43 (d, J=9.5 Hz, 1H) 3.47 - 3.54 (m, 1 H) 5.51 (br. s., 1 H) 6.87 - 6.93 (m, 1H) 6.96 - 7.02 (m, 1H) 7.10 - 7.21 (m, 4H) 7.34 - 7.43 (m, 1H), 454.2 (M+1). |
| 43 | Me | 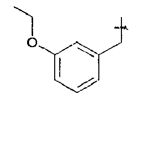 | A; P1 | (5R,7S)-8-(3-ethoxybenzyl)-1-(3-fluorophenyl)-3,7-dimethyl-2-thia-1,3,8-triazaspiro[4.5]deca ne 2,2-dioxide | 1.05 (d, *J*=6.6Hz, 3H) 1.40 (t, *J*=7.1 Hz, 3H) 1.65 (dd, *J*=13.3, 5.4 Hz, 1H) 1.91 -1.97 (m, 2H) 2.05 (dd, *J*=13.1, 5.18 Hz, 1H) 2.28 - 2.37 (m, 1H) 2 49 - 2.59 (m, 1H) 2.76 - 2.81 (m, 1H) 2 83 (s, 3H) 3.26 (d, *J*=9.1 Hz, 1H) 3.32 (d, *J*=*13.3* Hz, 1H) 345 (d, *J*=9.1 Hz, 1H) 3.57 (d, *J*=13.7 Hz, 1H) 3.99 (q, *J*=7.1 Hz, 2H) 6.73 -6 79 (m, 3H) 7.10 - 7.23 (m, 4H) 7 35 -743 (m, 1H); 448.3 (M+1). |
| 44 | Me | 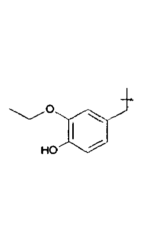 | A; P1 | 2-ethoxy-4-{[(5R,7S)-1-(3-fluorophenyl)-3,7-dimethyl-2,2-dioxdo-2-thia-1,3,8-triazaspiro[4.5]dec-8-yl]methyl}phenol | 1.01 (d, *J*=6.8 Hz, 3H) 1.38 (t, *J*=7.0 Hz, 3H) 1.61 (dd, *J*=13.6, 54 Hz, 1H) 1.87 -1.93 (m, 2H) 2.00 (dd, *J*=13.7, 4.9 Hz, 1 H) 2.23 - 2.32 (m, 1H) 2.44 - 2.53 (m, 1 H) 2.72 - 2.78 (m, 1 H) 2.79 (s, 3H) 3.22 (d, *J*=9.4 Hz, 1 H) 3.26 (d, *J*=13 3 Hz, 1 H) 3.41 (d, *J*=9.2 Hz, 1H) 3 44 - 3.50 (m, 1H) 4.01 (q, *J*=7.0 Hz, 1H) 5.58 (br. s., 1 H) 6.62 (dd, *J*=8.0, 2.0 Hz, 1 H) 6.66 (d, *J*=1.8 Hz, 1H) 6.77 (d, *J*=8.0 Hz, 1H) 7.07 - 7.18 (m, 3H) 7.31 - 7.39 (m, 1H); 464.3 (M+1) |
| 45 | Me | 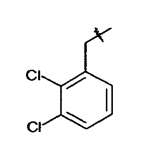 | A; P1 | (5R,7S)-8-(2,3-dichlorobenzyl)-1-(3-fluorophenyl)-3,7-dimethyl-2-thia-1,3,8-triazaspiro[4.5]deca ne 2,2-dioxide | 1.05 (d, *J*=6.8 Hz, 3H) 1.62 - 1.69 (m, 1H) 1.88 - 1.94 (m, 2H) 1.98 - 2.06 (m, 1H) 2.31 - 2.40 (m, 1H) 2.53 - 2.61 (m, 1H) 2.80 (s, 3H) 2.87 - 2.94 (m, 1H) 3.26 (d, *J*=9.2 Hz, 1H) 3.41 - 3.49 (m, 2H) 3.65 (d, *J*=15.0 Hz, 1H) 7.07 - 7.26 (m, 5 H)7.30 (dd, *J*=7.9, 1.7 Hz, 1H) 7.36 -7 44 (m, 1H); 472.1 (M+1). |
| 46 | Me | 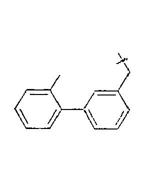 | A; P1 | (5R,75)-1-(3-fluorophenyl-3,7-dimethyl-8-[(2'-methylbiphenyl-3-yl)methyl]-2-thia-1,3,8-triazaspiro[4.5]deca ne 2,2-dioxide | 1.01 - 1.06 (m, 3H) 1.23 - 1.25 (m, 1H) 1.63 (dd, *J*=13.9, 5.3 Hz, 1H) 1 89 - 1.94 (m, 2H) 1.98 - 2.05 (m, 1H) 2.20 (s, 3H) 2.30 - 2.38 (m, 1H) 2.51 - 2.60 (m, 1H) 2.80 (s, 3H) 2.82 - 2.86 (m, 1H) 3.24 (d, *J*=9.2 Hz, 1H) 3.36 (d, *J*=13.5 Hz, 1H) 3.44 (d, *J*=9.2 Hz, 1H) 3.62 (d, *J*=13.5 Hz, 1H) 7.08 - 7.40 (m, 12H); 494.3 (M+1). |
| 47 | Me | 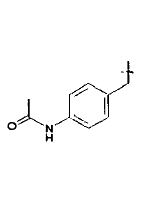 | A; P1 | N-(4-{[(5R,7S)-1-(3-fluorophenyl)-3,7-dimethyl-2,2-dioxido-2-thia-1,3,8-triazaspiro[4.5]dec-8-yl]methyl}phenyl)ac etamide | 1.01 (d, *J*=6.8 Hz, 3H) 1.61 (dd, *J*=13.4, 5.4 Hz, 1 H) 1.87 - 1.92 (m, 1H) 2.00 (dd, *J*=13.5, 4.9 Hz, 1 H) 2.11 (s, 3H) 2 22 -2.30 (m, 1H) 2.44 - 2.53 (m, 1H) 2 70 -2.77 (m, 1H) 2.79 (s, 3H) 3.23 (d, *J*=9.2 Hz, 1H) 3.29 (d, *J*=13.5 Hz, 1H) 3.41 (d, *J*=9.2 Hz, 1H) 3.45 (q, *J*=7.0 Hz, 1H) 3.52 (d, *J*=13.5 Hz, 1H) 7.05 - 7.17 (m, 5 H) 7.26 (s, 1H) 7.31 - 7.39 (m, 3H); 461.3 (M+1) |
| 48 | Me | 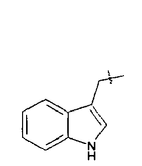 | A; P1 | (5R,7S)-1-(3-fluorophenyl)-8-(1H-indol-3-ylmethyl)-3,7-dimethyl-2-thia-1,3,8-triazaspiro[4 5]deca ne 2,2-dioxide | 1.09 (d, *J*=6.6 Hz, 3H) 1.41 (br s., 1H) 1.61 (dd, *J*=13.5, 6.8 Hz, 1H) 1 85 (d, *J*=2.9 Hz, 1H) 1.92 - 2.00 (m, 1H) 2.04 -2.12 (m, 1H) 2.26 - 2.37 (m, 1H) 2.54 -2.64 (m, 1H) 2.68 - 2.75 (m, 1H) 2.77 (s, 3H) 3.18 - 3.24 (m, 1H) 3.35 (d, *J*=9.4 Hz, 1H) 3.57 (d, *J*=13.9 Hz, 1H) 3.81 (d, *J*=13.7 Hz, 1 H) 6.94 - 7 08 (m, 5H) 7.11 - 7.21 (m, 2H) 7.31 (d, *J*=8.2 Hz, 1H) 7.49 (d, *J*=7.8 Hz, 1H); 443.3 (M+1) 441.2 (M-1). |
| 49 | Me | 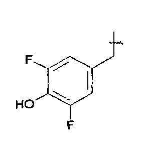 | A; P1 | 2,6-difluoro-4-{[(5R,7S)-1-(3-fluorophenyl)-3,7-dimethyl-2,2-dioxido-2-thia-1,3,8-triazaspiro[4.5]dec-8-yl]methyl}phenol | 1 03 (d, *J*=6.6 Hz, 3H) 1 65 (dd, *J*=13.7, 5.8 Hz, 1H) 1.90 - 1.97 (m, 1H) 2.00 -2.08 (m, 1H) 2.27 (dd, *J*=8.1, 4.8 Hz, 1H) 2.45 - 2.56 (m, 1H) 2.75 (dd, *J*=11 4, 5.2 Hz, 1H) 2.82 (s, 3H) 3.22 - 3.28 (m, 2H) 3 39 - 3.52 (m, 3H) 6.75 (d, *J*=8.3 Hz, 2H) 7.09 - 7.21 (m, 3H) 7.35 - 7.43 (m, 1H); 456.3 (M+1). |
| 50 | Me | 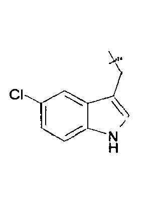 | A; P1 | (5R,7S)-8-[(5-chloro-1H-indol-3-yl)methyl]-1-(3-fluorophenyl)-3,7-dimethyl-2-thia-1,3,8-triazaspiro[4.5]deca ne 2,2-dioxide | 1 10 (d, *J*=6.5 Hz, 3H) 1.65 (dd, *J*=13.4, 6.4 Hz, 1H) 1.88 - 1.93 (m, 1H) 1.95 -1.99 (m, 1H) 2.10 (dd, *J*=13.8, 4.8 Hz, 1H) 2.23 - 2.33 (m, 1H) 2.53 - 2.61 (m, 1 H) 2.71 - 2.78 (m, 1H) 2.80 (s, 3H) 3.23 (d, *J*=9.2 Hz, 1 H) 3.38 (d, *J*=9.2 Hz, 1 H) 3.45 - 3.48 (m, 1H) 3.52 (d, *J*=13 9 Hz, 1H) 3.78 (d, *J*=13.7 Hz, 1H) 6.98 - 7.14 (m, 5 H) 7.21 - 7.30 (m, 2H) 7.49 (d, *J*=2.2 Hz, 1H); 475.3 (M-1). |
| 51 | Me | 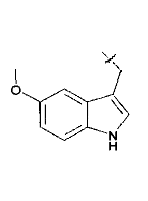 | A; P1 | (5R,7S)-1-(3-fluorophenyl)-8-[(5-methoxy-1H-indol-3-yl)methyl]-3,7-dimethyl-2-thia-1,3,8-triazaspiro[4.5]deca ne 2,2-dioxide | 1.09 (d, *J*=6.6 Hz, 3H) 1.66 (d, *J*=8.2 Hz, 1H) 1.92 - 1.98 (m, 2H) 2.06 (dd, *J*=13.7, 4.7 Hz, 1H) 2.36 - 2.44 (m, 1H) 2.59 -2.65 (m, 1H) 2.80 (s, 3H) 281 (s, 3H) 2.83 - 2.87 (m, 1H) 3.16 - 3.29 (m, 2H) 3.41 (d, *J*=9.2 Hz, 1H) 3.59 (d, *J*=13.7 Hz, 1H) 3.80 (d, *J*=13.7 Hz, 1H) 6.82 (dd, *J*=8.8, 2.54Hz, 1H) 6.98 (dd, *J*=12.7, 2.4 Hz, 2H) 7.03 - 7.13 (m, 2H) 7.18 - 7.27 (m, 3H); 473.2 (M+1). |
| 52 | Me | 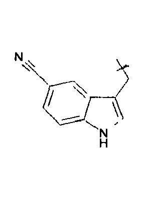 | A, P1 | 3-{[(5*R*,7*S*)-1-(3-fluorophenyl)-3,7-dimethyl-2,2-dioxido-2-thia-1,3,8-triazaspiro[4.5]dec-8-yl]methyl}-1H indole-5-carbonitrile | 1.11 (d, *J*=6.6Hz, 3H) 1 66 (dd, *J*=14.0, 6.4 Hz, 1H) 1.88 (d, *J*=3.3 Hz, 1H) 1.94 -1.99 (m, 1H) 2.08 - 2.14 (m, 1H) 2.18 -2.27 (m, 1H) 2 51 - 2.60 (m, 1H) 2.67 -2.73 (m, 1H) 2.81 (s, 3H) 3.24 (d, *J*=9.4 Hz, 1H) 338 (d, *J*=9.2 Hz, 1H) 3.50 (d, *J*=13.7 Hz, 1H) 3.84 (d, *J*=13.9 Hz, 1H) 704 - 7.16 (m, 4H) 7.26 - 7.40 (m, 3H) 7.93 (s, 1 H) 8.32 (br. s., 1H); 468.5 (M+1) 466.4 (M-1). |
| 53 | Me | 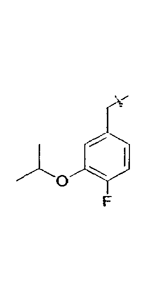 | A¹¹, P1 | (5R,7S)-8-(4-fluoro-3-isopropoxybenzyl)-1-(3-fluorophenyl)-3,7-dimethyl-2-thia-1,3,8-triazaspiro[4.5]deca ne 2,2-dioxide | 1.02 (d, *J*=6.6 Hz, 3H) 1.27 - 1.31 (m, 6H) 1.63 (dd, *J*=13.2, 5.0 Hz, 1H) 1.88 -1.94 (m, 2H) 2.01 (dd, *J*=13.6, 5.0 Hz, 1H) 2.24 - 2.33 (m, 1H) 2.47 - 2.55 (m, 1H) 2.74 - 2.79 (m, 1H) 2.81 (s, 3H) 3.22 - 3 31 (m, 2H) 3.43 (d, *J*=9 2 Hz, 1H) 3.51 (d, *J*=13.7 Hz, 1H) 4.39 - 4.48 (m, 1H) 6.65 - 6.71 (m, 1H) 6.82 (dd, *J*=8.1, 2.1 Hz, 1H) 6.92 (dd, *J*=11.1, 8.2 Hz, 1H) 7.09 - 7.20 (m, 3H) 7.34 - 7.41 (m, 1H); 22 2, 22.3, 33.4, 34.4, 42.2, 44.2, 51.5, 57.6, 60.6, 61.8, 72.5, 116.0, 116.2, 116.8, 117.0, 118.1, 120.7, 120.9, 121.4, 121.5, 129.3, 129.4, 130.3, 130.4; 480.2 (M+1). |
| 54 | iPr | 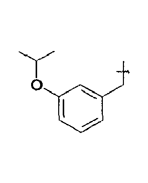 | B, Cpd 2 | (5R,7S)-1-(3-fluorophenyl)-8-(3-isopropoxybenzyl)-3-isopropyl-7-methyl-2-thia-1,3,8-triazaspiro[4.5]deca ne 2,2-dioxide | 1.03 (d, *J*=6.6 Hz, 3H) 1.28 (d, *J*=6.1 Hz, 6H) 1.31 (d, *J*=6.6 Hz, 6H) 1.55 (s, 1H) 1.62 (dd, *J*=12.5, 6.1 Hz, 1H) 1.82 - 1.89 (m, 1H) 1.94 (dd, *J*=8.2, 4.3 Hz, 1H) 2.05 (dd, *J*=14.1, 4.3 Hz, 1H) 2 22 - 2.32 (m, 1H) 2.45 - 2.56 (m, 1H) 270 - 2.76 (m, 1H) 3.23 - 3.30 (m, 2H) 3.34 - 3.41 (m, 1H) 3.58 (d, *J*=13.5 Hz, 1H) 3.75 - 3.84 (m, 1H) 4.44 - 4.52 (m, 1H) 6.72 (d, *J*=2.0 Hz, 1H) 6.73 (s, 2H) 7.09 - 7.19 (m, 4H) 7.30 - 7.39 (m, 1H); 490.3 (M+1). |
| 55 | Et | 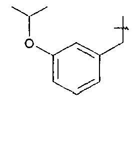 | B; Cpd 2 | (5R,7S)-3-ethyl-1-(3-fluorophenyl)-8-(3-isopropoxybenzyl)-7-methyl-2-thia-1,3,8-triazaspiro[4.5]deca ne 2,2-dioxide | 1.03 (d, *J*=6.6 Hz, 3H) 1.28 (d, *J*=6.1 Hz, 6H) 1.30 (t, *J*=7.2 Hz, 3H) 1.62 (ddd, *J*=13 6, 5.5, 1.4 Hz, 1H) 1.85 - 1.97 (m, 2H) 203 (dd, *J*=13.4, 5.0 Hz, 1H) 2.23 -2.34 (m, 1H) 2.48 - 2.57 (m, 1H) 2.73 -2.80 (m, 1H) 3.09 (dd, *J*=12.9, 7.2 Hz, 1H) 3 18 - 3.31 (m, 3H) 3.42 (d, *J*=9.2 Hz, 1H) 3.56 (d, *J*=13.5 Hz, 1H) 4.43 -4.53 (m, 1H) 6.68 - 6.76 (m, 3H) 7.06 -7.21 (m, 4H) 7.30 - 7.41 (m, 1H); 476.3 (M+1). |
| 56 | Me | 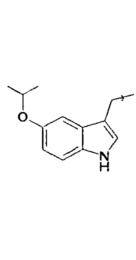 | A¹²; P1 | (5R,7S)-1-(3-fluorophenyl)-8-[(5-isopropoxy-1H-indol-3-yl)methyl]-3,7-dimethyl-2-thia-1,3,8-triazaspiro[4.5]deca ne 2,2-dioxide | 1.09 (d, *J*=6.6 Hz, 3H) 1.26 (t, *J*=6.0 Hz, 6H) 1.58 (dd, *J*=14.0, 8.3 Hz, 1H) 1 63 -1.70 (m, 1H) 1.95 (d, *J*=4.5 Hz, 2H) 2.04 - 2.09 (m, 1H) 2.34 - 2.42 (m, 1H) 2.59 (dd, *J*=12.8, 6.5 Hz, 1H) 2.79 (s, 3H) 2.81 - 2.86 (m, 1H) 3.23 (d, *J*=9.2 Hz, 1H) 3.40 (d, *J*=9.2 Hz, 1H) 3.58 (d, *J*=13.1 Hz, 1H) 3 78 (d, *J*=13.7 Hz, 1H) 4.33 -4.40 (m, 1H) 6.81 (dd, *J*=8.8, 2.34 Hz, 1H) 6.98 (dd, *J*=15.0, 2.0 Hz, 2H) 7.02 -7.11 (m, 2H) 7.20 (d, *J*=8.8 Hz, 2H) 7.23 (d, *J*=0.8 Hz, 1H) 7.91 (br. s., 1H); 501.2 (M+1). |
| 57 | Me | 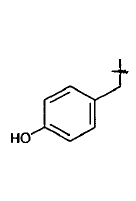 | A; P1 | 4-{[(5*R*,7*S*)-1-(3-fluorophenyl)-3,7-dimethyl-2,2-dioxido-2-thia-1,3,8-triazaspiro[4.5]dec-8-yl]methyl}phenol | 1.07 (d, *J*=6.6 Hz, 3H) 1.66 (dd, *J*=14.3, 5.7 Hz, 1H) 1.92 - 1.99 (m, 2H) 2.02 -2.10 (m, 1H) 2.29 - 2.36 (m, 1H) 2.48 -2.58 (m, 1H) 2.74 - 2.81 (m, 1H) 2.83 (s, 3H) 3.27 (d, *J*=9.3 Hz, 1H) 3.33 (d, *J*=13.2 Hz, 1H) 3.44 (d, *J*=9.3 Hz, 1H) 3 55 (d, *J*=13 4 Hz, 1 H) 5.10 (br. s., 1H) 6.72 (d, *J*=8.5 Hz, 2H) 7.05 (d, *J*=8.3 Hz, 2H) 7.10 - 7.15 (m, 1H) 7.15 - 7.21 (m, 2H) 7.34 - 7.43 (m, 1H); 420.1 (M+1). |
| 58 | Me | 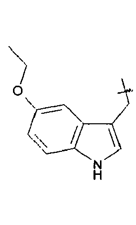 | A, P1 | (5R,7S)-8-[(5-ethoxy-1H-indol-3-yl)methyl]-1-(3-fluorophenyl)-3,7-dimethyl-2-thia-1,3,8-triazaspiro[4.5]deca ne 2,2-dioxide | 1.13 (d, *J*=6.3 Hz, 3H) 1.40 (t, *J*=7.0 Hz, 3H) 1.67 - 1 74 (m, 1H) 1.95 - 2.00 (m, 2H) 2.17 - 2.23 (m, 1H) 2.38 - 2.47 (m, 1H) 2.60 - 2.67 (m, 1H) 2.83 (s, 3H) 2.84 (s, 1H) 3.27 (d, *J*=9.3 Hz, 1H) 3.44 (d, *J*=9.3 Hz, 1H) 3 62 (d, *J*=12.9 Hz, 1H) 3.82 (d, *J*=13.4 Hz, 1H) 3.93 (qd, *J*=7.0, 2.1 Hz, 2H) 6.85 (dd, *J*=8.7, 2.3 Hz, 1H) 7.00 (d, *J*=10.3 Hz, 2H) 7.05 - 7.15 (m, 3H) 7.24 (d, *J*.8.5 Hz, 1H) 7.26 - 7.29 (m, 1H), 487.2 (M+1). |
| 59 | Me | 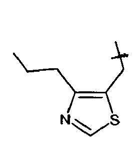 | A¹³, P1 | (5R,7S)-1-(3-fluorophenyl)-3,7-dimethyl-8-[(4-propyl-1,3-thiazol-5-yl)methyl]-2-thia-1,3,8-triazaspiro[4.5]deca ne 2,2-dioxide | 0.85 (t, *J*=7.4 Hz, 3H), 1.03 (d, *J*=6.8 Hz, 3H), 1.57-1.65 (m, 4H), 1.87-1.95 (m, 1H), 2 02 (dd, *J*=13.8, 4.6Hz, 1 H), 2.32 (dt, *J*=12.7, 5.3 Hz, 5.1 Hz, 1H), 2.51-2.60 (m, 3H), 2.80 (s, 3H), 2.81-2.87 (m, 1H), 3.23 (d, *J*=9.2 Hz, 1H), 3.48 (d, *J*=14 1 Hz, 1H), 3.71 (d, *J*=14.4 Hz, 1H), 7.08-7.20 (m, 3H). 7.33-7.41 (m, 1H), 8.58 (s, 1H); 453.3 (M+1). |
| 60 | Me | 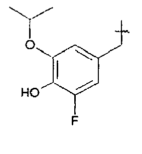 I | A¹⁴, P1 | 2-fluoro-4-{[(5R,7S)-1-(3-fluorophenyl)-3,7-dimethyl-2,2-dioxido-2-thia-1,3,8-triazaspiro[4.5]dec-8-yl]methyl}-6-isopropoxyphenol | 1.00 (d, *J*=6 6 Hz, 3H) 1.29 (d, *J*=6.1 Hz, 6H) 1.62 (dd, *J*=13.5, 5.3 Hz, 1H) 1.87 -1.93 (m, 2H) 1.97 - 2.01 (m, 1 H) 2.27 (dt, *J*=12.8, 5.2, 4.9 Hz, 1H) 2.44 - 2.54 (m, 1 H) 2.72 - 2 78 (m, 1H) 2.79 (s, 3H) 3 20 - 3.27 (m, 2H) 3.38 - 3.47 (m, 2H) 4.42 -4.53 (m, 1H) 5.39 (br. s., 1H) 6.49 (s, 1H) 6.53 (dd, *J*=10.9, 1 6 Hz, 1H) 7.07 - 7.19 (m, 3H) 7.32 - 7.40 (m, 1H); 496.4 (M+1). |
| 61 | Me | 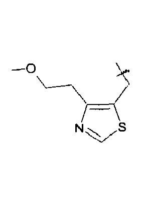 | A¹³, P1 | (5R,7S)-1-(3-fluorophenyl)-8-{[4-(2-methoxyethyl)-1,3-thiazol-5-yl]methyl}-3,7-dimethyl-2-thia-1,3,8-triazaspiro[4.5]deca ne 2,2-dioxide | 1.03 (d, *J*=6.8 Hz, 3H) 1.56 - 1.66 (m, 2H) 1.88 - 1.94 (m, 2H) 2.02 (dd, *J*=13.6, 5.0 Hz, 1H) 2.29 - 2.38 (m, 1H) 2 50 -2.60 (m, 1H) 2.80 (s, 3H) 2.88 (t, *J*=6.8 Hz, 2H) 3.23 (d, *J*=94 4 Hz, 1H) 3.25 (s, 3H) 3.41 (d, *J*=9.18 Hz, 1H) 3.50 (d, *J*=14.5 Hz, 1H) 3.60 (t, *J*=6.8 Hz, 2H) 3.73 (d, *J*=14.5 Hz, 1H) 7.07 - 7.19 (m, 3H) 7.31 - 7.41 (m, 1H) 8.58 (s, 1H); 469.3 (M+1). |
| 62 | Me | 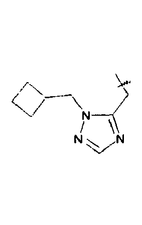 | A, D; P1 | (5*R*,7*S*)-8-{[1-(cyclobutylmethyl)-1 H-1,2,4-triazol-5-yl]methyl}-1-(3-fluorophenyl)-3,7-dimethyl-2-thia-1,3,8-triazaspiro[4.5]deca ne 2,2-dioxide | 1.08 (d, *J*=6.8 Hz, 3H) 1.60 - 1.76 (m, 5 H) 1.81 - 1.94 (m, 4H) 2.00 (dd, *J*=13.1, 4.7 Hz, 1H) 2.35 (td, *J*=12.7, 5.1, 4.9 Hz, 1H) 2.57 (td, *J*=12.7, 8.4, 4.2 Hz, 1H) 2.64 - 2 73 (m, 1H) 2.80 (s, 3H) 2.81 -2.85 (m, 1H) 3.24 (d, *J*=9.2 Hz, 1H) 3.43 (d, *J*=9.2 Hz, 1H) 3.51 (d, *J*=13.9 Hz, 1H) 3.71 (d, *J*=13.9 Hz, 1H) 4.02 (dd, *J*=7.2, 1.8 Hz, 2H) 7.07 - 7.19 (m, 3H) 7.37(td, *J*=8.1, 6.4 Hz, 1H) 7.71 (s, 1H); 463 3 (M+1). |
| 63 | Me | 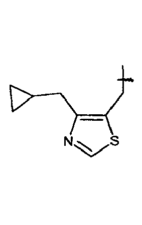 | A¹³; P1 | (5R,7S)-8-{[4-(cyclopropylmethyl) -1,3-thiazol-5-yl]methyl}-1-(3-fluorophenyl)-3,7-dimethyl-2-thia-1,3,8-triazaspiro[4.5]deca ne 2,2-dioxide | 0.08 - 0.14 (m, 2H) 0.38 - 0.45 (m, 2H) 0.95 - 1.00 (m, 1H) 1.03 (d, *J*=6 6 Hz, 3H) 1.61 (ddd, *J*=13.6, 5.4, 1.1 Hz, 1H) 1.87 - 1.94 (m, 2H) 2.02 (dd, *J*=13.3, 4.5 Hz, 1H) 2.28 - 2.36 (m, 1H) 2.54 (d, *J*=6 6 Hz, 2H) 2 55 - 2.58 (m, 1H) 2.79 (s, 3H) 2.80 - 2.86 (m, 1H) 3.23 (d, *J*=9.2 Hz, 1H) 3.40 (d, *J*=9.2 Hz, 1H) 3.46 (d, *J*=14.3 Hz, 1H) 3.71 (d, *J*=14.3 Hz, 1H) 7.08 - 7 19 (m, 3H) 7.33 - 7.40 (m, 1H) 8.60 (s, 1H); 465.0 (M+1). |
| 64 | Me | 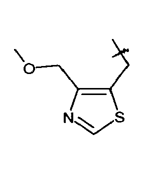 | A¹³, P1 | (5*R*,7*S*)-1-(3-fluorophenyl)-8-{[4-(methoxymethyl)-1,3-thiazol-5-yl]methyl}-3,7-dimethyl-2-thia-1,3,8-triazaspiro[4.5]deca ne 2,2-dioxide | 455.0 (M+1) |
| 65 | Me | 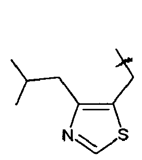 | A¹³; P1 | (5R,7S)-1-(3-fluorophenyl)-8-[(4-isobutyl-1,3-thiazol-5-yl)methyl]-3,7-dimethyl-2-thia-1,3,8-triazaspiro[4.5]deca ne 2,2-dioxide | 0.83 (d, *J*=6.6 Hz, 6H) 1.03 (d, J=6 6 Hz, 3H) 1.61 (dd, *J*=13.6, 5.4 Hz, 1H) 1.87 -1.94 (m, 2H) 1.98 - 2.05 (m, 2H) 2.29 -2.37 (m, 1H) 2.47 (d, *J*=7.2 Hz, 2H) 2.51 - 2.59 (m, 1H) 2.80 (s, 3H) 2.81 - 2.87 (m, 1H) 3.24 (d, J=9.2 Hz, 1H) 3 44 (d, *J*=30.1 Hz, 1H) 3.43 (d, *J*=6.3 Hz, 1H) 3.70 (d, J=14.5 Hz, 1H) 7.08 - 7.20 (m, 3H) 7.32 - 7.42 (m, 1H) 8.57 (s, 1H); 467.1 (M+1). |
| 66 | Me | 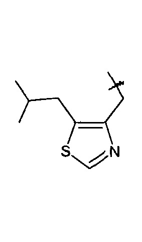 | A¹⁵, P1 | (5R,7S)-1-(3-fluorophenyl)-8-[(5-isobutyl-1,3-thiazol-4-yl)methyl]-3,7-dimethyl-2-thia-1,3,8-triazaspiro[4.5]deca ne 2,2-dioxide | 0.83 (dd, *J*=6.5, 4.0 Hz, 6H) 1.07 (d, *J*=6.8 Hz, 3H) 1.62 (dd, *J*=13 1, 5.7 Hz, 1H) 1.72 - 1.81 (m, 1H) 1.83 -1.98 (m, 2H) 2.06 (dd, *J*=13.5, 4.7 Hz, 1 H) 2.25 - 2 33 (m, 1 H) 2.47 - 2.53 (m, 1H) 2.56 (d, *J*=7.2 Hz, 2H) 2 79 (s, 3H) 2.80 - 2.85 (m, 1 H) 3.22 (d, J=9.2 Hz, 1H) 3.40 (d, *J*=9.4 Hz, 1H) 3.45 (d, *J*=13.3 Hz, 1H) 3.66 (d, *J*=13.3 Hz, 1H) 7.06 - 7.11 (m, 2H) 7.12 - 7.17 (m, 1H) 7.28 - 7.37 (m, 1 H) 8.51 (s, 1 H); 467.1 (M+1). |
| 67 | Me | 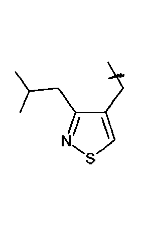 | A¹⁶, P1 | (5R,7S)-1-(3-fluorophenyl)-8-[(3-isobutylisothiazol-4-yl)methyl]-3,7-dimethyl-2-thia-1,3,8-triazaspiro[4.5]deca ne 2,2-dioxide | 0.80 (dd, *J*=6 6, 2 7 Hz, 6H) 1.03 (d, *J*=6 6 Hz, 3H) 1.63 (dd, *J*=13.7, 4.9 Hz, 1H) 1.89 (d, *J*=4.9 Hz, 2H) 1.95 - 2.07 (m, 2H) 2.29 (dt, *J*=12.6, 4.83 Hz, 1H) 2.48 - 2.50 (m, 1H) 2.53 (d, *J*=7.2 Hz, 2H) 2.80 (s, 3H) 2.81 - 2.86 (m, 1H) 3.24 (d, *J*=9 2 Hz, 1H) 3.29 (d, *J*=13.9 Hz, 1H) 3.43 (d, *J*=9.2 Hz, 1H) 3.55 (d, *J*=13.9 Hz, 1H) 7.12 (dd, *J*=9.4, 1.4 Hz, 1H) 7.14 - 7.20 (m, 2H) 7 34 - 7.42 (m, 1H) 8.22 (s, 1H); 467.1 (M+1). |
| 68 | Me | 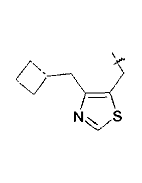 | A¹³; P1 | (5R,7S)-8-{[4-(cyclobutylmethyl)-1,3-thiazol-5-yl]methyl}-1-(3-fluorophenyl)-3,7-dimethyl-2-thia-1,3,8-triazaspiro[4.5]deca ne 2,2-dioxide | 1.04 (d, *J*=6.8 Hz, 3H) 1.54 - 1.67 (m, 4H) 1.72 - 1.80 (m, 2H) 1.88 - 1.96 (m, 3H) 1.99 - 2.06 (m, 1H) 2.28 - 2.37 (m, 1H) 2.50 - 2.65 (m, 2H) 2.65 - 2.71 (m, 2H) 2.80 (s, 3H) 2.81 - 2.89 (m, 1H) 3.24 (d, *J*=9.4 Hz, 1H) 3.37 - 3.54 (m, 2H) 3.72 (d, *J*=14.5 Hz, 1H) 7.08 - 7.20 (m, 3H) 7.33 - 7.41 (m, 1H) 8.56 (s, 1H). |
| 69 | Me | 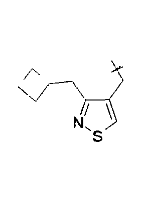 | A¹⁶, P1 | (5*R*,7*S*)-8-{[3-(cyclobutylmethyl)is othiazol-4-yl]methyl}-1-(3-fluorophenyl)-3,7-dimethyl-2-thia-1,3,8-triazaspiro[4.5]deca ne 2,2-dioxide | 1.03 (d, *J*=6 8 Hz, 3H) 1.54 - 1.67 (m, 3H) 1.68 - 1.78 (m, 2H) 1.86 - 2.03 (m, 5 H) 2.31 (td, *J*=12 5, 5.0, 4.8 Hz, 1H) 2.45 - 2.57 (m, 1H) 2 61 - 2.70 (m, 1H) 2.71 -2.76 (m, 2H) 2.80 (s, 3H) 2.83 (d, *J*=6.3 Hz, 1H) 3.25 (d, *J*=9.2 Hz, 1H) 3.29 (d, *J*=13.9 Hz, 1H) 3 44 (d, *J*=9.2 Hz, 1H) 3.54 (d, *J*=13.9 Hz, 1H) 7.09 - 7.15 (m, 2H) 7.18 (d, *J*=8.0 Hz, 1H) 7.33 - 7.42 (m, 1H) 8.20 (s, 1 H). |
| 70 | Me | 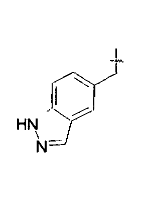 | A; P1 | (5R,7S)-1-(3-fluorophenyl)-8-(1H-indazol-5-ylmethyl)-3,7-dimethyl-2-thia-1,3,8-triazaspiro[4.5]deca ne 2,2-dioxide | 1.07 (d, *J*=6.6 Hz, 3H), 1.64 (dd, *J*=4.9. 12.7 Hz, 2H), 1.88-1.95 (m, 2H), 2.27-2.34 (m, 1H), 250-2.58 (m, 1H), 2.74-2.78 (m, 1H), 2.79 (s, 3H), 3.24 (d, *J*=9.2 Hz, 1H), 3.41 (d, *J*=9.2 Hz, 1H), 3.69 (d, *J*=13.7 Hz, 1H), 5.08-5.12 (m, 1H), 7.05-7.12 (m, 2H), 7.15 (d, *J*=8.0 Hz, 2H), 7.25-7.39 (m, 4H), 7.50 (br s, 1H); 33.51, 34.55, 60.67, 109.56, 128.44; 444.2 (M+1). |
| 71 | Me | 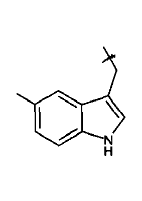 | A; P1 | (5R,7S)-1-(3-fluorophenyl)-3,7-dimethyl-8-[(5-methyl-1H-indol-3-yl)methyl]-2-thia-1,3,8-triazaspiro[4.5]deca ne 2,2-dioxide | 1.09 (d, *J*=6.6 Hz, 3H), 1.64 (dd, *J*=13.7, 6.4 Hz, 1H), 1.87-1.92 (m, 1H), 1.95 (br s, 1H), 2.08 (dd, *J*=4.5, 13.7 Hz, 1H), 2.13-2.21 (m, 1H), 2.30-2.35 (m, 1H), 2.36 (s, 3H), 2.56-2.64 (m, 1H), 2.78 (s, 3H), 3.16-3 27 (m, 1H), 3.38 (d, *J*=9.4 Hz, 1H), 3.56 (d, *J*=13.7 Hz, 1 H), 3.80 (d, *J*=13.7 Hz, 1H), 6.93 (d, *J*=2.2 Hz, 1H), 6.97 (d, *J*=8.2 Hz, 1H), 6.99-7.17 (m, 3H), 7.18-7.26 (m, 3H), 7.27 (br s, 1H); 33.51, 34.55, 41.27, 42.57, 44 38, 44.64, 47.22, 48.77, 50.84, 51.10, 60 93, 61.97, 63.00, 110.85. 111 11, 116.54, 116.80, 119.13, 120.42, 120.68, 123.79, 128.96, 129.22, 129.99, 130.25, 130.51, 134.65; 457.3 (M+1). |
| 72 | Me | 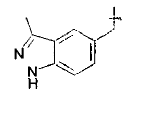 | A¹⁷, P1 | (5R,7S)-1-(3-fluorophenyl)-3,7-dimethyl-8-[(3-methyl-1H-indazol-5-yl)methyl]-2-thia-1,3,8-triazaspiro[4.5]deca ne 2,2-dioxide | 1.08 (d, *J*=6.6 Hz, 3H), 1.65 (dd, *J*=6.8, 13.5 Hz, 2H), 1 88-1.97 (m, 2H), 2.05 (dd, *J*=4.9, 13 6Hz, 1H), 2.28-2.35 (m, 1H), 2.54 (s, 3H), 2.54-2.59 (m, 1 H), 2.74-2.79 (m, 1H), 2.81 (s, 3H), 3.25 (d, *J*=9.4 Hz, 1H), 3 4-3 48 (m, 2H), 7.06-7.18 (m, 3H), 7.27-7 43 (m, 4H); 15.65, 33.50, 51.35, 128 45; 458 4 (M+1). |
| 73 | Me | 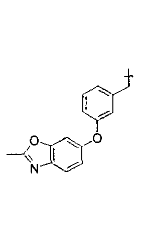 | A¹⁸; P1 | (5*R*,7*S*)-1-(3-fluorophenyl)-3,7-dimethyl-8-{3-[(2-methyl-1,3-benzoxazol-6-yl)oxy]benzyl}-2-thia-1,3,8-triazaspiro[4.5]deca ne 2,2-dioxide | 1.045 (d, *J*=6.6 Hz, 3H), 1.65 (dd, *J*=5.1, 12.9 Hz, 1H), 1 92-1 97 (m, 2H), 2.02-2.07 (m, 1H), 2.31-2.37 (m, 1H), 2.52-2.59 (m, 1H), 2.64 (s, 3H), 2.79-2.83 (m, 1H), 2.84 (s, 3H), 3 27 (d, *J*=9.0 Hz, 1H), 3 48 (d, *J*=7 0 Hz, 2H), 6.86 (dd, *J*=7.8, 2.2 Hz, 1H), 6.91-6.93 (m, 1H), 6.96-6.97 (m, 1H), 6.99 (dd, *J*=8.5, 2.2 Hz, 2H), 7.095 (d, *J*=2.2 Hz, 1H), 7.12-7.21 (m, 3H), 7.23 (t, *J*=7.8 Hz, 1H), 7.37-7.43 (q, 1H), 7.58 (d, *J* = 8.8 Hz, 1H); 551.6 (M+1). |
| 74 | Me | 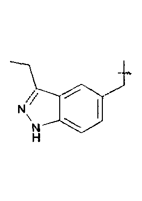 | A¹⁹; P1 | (5R,7S)-8-[(3-ethyl-1H-indazol-5-yl)methyl]-1-(3-fluorophenyl)-3,7-dimethyl-2-thia-1,3,8-triazaspiro[4.5]deca ne 2,2-dioxide | 1.11 (d, *J*=6.6 Hz, 3H), 1.27 (t, *J*=7.1 Hz, 3H), 1.64-1.71 (m, 1H), 2.06 (s, 3H), 2.32-2 37 (m, 1H), 2 55-2.62 (m, 1 H), 2.79-2.82 (m, 1H), 2.83 (s, 3H), 3.01-3.06 (q, 2H), 3.28 (d, *J*=9 3 Hz, 1H), 3.44-3.50 (m, 2H), 3.73 (d, *J* =13 7 Hz, 1 H), 4.82 (s, 3H), 7.09-7.15 (m, 2H), 7.19 (d, *J*=8.5 Hz, 1H), 7.31-7.36 (m, 2H), 7.40-7.46 (m, 2H), 7.48 (s, 1H), 7.72 (s, 1H); 472.5 (M+1). |
| 75 | Me | 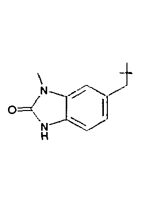 | A²⁰, P1 | 6-{[(5R,7S)-1-(3-fluorophenyl)-3,7-dimethyl-2,2-dioxido-2-thia-1,3,8-triazaspiro[4.5]dec-8-yl]methyl}-1-methyl-1,3-dihydro-2H-benzimidazol-2-one | 1.06-1.13 (br s, 3H), 1.28 (t, *J*=7.1 Hz, 1H), 1.64-1.79 (m, 1H), 1.90-2.03 (m, 1H), 2.06-2.12 (m, 1H), 2.30-2.36 (m, 1H), 2.53-2.61 (m, 1H), 2.78-2.83 (m, 1H), 2.84 (s, 3H), 3.29 (d, *J*=9.3 Hz, 1H), 3.38 (s, 3H), 3.43-3.47 (m, 2H), 3.63-3.71 (br s, 1H), 6.85 (s, 1H), 6.90 (d, *J*=8.8 Hz, 1H), 6.95 (d, *J*=8.1 Hz, 1H), 7.12-7.18 (m, 2H), 7.21 (d, *J*=7.8 Hz, 1H), 7.35-7.42 (m, 1H); 474 12 (M+1). |
| 76 | Me | 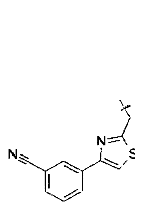 | A; P1 | 3-(2-{[(5R,7S)-1-(3-fluorophenyl)-3,7-dimethyl-2,2-dioxido-2-thia-1,3,8-triazaspiro[4.5]dec-8-yl]methyl}-1,3-thiazol-4-yl)benzonitrile | 1.11 (d, *J=*6.6 Hz, 3H), 1.66 (dd, *J*=4.9, 14.5 Hz, 1H), 1.93-2.08 (m, 2H), 2.11 (dd, *J*=4.7, 13.7 Hz, 1H), 2.47-2.55 (m, 1H), 2.7-2.77 (m, 1H), 2 82 (s, 3H), 2.84-2.9 (m, 1 H), 3.27 (d, *J*=9.4 Hz, 1H), 3.44 (d, *J*=9.2 Hz, 1H), 3.82 (d, *J*=15.4 Hz, 1H), 3.92 (d, *J*=15 4 Hz, 1H), 7.06-7.14 (m, 2H), 7.17-7.21 (m, 1H), 7.31-7.38 (m, 1H), 7.49 (t, *J*=7.6Hz, 1H), 7.56-7.60 (m, 1 H), 8.0-8.05 (m, 1 H), 8.13 (br s, 1 H); 16.42, 33.50, 34.53, 42.03, 45.66, 52.13, 55.49, 60.66, 61.44, 113.19, 115.00, 115.26, 117.07, 120.69, 120.95, 129.23, 129.74, 130.00, 130.26, 130.52, 131.30, 131.55; 512. 1 (M+1). |
| 77 | Me | 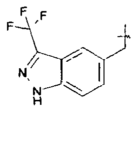 I | A²¹, P1 | (5R,7S)-1-(3-fluorophenyl)-3,7-dimethyl-8-{[3-(trifluoromethyl)-1H-indazol-5-yl]methyl}-2-thia-1,3,8-triazaspiro[4.5]deca ne 2,2-dioxide | 1.06 (d, *J*=6.6 Hz, 3H), 1.65 (dd, *J*=13.1, 4.9 Hz, 2H), 1.88-1 94 (m, 2H), 2.05 (d, *J*=4.9 Hz, 1H), 225-2.33 (m, 1H), 2.50-2.58 (m, 1H), 2.74-2.79 (m, 1H), 2.80 (s, 3H), 3.25 (d, *J*=9.4 Hz, 1H), 3.40-3.49 (m, 2H), 7.04-7.10 (m, 2H), 7 13-7.16 (m, 1H), 7.29-7.44 (m, 3H), 7.58 (br s, 1H). |
| 78 | Me | 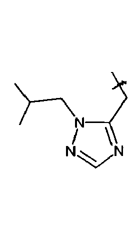 | A²², P1 | (5R,7S)-1-(3-fluorophenyl)-8-[(1-isobutyl-1H-1,2,4-triazol-5-yl)methyl]-3,7-dimethyl-2-thia-1,3,8-triazaspiro[4 5]deca ne 2,2-dioxide | 0.75 (t, *J*=6.5 Hz, 6H) 0.89 (d, *J*=6.8 Hz, 3H) 1.06 (d, *J*=6.6Hz, 2H) 162 (dd, *J*=13.8, 5.4 Hz, 1H) 188 (t, *J*=5.7 Hz, 1H) 2.00 (dd, *J*=13.7, 4.9 Hz, 1H) 2 09 -2 17 (m, 1H) 2.22 - 2.34 (m, 1H) 2.50 -2 58 (m, 1H) 2.78 (s, 3H) 3 21 (d, *J*=9.4 Hz, 1H) 3.39 (d, *J*=9.2 Hz, 1H) 3.47 (d, *J*=14.1 Hz, 1H) 3.71 (d, *J*=14.1 Hz, 1H) . 3.81 (dd, *J*=7.3, 2.4 Hz, 1H) 3.94 (d, *J*=7.4 Hz, 1 H) 7.06 - 7.18 (m, 3H) 7.31 -7.40 (m, 1H) 7.70 - 7.79 (m, 1H); 451.2 (M+1). |
| 79 | H | 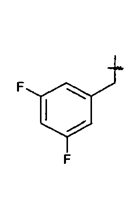 | A, P2 | (5R,7S)-8-(3,5-difluorobenzyl)-1-(3-fluorophenyl)-7-methyl-2-thia-1,3,8-triazaspiro[4 5]deca ne 2,2-dioxide | 1.04 (d, *J*=6.6Hz, 3H) 1.64 (dd, *J*=13.5, 5.6Hz, 1H) 1.81 - 1.91 (m, 1H) 1.98 (dd, *J*=8.5, 3.9 Hz, 1H) 2.01 - 2.08 (m, 1H) 2.18 - 2.31 (m, 1H) 2.47 - 2.57 (m, 1H) 2.65 - 2.76 (m, 1H) 3.28 (d, *J*=14.5 Hz, 1H) 3.44 - 3.59 (m, 2H) 3.62 (d, *J*=14.1 Hz, 1 H) 4.85 (t, *J*=7.67 Hz, 1H) 6.66 (dq, *J*=8.9, 2.50 Hz, 1H) 6.71 - 6.81 (m, 2H) 7.09 - 7.16 (m, 1H) 7.16 - 7.23 (m, 2H) 7.36 - 7.46 (m, 1H); 426.2 (M+1) 424.1 (M-1). |
| 80 | H | 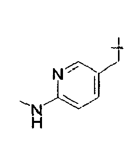 | A; P2 | 5-{[(5R,7S)-1-(3-fluorophenyl)-7-methyl-2,2-dioxido-2-thia-1,3,8-triazaspiro[4.5]dec-8-yl]methyl}-N-methylpyridin-2-amine | 1.05 (d, 3H) 1.54 - 1.66 (m, 1H) 1.69 -1.78 (m, 1H) 1.84 - 1.98 (m, 1H) 2.09 -2.20 (m, 1 H) 2.29 - 2.38 (m, 1 H) 2.79 -2.90 (m, 4H) 3.26 (d, 1H) 3.36 (d, 1H) 3.50 (d, 1H) 3.60 (d, 1H) 5.18 (s, 1H) 6.26 - 6.35 (m, 1H) 7 01 - 7.16 (m, 3H) 7.28 - 7.42 (m, 2H) 7.72 (s, 1H); 420.2 (M+1). |
| 81 | Me | 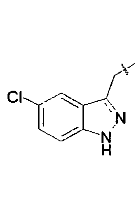 | A; P1 | (5R,7S)-8-[(5-chloro-1H-indazol-3-yl)methyl]-1-(3-fluorophenyl)-3,7-dimethyl-2-thia-1,3,8-triazaspiro[4.5]deca ne 2,2-dioxide | 1.13 (d, *J*=6.6 Hz, 3H) 1.61 - 1.68 (m, 1H) 1.84 - 1.93 (m, 1H) 1.99 (dd, *J*=8.2, 3.9 Hz, 1H) 2.09 - 2.16 (m, 1H) 2.28 -2.36 (m, 1H) 2.57 - 2.65 (m, 1H) 2.72 -2.78 (m, 1 H) 2.80 (s, 3H) 3.24 (d, *J*=9.2 Hz, 1H) 3.39 (d, *J*=9.2 Hz, 1H) 3.77 (d, *J*=14.1 Hz, 1H) 3.94 (d, *J*=14.1 Hz, 1H) 7.01 - 7.09 (m, 2H) 7.10 - 7.16 (m, 1H) 7.25 - 7.41 (m, 3H) 7 62 - 7.66 (m, 1H) 9.97 (br. s., 1H); 4784 (M+1) 4764 (M-1). |
| 82 | Me | 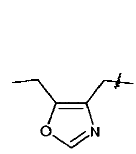 | A²; P1 | (5*R*,7*S*)-8-[(5-ethyl-1,3-oxazol-4-yl)methyl]-1-(3-fluorophenyl)-3,7-dimethyl-2-thia-1,3,8-triazaspiro[4.5]deca ne 2,2-dioxide | 1.08 (d, *J*=6.6Hz, 3H), 1.12 (t, *J*=7.6Hz, 3H), 1.63 (dd, *J*=7.0, 13.9 Hz, 1H), 1.88-1.97 (m, 1H), 2.01-2.08 (m, 1H), 2.12 (dd, *J*=4.3. 13.7 Hz, 1H), 2.27-2.35 (m, 1H), 2.50-2.57 (m, 3H), 2.68-2.77 (m, 1H), 2.79 (s, 3H), 3.21 (d, *J*=9.4 Hz, 1H), 3 35 (d, *J*=9.4 Hz, 1H), 3.42 (d, *J*=7.4 Hz, 2H), 7.04-7.16 (m, 3H), 7.28-7.34 (m, 1H), 7.64 (s, 1H); 12.80, 16.42, 18.23, 18 49, 33.50, 34.53, 42.55, 42.81, 44.88, 45 14, 47.99, 48.24, 51.09, 60.92, 61.18, 6144, 61.70, 9999, 116.81, 117.07, 120.69, 120.95, 129.23, 129 49, 130.26, 149.15, 149.41, 151.48; APCI 423.3 (M+1) |
| 83 | H | 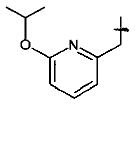 | A, Ex 1 | (5*R*,7*S*)-1-(3-fluorophenyl)-8-[(6-isopropoxypyridin-2-yl)methyl]-7-methyl-2-thia-1,3,8-tnazaspiro[4.5]deca ne 2,2-dioxide | 1.12 (d, *J*=6.64 Hz, 3 H) 1.19 (t, *J*=6.85 Hz, 1 H) 1.28 (t, *J*=5.81 Hz, 6 H) 1.60 (dd, *J*=13.50, 7.27 Hz, 1 H) 1.82 - 1.92 (m, 1 H) 2.00 - 2.11 (m, 2 H) 2.32 - 2.41 (m, 1 H) 2 57 - 2.69 (m, 2 H) 3.45 - 3.50 (m, 2 H) 3.58 (d, *J*=7.48 Hz, 2 H) 5 06 -5 17 (m, 1 H) 6.50 (d, *J*=8.31 Hz, 1 H) 6 71 (d, *J*=7.48 Hz, 1 H) 7.01 - 7.11 (m, 3 H) 7.23 - 7.31 (m, 1 H) 7.43 (dd, *J*=8.10, 7.27 Hz, 1 H), MS m/z 449.2 (M+1) |
| 84 | H | 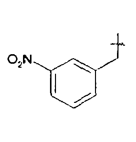 | A, Ex 1 | (5R,7S)-1-(3-fluorophenyl)-7-methyl-8-(3-nitrobenzyl)-2-thia-1.3,8-triazaspiro[4.5]deca ne 2,2-dioxide | APCI 434.9 (M+1) 434.0 (M-1) |
| 85 | H | 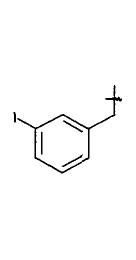 | A, P2 | (5*R*,7*S*)-1-(3-fluorophenyl)-8-(3-iodobenzyl)-7-methyl-2-thia-1,3,8-triazaspiro[4.5]deca ne 2,2-dioxide | 1.01 (d, *J*=6.7 Hz, 3H), 1.60 (ddd, *J*=13 6, 6.1, 1.0 Hz, 1 H), 181 (m, 1H), 1 92 (m, 1 H), 1.99 (dd, *J*=13 6, 4.6 Hz, 1H), 2.19 (ddd, *J*=12.6, 7.0, 3 8 Hz, 1 H), 2.46 (ddd, *J*=12.6, 8.1, 3.5 Hz, 1 H), 2.67 (m, 1H), 3.23 (d, *J*=13.8, 1H), 3.43-3.53 (m, 2H), 3 54 (d, *J*=13.7 Hz, 1H), 5.40 (br s, 1H), 6.99 (dd, *J*=8.2, 7.7 Hz, 1H), 7.11-7 19 (m, 4H), 7.37 (m, 1H), 7.53-7.56 (m, 2H).; 16 1, 30.38, 42.01, 44.2, 51.3, 53.5, 57.5, 66.2, 94.3, 117.1, 128.0, 128.5, 130.1, 136.2, 138.0, 141.7, 161.8, 164.2; 516.0 (M+1) 514.1 (M-1). |
| 86 | H | 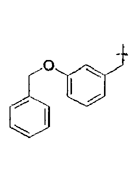 | A; P2 | (5*R*,7*S*)-8-[3-(benzyloxy)benzyl]-1-(3-fluorophenyl)-7-methyl-2-thia-1,3,8-triazaspiro[4.5]deca ne 2,2-dioxide | 1.03 (d, *J*=6.6Hz, 3H), 1.59 (dd, *J*=6.2, 13.3 Hz, 1H), 1.76-1.84 (m, 1H), 1.92-2.05 (m, 2H), 2.21-2.29 (m, 1H), 2.46-2.54 (m, 1H), 2.65-2.71 (m, 1H), 3.27 (d, *J*=13.7 Hz, 1H), 3.46-3.54 (m, 2H), 3.62 (d, *J*=13.3 Hz, 1H), 4.68-4.75 (br s, 1H), 5.02 (s, 2H), 6.78 (d, *J*=7.5 Hz, 1H), 6.81-6.86 (m, 2H), 7.08-7.20 (m, 4H), 7.29-7.43 (m, 5H): 496.6 (M+1) 494.5 (M-1). |
| 87 | H | 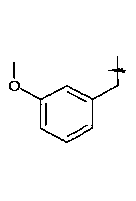 | A; P2 | (5*R*,7*S*)-1-(3-fluorophenyl)-8-(3-methoxybenzyl)-7-methyl-2-thia-1,3,8-triazaspiro[4.5]deca ne 2,2-dioxide | 1.05 (d, *J*=6.6 Hz, 3H) 1.56 - 1.65 (m, 2H) 1.77 - 1.87 (m, 1H) 1.96 (dd, *J*=8.5, 3.9 Hz, 1H) 2.01 (dd, *J*=13.9, 4.77 Hz, 1H) 2.21 - 2.31 (m, 1H) 2.47 - 2.58 (m, 1H) 2.65 - 2.76 (m, 1H) 3.28 (d, *J*=13.3 Hz, 1H) 3.44 - 3.58 (m, 2H) 3.62 (d, *J*=13.7 Hz, 1 H) 3.77 (s, 3H) 4.68 (t, *J*=7.9 Hz, 1H) 6.73 - 6.80 (m, 3H) 7.07 - 7.13 (m, 1H) 7.13 - 7.21 (m, 2H) 733 - 7.41 (m, 1 H); 420 (M+1) 418.5 (M-1). |
| 88 | H | 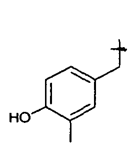 | A; P2 | 4-{[(5*R*,7*S*)-1-(3-fluorophenyl)-7-methyl-2,2-dioxido-2-thia-1,3,8-triazaspiro[4.5]dec-8-yl]methyl}-2-methylphenol | 1.14 (d, *J*=6.6Hz, 3H) 2.01 (s, 3H) 2.16 (s, 1H) 2.27 (dd, *J=*14.1, 75 Hz, 1H) 2.37 (t, *J*=6 0 Hz, 2H) 2.44 - 2.55 (m, 2H) 2.96 (dd, *J*=12.0, 5.8 Hz, 1H) 3.36 (d, *J*=13.7 Hz, 1H) 3.44 (s, 1H) 3.88 (d, *J*=13.7 Hz, 1 H) 4.43 (br. s., 1 H) 4.61 (s, 2H) 7.06 (td, *J*=7.8, 4.8 Hz, 2H) 7.19 - 7.26 (m, 1H) 7.31 (dd, *J*=7.7, 3.5 Hz, 2H) 7.58 (d, *J*=7.9 Hz, 1H) 7.72 (s. 1H); 420 (M+1) 418(M-1). |
| 89 | H | 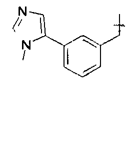 | A²³; P2 | (5*R*,7*S*)-1-(3-fluorophenyl)-7-methyl-8-[3-(1-methyl-1H-imidazol-5-yl)benzyl]-2-thia-1,3,8-triazaspiro[4.5]deca ne 2,2-dioxide | 1.07 (d, *J*=6.6Hz, 3H) 1.64 (d, *J*=6.6Hz, 1H) 1.79 - 1.90 (m, 1H) 1 96 - 2.07 (m, 1H) 2.26 - 2.37 (m, 1H) 2.54 - 2 62 (m, 1H) 2.69 - 2.78 (m, 2H) 3.36 (d, *J*=13.3 Hz, 1H) 3.45 - 3.61 (m, 2H) 3.68 (s, 3H) 3 72 (d, *J*=13.3 Hz, 1 H) 4.62 (t, *J*=8.3 Hz, 1H) 7.08 - 7 11 (m, 1H) 7.11 - 7.16 (m, 1H) 7.16 - 7.22 (m, 1H) 7.21 - 7.26 (m, 1H) 7.32 (t, *J*=9.1 Hz, 2H) 7.36 - 7.41 (m, 1H) 7.40 - 7.45 (m, 2H) 7.52 - 7.56 (m, 1H); 469.9 (M+1) 467.9 (M-1) |
| 90 | H | 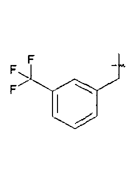 | A, P2 | (5*R*,7*S*)-1-(3-fluorophenyl)-7-methyl-8-[3-(trifluoromethyl)ben zyl]-2-thia-1,3,8-triazaspiro[4.5]deca ne 2,2-dioxide | 1.07 (d, *J*=6.6 Hz, 3H) 1.64 (dd, *J*=13.5, 6 4 Hz, 1H) 1.77 - 1.89 (m, 1H) 1.95 -2.11 (m, 2H) 2.18 - 2.29 (m, 1H) 2.46 -2.58 (m, 1 H) 2.63 - 2.75 (m, 1 H) 3.34 (d, *J*=14.1 Hz, 1H) 3.45 - 3.61 (m, 2H) 3.70 (d, *J*=13.69 Hz, 1H) 4.77 (t, *J*=7.5 Hz, 1H) 7.08 - 7.15 (m, 1H) 7.15 - 7.23 (m, 2H) 7.35 - 7.43 (m, 3H) 7.44 - 7.53 (m, 2H); 458.2 (M+1) 456.2 (M-1) |
| 91 | Me | 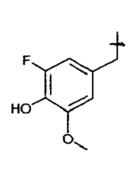 | A; P1 | 2-fluoro-4-{[(5*R*,7*S*)-1-(3-fluorophenyl)-3,7-dimethyl-2,2-dioxido-2-thia-1,3,8-triazaspiro[4.5]dec-8-yl]methyl}-6-methoxyphenol | 1.02 (d, *J*=6.8 Hz, 3H) 1.63 (ddd, *J*=13.5, 5.5, 1.1 Hz, 1H) 1.88 - 1.96 (m, 2H) 2.03 (dd, *J*=13.6, 4 8 Hz, 1H) 2.22 - 2.31 (m, 1 H) 2.45 - 2.56 (m, 1H) 2.74 (d, *J*=6.6Hz, 1 H) 2.80 (s, 3H) 3.24 (dd, *J*=11.5, 2.0 Hz, 2H) 3.37 - 3.52 (m, 2H) 3.82 (s, 3H) 5.37 (br. s., 1 H) 6.49 (s, 1H) 6.57 (dd, *J*=10.9, 1.8 Hz, 1H) 7.07 - 7.20 (m, 3H) 7.32 -7.44 (m, 1H); 468.2 (M+1) 466.1 (M-1). |
| 92 | Me | 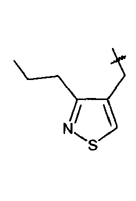 | A¹⁶; P1 | (5*R*,7*S*)-1-(3-fluorophenyl)-3,7-dimethyl-8-[(3-propylisothiazol-4-yl)methyl]-2-thia-1,3,8-triazaspiro[4.5]deca ne 2,2-dioxide | 0.82 (t, *J*=7.4 Hz, 3H) 1.03 (d, *J*=6.8 Hz, 3H) 1.58 - 1.67 (m, 3H) 1.86 - 1.91 (m, 2H) 1.99 (dd, *J*=13.7, 5.1 Hz, 1H) 2.29 (dt, *J*=12.6, 5.0 Hz, 1 H) 2.46 - 2.56 (m, 1H) 2.59 - 2.64 (m, 2H) 2.80 (s, 3H) 2.81 - 2.85 (m, 1H) 3.24 (d, *J*=9.2 Hz, 1H) 3.29 (d, *J*=13.9 Hz, 1H) 3.43 (d, *J*=9.2 Hz, 1H) 3.56 (d, *J*=13.9 Hz, 1H) 7.08 -7.20 (m, 3H) 7.32 - 7.43 (m, 1H) 8.22 (s, 1H); 453.3 (M+1). |
| 93 | Me | 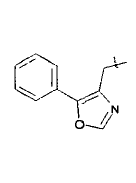 | A²⁴; P1 | (5R,7S)-1-(3-fluorophenyl)-3,7-dimethyl-8-[(5-phenyl-1,3-oxazol-4-yl)methyl]-2-thia-1,3.8-triazaspiro[4.5]deca ne 2,2-dioxide | 1.05 (d, *J*=6.6 Hz, 3H) 1.63 (dd, *J*=13.6, 5.4 Hz, 1 H) 1.88 - 1.93 (m, 2H) 2.02 (dd, *J*=13.5, 4.9 Hz, 1 H) 2.40 - 2.48 (m, 1 H) 2.55 - 2.64 (m, 1 H) 2.78 (s, 3H) 2.83 -2.89 (m, 1H) 3.22 (d, *J*=9.2 Hz, 1H) 3.40 (d, *J*=9.2 Hz, 1H) 3.51 (d, *J*=13.7 Hz, 1H) 3.72 (d, *J*=13 7 Hz, 1H) 7 05 - 7.17 (m, 3H) 7.26 - 7.39 (m, 4H) 7.60 (d, *J*=7.2 Hz, 2H) 7.78 (s, 1H): 471.0 (M+1). |
| 94 | Me | 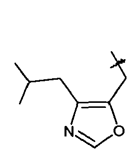 | A²⁵; P1 | (5*R*,7*S*)-1-(3-fluorophenyl)-8-[(4-isobutyl-1,3-oxazol-5-yl)methyl]-3,7-dimethyl-2-thia-1,3,8-triazaspiro[4.5]deca ne 2,2-dioxide | 0.83 (t, *J*=6.5 Hz, 6H) 1.06 (d, *J*=6.6Hz, 3H) 1.60 (dd, *J*=13.8, 7.3 Hz, 1H) 1.89 (d, *J*=6.8 Hz, 1H) 1.91 - 1.97 (m, 1 H) 2.03 -2.08 (m, 1H) 2.12 (dd, *J*=14.3, 3.3 Hz, 1H) 2.20 (d, *J*=7.4 Hz, 2H) 2.23 - 2.29 (m, 1H) 2.47 - 2.54 (m, 1H) 2.55 - 2.62 (m, 1H) 2.79 (s, 3H) 3.20 (d, *J*=9.2 Hz, 1H) 3.33 (d, *J*=9.4 Hz, 1H) 3.48 - 3.58 (m, 2H) 7.06 - 7.15 (m, 3H) 7.27 - 7.34 (m, 1H) 7.67 (s, 1H); 451.0 (M+1). |
| 95 | Me | 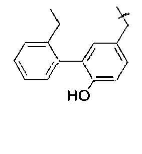 | A⁵; P1 | 2'-ethyl-5-{[(5*R*,7*S*)-1-(3-fluorophenyl)-3,7 -dimethyl-2,2-dioxido-2-thia-1,3,8-triazaspiro[4.5]dec-8-yl]methyl}biphenyl-2-ol | 0.96 - 1.02 (m, 6H) 1.60 (d, *J*=13.5 Hz, 1H) 1.89 (t, *J*=5.2 Hz, 2H) 1.99 (dd, *J*=13.6, 4.8 Hz, 1 H) 2.27 - 2.57 (m, 4H) 2.70 - 2 78 (m, 1H) 2 79 (s, 3H) 3.23 (d, *J*=9.2 Hz, 1H) 3.29 (d, *J*=14.4 Hz, 1H) 3.38 - 343 (m, 1H) 3.48 - 3.58 (m, 1H) 4.69 (br. s., 1 H) 6.85 (d, *J*=8.20 Hz, 1 H) 6.95 (d, *J*=2.2 Hz, 1H) 7.04 - 7.18 (m, 5 H) 724 - 7.28 (m, 1H) 7.31 - 7.39 (m, 3H). |
| 96 | Me | 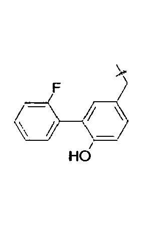 | A⁵; P1 | 2'-fluoro-5-{[(5*R*,7*S*)-1-(3-fluorophenyl)-3,7-dimethyl-2,2-dioxido-2-thia-1,3,8-tnazaspiro[4.5]dec-8-yl]methyl}biphenyl-2-ol | 1 02 (d, *J*=6.6 Hz, 3H) 1.61 (dd, *J*=13.0, 5.4 Hz, 1H) 1.84 - 1.99 (m, 2H) 1.99 -2.06 (m, 1H) 2.30 (dq, *J*=6.6, 6.2, 4.1 Hz, 1H) 2.48 - 2.57 (m, 1H) 2.71 - 2.77 (m, 1H) 2.79 (s, 3H) 3.22 (d, *J*=9.2 Hz, 1H) 3.30 (d, *J*=13 5 Hz, 1H) 3.39 (d, *J*=9.2 Hz, 1H) 3 54 (d, *J*=13.3 Hz, 1H) 5.12 (br. s., 1H) 6.85 (d, *J*=82 Hz, 1H) 7.03 (d, *J*=1.8 Hz, 1H) 7.06 - 7.13 (m, 3H) 7.13 -7.17 (m, 2H) 7 18 - 7.23 (m, 1 H) 7.28 -7.40 (m, 3H); 512.0 (M-1). |
| 97 | Me | 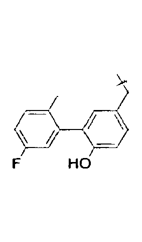 | A⁵; P1 | 5'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol | 1.01 (d, *J*=6.6 Hz, 3H) 1.61 (dd, *J*=13.6, 4.6 Hz, 1H) 1.87 - 1.95 (m, 2H) 1.97 -2.04 (m, 1 H) 2.06 (s, 3H) 2.29 (dq, *J*=6.3, 6.0, 4.3 Hz, 1 H) 2.52 (dq, *J*=8.4, 4.2, 3.9 Hz, 1H) 2.72 - 2.78 (m, 1H) 2.79 (s, 3H) 3.20 - 3.29 (m, 2H) 3.40 (d, *J*=9.4 Hz, 1H) 3.54 (d, *J*=13.3 Hz, 1H) 4.77 (br. s., 1H) 6 84 (d, *J*=8.2 Hz, 1 H) 6.90 (d, *J*=2.3 Hz, 1H) 6.99 (dt, *J*=.4, 2.8 Hz, 1H) 7.05 - 7 13 (m, 3H) 7.13 - 7.19 (m, 2H) 7.21 -7.27 (m, 1H) 7.31 - 7.40 (m, 1H); 528.0 (M+1) 526.0 (M-1). |
| 98 | Me | 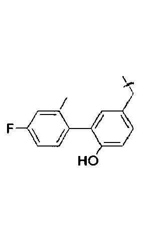 | A⁵; P1 | 4'-fluoro-5-{[(5*R*,7*S*)-1-(3-fluorophenyl)-3,7-dimethyl-2,2-dioxido-2-thia-1.3,8-triazaspiro[4.5]dec-8-yl]methyl}-2'-methylbiphenyl-2-ol | 1.01 (d, *J*=6.8 Hz, 3H) 1.61 (dd, *J*=13.4, 5.6Hz, 1H) 1.83 - 1.94 (m, 1H) 1.97 -2.04 (m, 1 H) 2.09 (s, 3H) 2.26 - 2.33 (m, 1 H) 2.52 (qd, *J*=8.4, 4.1, 3.9 Hz, 1 H) 2.75 (q, *J*=5.9 Hz, 1H) 2.79 (s, 3H) 3.21 - 3.28 (m, 3H) 340 (d, *J*=.2 Hz, 1H) 3.53 (d, *J*=13.3 Hz, 1H) 4.73 (br. s., 1H) 6.83 (d, *J*=8.2 Hz, 1H) 6.88 (d, *J*=2.2 Hz, 1H) 6.94 (dt, *J*=.3, 2.73Hz, 1H) 7.00 (dd, *J*=9.7, 2.6Hz, 1H) 7.04 - 7.18 (m, 5 H) 7.31 - 7.39 (m, 1H); 528.0 (M+1) 526.0 (M-1). |
| 99 | Me | 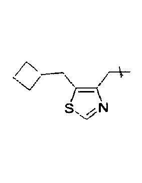 | A¹⁵; P1 | (5*R*,7*S*)-8-{[5-(cyclobutylmethyl)-1,3-thiazol-4-yl]methyl}-1-(3-fluorophenyl)-3,7-dimethyl-2-thia-1,3,8-triazaspiro[4.5]deca ne 2,2-dioxide | 1.09 (d, *J*=6.4 Hz, 3H) 1.56 - 1.68 (m, 3H) 1.71 - 1.81 (m, 3H) 1.89 - 2.01 (m, 5 H) 2.26 - 2.34 (m, 1 H) 2.35 - 2.44 (m, 1 H) 2.49 - 2.58 (m, 1H) 2.76 (d, *J*=7.4 Hz, 2H) 2.79 (s, 3H) 3.23 (d, *J*=9.2 Hz, 1H) 3.40 (d, *J*=9.2 Hz, 1H) 3.51 (d, *J*=13.66Hz, 1H) 3.67 (d, *J*=13.3 Hz, 1H) 7.04 - 7.18 (m, 3H) 7.28 - 7.36 (m, 1H) 8.49 (s, 1H); 479.3 (M+1). |
| 100 | Me | 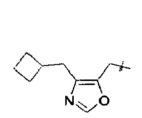 | A²⁵; P1 | (5*R*,7*S*)-8-{[4-(cyclobutylmethyl)-1,3-oxazol-5-yl]methyl}-1-(3-fluorophenyl)-3,7-dimethyl-2-thia-1,3,8-triazaspiro[4.5]decane 2,2-dioxide | 1.06 (d, *J*=6.4 Hz, 3H) 1.54 - 1.65 (m, 3H) 1.71 - 1.85 (m, 2H) 1.87 - 2.01 (m, 3H) 2.02 - 2.16 (m, 2H) 2.21 - 2.29 (m, 1H) 2.42 (d, *J*=7.6Hz, 2H) 2.46 - 2.62 (m, 3H) 2.79 (s, 3H) 3.21 (d, *J*=9.4 Hz, 1H) 3.34 (d, *J*=9.2 Hz, 1H) 3.53 (d, *J*=9.37 Hz, 2H) 7.06 - 7.15 (m, 3H) 7.27 - 7.35 (m, 1H) 7.64 (s, 1H); 463.4 (M+1). |
| 101 | Me | 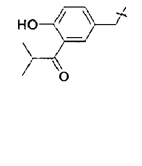 | A²⁶, P1 | 1-(5-{[(5*R*,7*S*)-1-(3-fluorophenyl)-3,7-dimethyl-2,2-dioxido-2-thia-1,3.8-triazaspiro[4.5]dec-8-yl]methyl}-2-hydroxyphenyl)-2-methylpropan-1-one | 1 03 (d, *J*=6.6 Hz, 3H) 1.17 (dd, *J*=6 8, 2.9 Hz, 6H) 1.64 (dd, *J*=13 5, 5.3 Hz, 1H) 1.88 - 1.94 (m, 2H) 1.98 - 2.04 (m, 1H) 2.29 (td, *J*=12.8, 5.1, 4.8 Hz, 1H) 2.47 -2.56 (m, 1H) 2.74 - 2 79 (m, 1H) 2.80 (s, 3H) 3.25 (d, *J*=9.2 Hz, 1H) 3 32 (d, *J*=13.5 Hz, 1H) 3.42 (d, *J*=9.2 Hz, 1H) 3.45 - 3.54 (m, 2H) 6.87 (d, *J*=8.6 Hz, 1H) 7.08 - 7 14 (m, 2H) 7.15 - 7.19 (m, 1H) 7.28 (dd, *J*=.6, 2.2 Hz, 1H) 7.32 -7 39 (m, 1H) 7.55 (d, *J*=2.0 Hz, 1H) 12.38 (s, 1H); 490.3 (M+1) 488.3 (M-1). |
| 102 | Me | 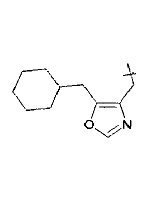 | A²: P1 | (5*R*,7*S*)-8-{[5-(cyclohexylmethyl)-1,3-oxazol-4-yl]methyl}-1-(3-fluorophenyl)-3,7-dimethyl-2-thia-1,3,8-triazaspiro[4.5]deca ne 2,2-dioxide | 0.78 - 0.87 (m, 2H) 0.89 - 0.98 (m, 1H) 1.06 (d, *J*=6.6Hz, 3H) 1.09 - 1.22 (m, 3H) 1.51 - 1.56 (m, 3H) 1.58 - 1.70 (m, 4H) 1.87 - 1.97 (m, 2H) 2.06 (dd, *J*=13.6, 4.6Hz, 1H) 2.28 - 2.35 (m, 1H) 2.37 (d, *J*=6.8 Hz, 2H) 2.47 - 2.56 (m, 1H) 2.74 -2.82 (m, 1H) 2.79 (s, 3H) 3.21 (d, *J*=9.18 Hz, 1H) 3.30 - 3.46 (m, 3H) 7.05 - 7.16 (m, 3H) 7.28 - 7.36 (m, 1H) 7.63 (s, 1H), 491.3 (M+1) |
| 103 | Me | 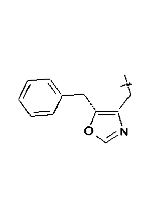 | A²⁷; P1 | (5*R*,7*S*)-8-[(5-benzyl-1,3-oxazol-4-yl)methyl]-1-(3-fluorophenyl)-3,7-dimethyl-2-thia-1,3,8-triazaspiro[4.5]deca ne 2,2-dioxide | 1.04 (d, *J*=6 6 Hz, 3H) 1.58 (ddd, *J*=13 6, 6.0, 1.2 Hz, 1H) 1 81 - 1.90 (m, 1H) 1 90 - 1.99 (m, 1H) 2.04 (dd, *J*=13.7, 4.7 Hz, 1H) 2.26 - 2.37 (m, 1H) 2.49 (td, *J*=12.4, 8.4, 3.8 Hz, 1H) 2.75 (dd, *J*=11.5, 6.3 Hz, 1H) 2.78 (s, 3H) 3.20 (d, *J*=9.2 Hz, 1H) 3.42 (d, *J*=65.0 Hz, 1H) 3.41 (d, *J*=43 Hz, 1H) 3.37 (d, *J*=3.3 Hz, 1H) 3.89 (s, 2H) 7.04 - 7.16 (m, 3H) 7.17 - 7.23 (m, 5 H) 7.25 - 7.36 (m, 1H) 7.65 (s, 1H); 485 3 (M+1). |
| 104 | Me | 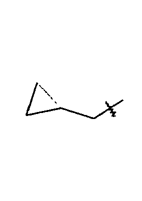 | A; P1 | (5*R*,7*S*)-8-(cyclopropylmethyl) -1-(3-fluorophenyl)-3,7-dimethyl-2-thia-1,3.8-triazaspiro[4.5]deca ne 2,2-dioxide | -0.05 - 0.01 (m, 2H) 0.37 - 0.43 (m, 2H) 0.59 - 0.67 (m, 1H) 0.94 (d, *J*=6.6 Hz, 3H) 1.61 (dd, *J*=13.7, 6.1 Hz, 1H) 1.90 -1.99 (m, 1H) 2.01 - 2.10 (m, 2H) 2.22 (dd, *J*=54.0, 6.5Hz, 1H) 2.22 (dd, *J*=27.2, 6.5Hz, 1H) 2.50 - 2.65 (m, 2H) 2.75 -2.83 (m, 1H) 280 (s, 3H) 3.22 (d, *J*=9.2 Hz, 1H) 3.38 (d, *J*=9.2 Hz, 1H) 7.08 -7.17 (m, 2H) 7.19 - 7.23 (m, 1H) 7.32 -7.40 (m, 1H); 368.1 (M+1) 366.4 (M-1). |
| 105 | Me | 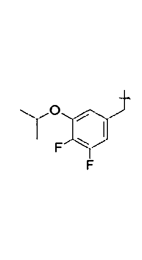 | A²⁸; P1 | (5*R*,7*S*)-8-(3,4-difluoro-5-isopropoxybenzyl)-1-(3-fluorophenyl)-3,7-dimethyl-2-thia-1,3,8-triazaspiro[4.5]deca ne 2,2-dioxide | 1.00 (d, *J*=6.6 Hz, 3H), 1.29 (d, *J*=6.0 Hz, 6H), 1.63 (dd, *J*=13.7, 5.5 Hz, 1H), 1.88-1.94 (m, 2H), 1.98-2.04 (m, 1H), 2.22-2.29 (m, 1H), 2.46-2.54 (m, 1H), 2.69-2.78 (m, 1H), 2.80 (s, 3H), 3.20-3.26 (m, 1H), 341 (d, *J*=9.2 Hz, 1H), 3.46 (d, J=13.9 Hz, 1H), 4.4-4.49 (m, 1H), 6.56-6.66 (m, 2H), 7.08-7.20 (m, 3H), 7.30-7.41 (m, 1H); 15.15, 22.13, 33.25, 33.51, 34.29, 34.55, 42.31, 44.38, 51.62, 57.57, 60.41, 60.67, 61.71, 72.83, 109.04, 112.41, 117.06, 120.68, 120.94, 129.48, 130.25, 130.51; 498.3 (M+1). |
| 106 | Me | 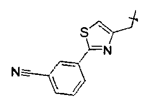 | A; P1 | 3-(4-{[(5*R*,7*S*)-1-(3-fluorophenyl)-3,7-dimethyl-2,2-dioxido-2-thia-1,3,8-triazaspiro[4.5]dec-8-yl]methyl}-1,3-thiazol-2-yl)benzonitrile | 1.12 (d, *J*=6.6 Hz, 3H), 1.63 (dd, *J*=6,2, 13.1 Hz, 1H), 1.89-1.97 (m, 1H), 2.05-2.14 (m, 2H), 2.39-2.47 (m, 1H), 2.61-2.74 (m, 1H), 2.80 (s, 3H), 3.23 (d, *J*=9.4 Hz, 1H), 3.38 (d, *J*=9.2 Hz. 1H). 3.75 (dd, *J*=14.8, 24.2 Hz, 2H), 6.91-6 98 (m, 1H), 7.02-7.07 (m, 2H), 7.10-7.14 (m, 1H), 7.20-7.26 (m, 1H), 7.51-756 (m, 1H), 7.65-7.69 (m, 1H), 8.05-809 (m, 1H), 8.15-8.18 (m, 1H); 512.1 (M+1). |
| 107 | Me | 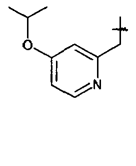 | A²⁹; P1 | (5*R*,7*S*)-1-(3-fluorophenyl)-8-[(4-isopropoxypyridin-2-yl)methyl]-3,7-dimethyl-2-thia-1,3,8-triazaspiro[4.5]deca ne 2,2-dioxide | 1.08 (d, *J*=6.8 Hz, 3H), 1.33 (d, *J*=6.1 Hz, 6H), 1.67 (dd, *J*=13.4, 6.1 Hz, 2H), 1.91-2.02 (m, 2H), 2.09 (dd, *J*=13.4, 4.9 Hz, 1H), 2.37-2.43 (m, 1H), 2.59-2.65 (m, 1H), 2.83 (s, 3H), 2.84-2.91 (m, 1H), 3.27 (d, *J*=9.3 Hz, 1 H), 3.48 (d, *J*=4.6 Hz, 1 H), 3.66 (d, *J*=14.2Hz, 1H), 4.54-4.61 (m, 1 H), 6.62 (dd, *J*=5.6, 2.4 Hz, 1H), 6.8 (d, *J*=2.4Hz, 1H), 7.12-7.18 (m, 2H), 7.21 (d, *J*=7.1 Hz, 1H), 7.36-7.42 (m, 1H), 8.28 (d, J=5.9 Hz, 1 H). |
| 108 | Me | 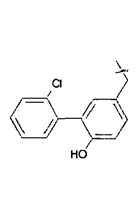 | A⁵; P1 | 2'-chloro-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol | 1.02 (d, *J*=6.6 Hz, 3H) 1.61 (dd, *J*=13.4, 5.6 Hz, 1 H) 1.87 - 1.95 (m, 2H) 2.01 (dd, *J*=13.4, 4.8 Hz, 1H) 2.26 - 2.35 (m, 1H) 2.49 - 2.58 (m, 1H) 2.76 (d, *J*=6.1 Hz, 1H) 2.79 (s, 3H) 3.23 (d, J=9.2 Hz, 1H) 3.28 (d, *J*=13.5 Hz, 1H) 3 40 (d, *J*=9.2 Hz, 1 H) 3.55 (d, *J*=13.3 Hz, 1 H) 4.85 (br. s., 1H) 6.85 (d, *J*=8.4 Hz, 1H) 6.98 (d, *J*=1.6Hz, 1H) 7.07 - 7.16 (m, 4H) 7.27 -7.39 (m, 4H) 7.47 - 7.52 (m, 1H); 529.9 (M+1) 527.9 (M-1). |
| 109 | Me | 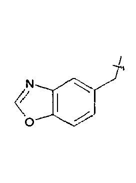 | A³⁰; P1 | (5*R*,7*S*)-8-(1,3-benzoxazol-5-ylmethyl)-1-(3-fluorophenyl)-3,7-dimethyl-2-thia-1,3,8-triazaspiro[4.5]deca ne 2,2-dioxide | 1.24 (d, *J*=6.1 Hz, 3H), 1.71-1.81 (m, 1H), 1.92-2.02 (m, 2H), 2.07-2.22 (m, 1H), 2.36-2.48 (br s, 1H), 2 74 (s, 3H), 2.76-2.92 (m, 1 H), 3.51 (d, *J*=9.4 Hz, 1H), 3.77-3.98 (m, 2H), 5.45 (s, 2H), 7.03-7.11 (m, 2H), 7.11-7.15 (m, 1 H), 7.25-7.29 (m, 1H), 7.32 (d, *J*=8.4 Hz, 1H), 7.56-7.63 (m, 1H), 8.47 (s, 1 H); 445 0 (M+1). |
| 110 | e | 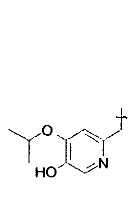 | C³¹; P1 | 6-{[(5*R*,7*S*)-1-(3-fluorophenyl)-3,7-dimethyl-2,2-dioxido-2-thia-1,3,8-triazaspiro[4.5]dec-8-yl]methyl}-4-isopropoxypyridin-3-ol | 1.08 (d, *J*=6.8 Hz, 3H), 1.35 (d, *J*=6.1 Hz, 6H), 1.67 (dd, *J*=4.9, 13.7 Hz, 1H), 1.95 (t, *J*=5.4 Hz, 2H), 2.05 (d, *J*=5.1 Hz, 1H), 2.08 (d, *J*=4.9 Hz, 1 H), 2.36-2.42 (m, 1H), 2.58-2.64 (m, 1H), 2.83 (s, 3H), 2.84-2.89 (m, 1H), 3.27 (d, *J*=9.0 Hz, 1H), 3.44-3.50 (m, 2H), 3.61 (d, *J*=13.7 Hz, 1 H), 4.56-4.64 (m, 1 H), 6.78 (s, 1 H), 7.11-7.18 (m, 2H), 7.19-7.22 (m, 1H), 7.35-7.41 (m, 1H), 8.07 (s, 1H); 21.86, 22.11, 33.50, 34.27, 42.04, 44.36, 51.87, 59.89, 60.41, 71.53, 106.46, 116.81, 117.07, 129.48, 130.26, 135.95, 142.16; 479.1 (M+1) 477.2 (M-1). |
| 111 | Me | 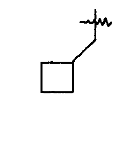 | C; P1 | (5*R*,7*S*)-8-(cyclobutylmethyl)-1-(3-fluorophenyl)-3,7-dimethyl-2-thia-1,3,8-triazaspiro[4.5]deca ne 2,2-dioxide | 0.97 (d, *J*=6.7 Hz, 3H), 1.53-1.65 (m, 3H), 1.75 (m, 1H), 1.83 (m, 1H), 1.89-2.06 (m, 5H), 2.28-2.37 (m, 3H), 2.46 (m, 1H), 2.55 (m, 1H), 2.70 (m, 1H), 2.83 (s, 3H), 3.24 (d, half of AB quartet, *J*=9.3 Hz, 1 H), 3.40 (d, half of AB quartet, *J*=9.3 Hz, 1 H), 7.15-7.19 (m, 2H), 7.23 (ddd, *J*=8.0, 1.6, 1.3 Hz, 1H), 7.41 (m,1H); 382.0 (M+1). |
| 112 | Me | 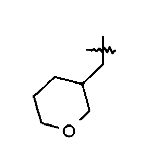 | A; P1 | (5*R*,7*S*)-1-(3-fluorophenyl)-3,7-dimethyl-8-(tetrahydro-2H-pyran-3-ylmethyl)-2-thia-1,3,8-triazaspiro[4.5]deca ne 2,2-dioxide | 412.4 (M+1) |
| 113 | Me | 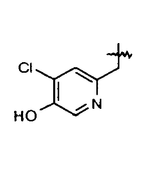 | C³²; P1 | 4-chloro-6-{[(5*R*,7*S*)-1-(3-fluorophenyl)-3,7-dimethyl-2,2-dioxido-2-thia-1.3.8-triazaspiro[4.5]dec-8-yl]methyl}pyridin-3-ol | ¹H NMR (500 MHz, CDCl₃) δ ppm 1.10 (d, *J*=6.6 Hz, 3H), 1.66-1.73 (m, 1H), 1.95-2.04 (m, 2H), 2.12 (dd, *J*=13 1, 4.0 Hz, 1 H), 2.34-2.41 (m, 1 H), 2.59-2.66 (m, 2H), 2 81-2 82 (m, 1 H), 2 83 (s, 3H), 3.27 (d, *J*=9.3 Hz, 1 H), 3.44 (d, *J*=9.3 Hz, 1 H), 3.50 (d, *J*=14.2 Hz, 1H), 3.64-3.70 (m, 1H), 7.11-7.22 (m, 3H), 726 (s, 1H), 7 34-7.43 (m, 1 H), 8.23 (s, 1H). |
| 114 | Me | 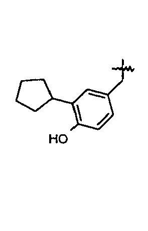 | A³³; P1 | 2-cyclopentyl-4-{[(5*R*,7*S*)-1-(3-fluorophenyl)-3,7-dimethyl-2,2-dioxido-2-thia-1,3,8-triazaspiro[4.5]dec-8-yl]methyl}phenol | 1.05 (d, *J*=6.7 Hz, 3H), 1.50-1.81 (m, 7H), 1.88-2.07 (m, 5H), 2.31 (ddd, *J*=12.4, 6.1, 4.3 Hz, 1H), 2.53 (ddd, *J*=12.5, 8.4, 3.8 Hz, 1H), 2.76 (m, 1H), 2.83 (s, 3H), 3.17 (m, 1H), 3.26 (d, half of AB quartet, *J*=9.3 Hz, 1H), 3.31 (d, half of AB quartet, *J*=13.3 Hz, 1 H), 3.44 (d, half of AB quartet, *J*=9.2 Hz, 1H), 3.52 (d, half of AB quartet, *J*=13.3 Hz, 1H), 6 63 (d, *J*=8.1 Hz, 1 H), 6.87 (dd, *J*=8.1, 2.2 Hz, 1H), 6.98 (d, *J*=2.1 Hz, 1H), 7.11-7.19 (m, 3H), 7.37 (ddd, *J*=8, 8, 6 4 Hz, 1H); APCI 488.1 (M+1). |
| 115 | Me | 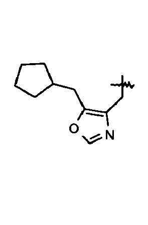 | A², P1 | (5*R*,7*S*)-8-{[5-(cyclopentylmethyl) -1,3-oxazol-4-yl]methyl}-1-(3-fluorophenyl)-3,7-dimethyl-2-thia-1,3,8-triazaspiro[4.5]deca ne 2,2-dioxide | 1.09 (d, *J*=6.6 Hz, 3H), 1.07-1.16 (m, 1H), 1.47-1.70 (m, 8H), 1.93 (m, 1H), 1.99-2.14 (m, 3H), 2.36 (ddd, *J*=12.5, 6.9, 4.0 Hz, 1H), 2.52 (d, *J*=7.4 Hz, 2H), 2.55 (m, 1H), 2.8 (m, 1H), 2.82 (s, 3H), 3.25 (d, half of AB quartet, *J*=9.3 Hz, 1H), 3.36 (d, half of AB quartet, *J*=13.9 Hz, 1 H), 3.41 (d, half of AB quartet, *J*=9.2 Hz, 1 H), 3.46 (d, half of AB quartet, *J*=13.9 Hz, 1H), 7.09-7.19 (m, 3H), 7.35 (ddd, J=8.0, 8.0, 6.5 Hz, 1 H), 7.66 (s, 1H); APCI 477.2 (M+1). |
| 116 | Me | 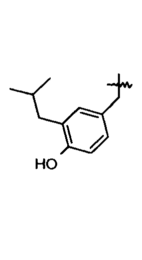 | A³³; P1 | 4-{[(5*R*,7*S*)-1-(3-fluorophenyl)-3,7-dimethyl-2,2-dioxido-2-thia-1,3.8-triazaspiro[4.5]dec-8-yl]methyl}-2-isobutylphenol | 0.89-0.91 (m, 6H), 1.04 (d, *J*=6.7 Hz, 3H), 1.64 (br dd, *J*=13.8, 5.7 Hz, 1H), 1.83-1.99 (m, 3H), 2.03 (dd, *J*=13.5, 4.8 Hz, 1H), 2.31 (ddd, *J*=12.6, 6.1, 4.4 Hz, 1H), 2.42 (br d, *J*=7 Hz, 2H), 2.53 (ddd, *J*=12.7, 8.3, 3.8 Hz, 1H), 2.76 (m, 1H), 2.83 (s, 3H), 3.26 (d, half of AB quartet, *J*=9.2 Hz, 1H), 3.28 (d, half of AB quartet, *J*=13.2 Hz, 1H), 3.44 (d, half of AB quartet, *J*=9.2 Hz, 1 H), 3.52 (d, half of AB quartet, *J*=13.2 Hz, 1 H), 6.64 (m, 1H), 6.86-6.89 (m, 2H), 7.10-7.20 (m, 3H), 7.37 (ddd, *J*=8.1, 8.1, 6.4 Hz, 1 H); APCI 476.1 (M+1). |
| 117 | Me | 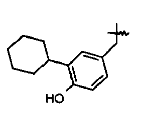 | A³³; P1 | 2-cyclohexyl-4-{[(5*R*,7*S*)-1-(3-fluorophenyl)-3,7-dimethyl-2,2-dioxido-2-thia-1,3,8-triazaspiro[4.5]dec-8-yl]methyl}phenol | 1.05 (d, *J*=6.7 Hz, 3H), 1.19-1.48 (m, 5H), 1.64 (br dd, *J*-13.6, 5.6 Hz, 1H), 1.74-2.01 (m, 7H), 2.05 (dd, *J*=13.7, 4.9 Hz, 1H), 2.30 (ddd, *J*=12.6, 6.4, 4.2 Hz, 1H), 2.53 (ddd, *J*=12.7, 8.3, 3.7 Hz, 1H), 2.72-2.80 (m, 2H), 2.83 (s, 3H), 3.26 (d,half of AB quartet, *J*=9.3 Hz, 1H), 3 30 (d, half of AB quartet, *J*=13.3 Hz, 1 H), 3.44 (d, half of AB quartet, *J*=9.3 Hz, 1 H), 3.53 (d, half of AB quartet, *J*=13.3 Hz, 1H), 662 (d, *J*=8.1 Hz, 1H), 6.86 (dd, *J*=8.1, 2.0 Hz, 1H), 6.96 (d, *J*=2.0 Hz, 1 H), 7.11-7.19 (m, 3H), 7.36 (ddd, *J*=8.0, 8.0, 6.5 Hz, 1H); APCI 502.2 (M+1). |

1: Alkylation of 1*H*-pyrazole via the method of A.V. lvachtchenko et al., J. Heterocyclic Chemistry 2004, 41, 931-939, was followed by introduction of the aldehyde group according to T Schläger et al., Bioorganic and Medicinal Chemistry 2008, 16, 2992-3001
2 The corresponding ester was prepared according to the procedure reported by W.L.F. Armarego et al., Eur. J. Med. Chem. 1987, 22, 283-291.
3: The aldyhyde was prepared via a Suzuki coupling between commercially available compounds.
4: The commercially available N-BOC denvative of the aldehyde was employed.
5: The methoxy intermediate was prepared according to the method of C. Garino et al., Bioorganic and Medicinal Chemistry Letters 2005, 15, 135-138. The methyl was removed with a reagent such as BBr₃ to form the desired phenol.
6: The aldehyde was prepared by treatment of the commercially available 3-hydroxybenzaldehyde with acrylonitrile in the presence of benzyltnmethylammonium hydroxide.
7: The aldehyde was prepared according to the method of N. Wang et al., Journal of Combinatorial Chemistry 2008, 10, 825-834.
8: The aldehyde was prepared according to the method of J. Letrouneau, PCT Int. Appl. 2006, WO 2006095014.
9: The aldehyde was prepared according to the method of M. Sriram et al., Bioorganic & Medicinal Chemistry2008, 16, 8161-8171.
10: The aldehyde was prepared according to the method of K. Dabak, Turkish Journal of Chemistry 2002, 26, 955.
11: The aldehyde was prepared according to the method of A. Binggeli et al., U.S. Pat. Appl. Publ., US 2007-724688.
12: The aldehyde was prepared according t the method outlined in *PCT Int. Appl.* **2007,** WO 2007030559
13: The corresponding ester was prepared from the appropriate β-keto ester via chlorina6on (see M G. Perrone et al., European Journal of Medicinal Chemistry 2005, 40, 143-154) followed by reaction with thioformamide according to the work of N. Hagirtoya et al., Bioorganic and Medicinal Chemistry Letters 2004, 14, 2935-2939
14: Commercially available 3-fluoro-4-hydroxy-5-methoxybenzaldehyde was demethylated with a reagent such as BBr₃. The dihydroxy compound was then selectively alkylated with 2-iodopropane by using a base such as sodium hydride;
15 The corresponding ester was prepared according to Y-M. Cui et al., Bioorganic and Medicinal Chemistry Letters 2005, 15, 3732-3736.
16 The corresponding thiazole ester was prepared according to the method of M. Muraoka et al., Journal of the Chemical Society, Perkin Transactions 11989, 7, 1241-52.
17 The corresponding ester was prepared according to the method of W. Xie et al., PCT Int. Appl. 2005, 2005092890.
18 The aldehyde was prepared according to the method of D. Dhanak and S.D. Knight, PCT Int. Appl. 2007, WO2007103755.
19 The corresponding acid was prepared according to the method of Q. Zang et al., PCT Int. Appl. 2006, WO2006044860.
20: The corresponding ester was prepared according to B. Zou et a/., Organic Letters 2007, 9, 4291-4294.
21. The bromoheterocycle was prepared according to P. Guzzo et al., PCT Int. Appl. 2008, WO 2008086404; introduction of the aldehyde moiety was carried out by the method of G. Luo et al., Journal of Organic Chemistry, 2006, 71, 5392-5395.
22: The triazole aldehyde was prepared according to the procedure outlined in U.S. Patent 4826833, 02 May 1989*.*
23: The corresponding acid was prepared accordmg to the method of S. Kumar et al., Journal of Medicinal Chemistry, 2008, 51, 4968-4977.
24: Prepared from 2-bromo-1-phenyl ethanone according to the procedure of V.J. Majo and P.T. Perumal, Journal of Organic Chemistry 1998, 63, 7136-7142.
25: The corresponding ester was prepared according to the procedure reported by A. Aguado et al., Journal of Heterocyclic Chemistry 2007, 44, 1517-1520.
26: The aldehyde was prepared according to the method of G Dona et al., European Journal of Medicinal Chemistry 1979, 14, 347-351
27: The corresponding ester was made according to the method of M. Brescia et al., Bioorganic & Medicinal Chemistry Letters 2007, 17, 1211-1215.
28: 5-Bromo-2,3-difluorophenol was reacted with 2-hydroxypropane under standard Mitsunobu condition. Introduction of the aldehyde moiety was carried out by using *n*-BuLi and DMF at low temperature.
29 The corresponding ester was prepared by the treatment of methyl 5-hydroxynicotinate with 2-iodopropane in the presence of base.
30 The aldehyde was prepared according to the method of C. Blackburn et al., PCT Int. Appl., 2003, WO2003106452.
31 2-Hydroxymethyl-5-hydroxy-gamma-pyrone was benzyl protected and then converted to 3-benzyloxy-6-hydroxymethylpyridin-4-one according to the method of T Teitei et al., Australian Journal of Chemistry 1983, 36, 2307-2316. Treatment of the pyridinone with 2-iodopropane and base formed the isopropoxypyridine. The final step was a debenzylation via hydrogenation.
32: L. Harris and T. Tsutomu, Australian Journal of Chemistry 1977, 30, 649-655.
33: The corresponding ester was prepared via redaction of the Grignard reagent derived from methyl 3-iodo-4-methoxybenzoate with the appropriate aliphatic ketone or aldehyde, according to R.S. Muthyala et al., Bioorganic & Medicinal Chemistry Letters 2003, 13, 4485-4488. Demethylation was carried out with boron tribromide.

**Table 2**

| | | | | | |
|---|---|---|---|---|---|
| 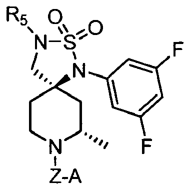 | | | | | |

| Ex# | R₅ | Z-A | Prep Metho d; Startin g Materi al | IUPAC Name | ¹H NMR (400 MHz, CDCl₃), ¹³C NMR (100 MHz, CDCl₃) (unless otherwise indicated): observed peaks, δ (ppm); Mass spectrum: LCMS, observed ion m/z (unless otherwise indicated) |
|---|---|---|---|---|---|
| 118 | H | 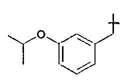 | C; P4 | (5*R*,7*S*)-1-(3,5-difluorophenyl)-8-(3-isopropoxybenzyl) -7-methyl-2-thia-1,3,8-triazaspiro[4.5]dec ane 2,2-dioxide | 1.05, (d, *J*=6.8 Hz, 3H), 1.29 (d, *J*=6.0 Hz, 6H), 1.76-1.86 (m, 1H), 1.90-2.03 (m, 2H), 2.26-2.33 (m, 1H), 2 48-2.57 (m, 1 H), 2.71-2.79 (m, 1H), 3.44-3.62 (m, 3H), 4.45-4.52 (m, 1H), 4.56-4.65 (m, 1H), 6.71-6.76 (m, 3H), 6.88-6.95 (m, 3H), 7.11-7.17 (m, 1H); APCI 465.6 (M+1) 463.6 (M-1).4.45-4.52 (m, 1H), 4.56-4.65 (m, 1H), 6.71-6.76 (m, 3H), 6.88-6.95 (m, 3H), 7.11-7.17 (m, 1H); APCI 465.6 (M+1) 463 6 (M-1) |

**Table 3**

| | | | | |
|---|---|---|---|---|
| 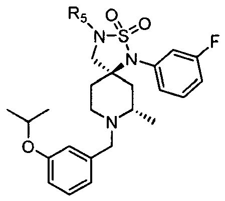 | | | | |

| Ex # | R₅ | Prep Method; Starting Material | IUPAC Name | ¹H NMR (400 MHz, CDCl₃), ¹³C NMR (100 MHz, CDCl₃) (unless otherwise indicated) observed peaks, δ (ppm); Mass spectrum: LCMS, observed ion *m*/*z* (unless otherwise ndicated) |
|---|---|---|---|---|
| 119 | 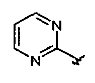 | Ex 4, C; Cpd 1 | (5*R*,7*S*)-1-(3-fluorophenyl)-8-(3-isopropoxybenzyl)-7 methyl-3-pyrimidin-2-yl-2 thia-1,3,8-triazaspiro[4.5]decane 2,2-dioxide | 1.03-1.14 (br s, 3H), 1.28 (d, *J*=6.1 Hz, 6H), 1.61-1.71 (m, 1H), 1.92-2.02 (m, 1H), 2.04-2.16 (m, 2H), 2.29-2.38 (br s, 1H), 2.53-2.63 (m, 1H), 2.73-2.83 (br s, 1H), 3.30 (d, *J*=13.3 Hz, 1H), 3.62 (d, *J*=14.3 Hz, 1H), 4.02 (d, *J*=9.6Hz, 1H), 4.27 (d, *J*=9.9 Hz, 1H), 4.42-4.53 (m, 1H), 6.69-6.78 (m, 3H), 6.96 (t, *J*=4.9 Hz, 1H), 7.10-7.19 (m, 2H), 7.20-7.28 (m, 1H, assumed, partially obscure by solvent peak), 7.33-7.42 (m, 2H), 8.57 (d, *J* = 4 9 Hz, 2H); 526.1 (M+1). |
| 120 | 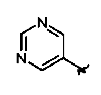 | Ex 4; Cpd 2 | (5*R*,7*S*)-1-(3-fluorophenyl)-8-(3-isopropoxybenzyl)-7-methyl-3-pyrimidin-5-yl-2-thia-1,3,8-triazaspiro[4.5]decane 2,2-dioxide | 1.09-1.17 (br s, 3H), 1.32 (d, *J*=6.1 Hz, 6H), 1.70-1.83 (br s, 1H), 2.0-2.11 (br s, 1H), 2.13-2.26 (m, 2H), 2 33-2.41 (m, 1H), 2.58-2.67 (m, 1H), 2.78-2.86 (m, 1H), 3.28-3.39 (br s, 1H), 3.62-3.72 (br s, 1H), 3.89 (d, half of AB quartet, *J*=8.7 Hz, 1H), 3.94 (d, *J*=10.3 Hz, 1H)), 4 52 (m, 1H), 6.75-6.80 (m, 3H), 7.14-7.26 (m, 4H), 7.41-7.47 (m, 1H), 8.77 (s, 2H), 9.06 (s, 1H); 526.0 (M+1). |
| 121 | 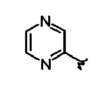 | Ex 4; Cpd 2 | *(5R,7S)-1-(3-*fluorophenyl)-8-(3-isopropoxybenzyl)-7-methyl-3-pyrazin-2-yl-2-thia-1,3,8-triazaspiro[4.5]decane 2,2-dioxide | 1.1 (d, *J*=5.9 Hz, 3H), 1.28 (d, *J*=6.1 Hz, 6H), 1.66-1.75 (m, 1H), 1.93-2.03 (m, 1H), 2.03-2.15 (m, 2H), 2.29-2.38 (m, 1H), 2.56-2.65 (m, 1H), 2.77-2.85 (m, 1 H), 3.31 (d, *J*=13.5 Hz, 1H), 3.62 (d, *J*=13.5 Hz, 1H), 4.01 (d, *J*=10.2Hz, 1H), 4.28 (d, *J*=10.2 Hz, 1H), 4.43-4.53 (m, 1H), 6.70-6.77 (m, 3H), 7.0-7.24 (m, 4H), 7.36-7.43 (m, 1H), 8.24-8.29 (m, 1H), 8.78-8.82 (s, 1H); 526.1 (M+1). |
| 122 | 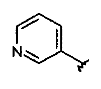 | Ex 4; Cpd 2 | (5R,7S)-1-(3-fluorophenyl)-8-(3-isopropoxybenzyl)-7-methyl-3-pyridin-3-yl-2-thia-1,3,8-tnazaspiro[4.5]decane 2,2-dioxide | 1H NMR (500 MHz, CHLOROFORM) 1.14 (br s, 3H), 1.32 (d, *J*=5.9 Hz, 3H), 1.71-1.84 (m, 1 H), 2.03-2.26 (m, 1H), 2.34-2.41 (m, 1H), 2.57-2.68 (m, 1H), 2.79-2.87 (m, 2H), 3.29-3.39 (m, 1H), 3.62-3.73 (m, 1H), 3.89 (d, half of AB quartet, *J*=8.8 Hz, 1H), 3.93 (d, half of AB quartet, *J*=8.8 Hz, 1H), 4.48-4.55 (m, 1H), 6.75-6.81 (m, 3H), 7.14-7.25 (m, 4H), 7.35 (dd, *J*=4.6Hz, 8.3Hz, 1H), 739-7.45 (m, 1H), 7.81 (d, *J*=9.5 Hz, 1H), 8.47 (br s, 1H), 8.55 (br s, 1H) |
| 123 | 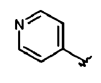 | B,C; Cpd 1 | (5*R*,7*S*)-1-(3-fluorophenyl)-8-(3-isopropoxybenzyl)-7-methyl-3-pyridin-4-yl-2-thia-1,3,8-triazaspiro[4.5]decane 2,2-dioxide | 1.1-1.19 (br s, 3H), 1.32 (d, *J*=6.1 Hz, 6H), 1.71-1.81 (m, 1H), 1.98-2.10 (m, 1H), 2.12-2-25 (m, 1H), 2.33-2.40 (m, 1H), 2.59-2.67 (m, 1 H), 2.76-2.86 (m, 1 H), 3.29-3.40 (m, 1H), 3.66-3.74 (m, 1H), 3.84 (d, *J*=8.5 Hz, 1H), 3.93 (d, *J*=8.5 Hz, 1H), 4.48-4.55 (m, 1H), 672-683 (m, 3H), 7.10-7.25 (m, 6H), 7.4-7.47 (m, 1H), 8.54 (d, *J*=5.9 Hz, 2H); 525.0 (M+1). |
| 124 | 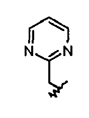 | B; Cpd 2 | (5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide | 1.03 (m, 3H), 1.31 (d, *J*=6.1 Hz, 6H), 1.70 (m, 1H), 1.96-2.12 (m, 3H), 2.33 (m, 1H), 2.54 (m, 1H), 2.81 (m, 1H), 3.31 (br d, *J*=13Hz, 1H), 3.58 (m, 1H), 3.60 (br d, *J*=9.0 Hz, 1 H), 3.78 (br d, *J*=9.0 Hz, 1 H), 4 50 (m, 1H), 4 51 (d, *J*=16.0 Hz, 1H), 4.65 (d, *J*=16.1 Hz, 1H), 6.74-6.77 (m, 3H), 7.13-7.28 (m, 5H), 7.38 (m, 1H), 8.78 (m, 2H); 541.1 (M+1) |
| 125 | 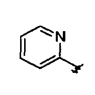 | Ex 4; Cpd 2 | (5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide | 1.14 (d, *J*=5.4 Hz, 3H), 1.32 (d, *J*=6.1 Hz, 6H), 1.7-1.77 (m, 1H), 1.97-2 18 (m, 3H), 2.32-2.39 (m, 1H), 2.60-2.68 (m, 1H), 2.80-2.87 (m, 1H), 3.34 (d, *J*=14.9 Hz, 1H), 3.64 (d, *J*=12.7 Hz, 1H), 4.07 (d, *J*=10.0 Hz, 1 H), 4.35 (d, *J*=10.3 Hz, 1 H), 4.48-4 54 (m, 1H), 6.73-6.80 (m, 3H), 7.03 (ddd, *J*=7.2, 5.0, 0 7 Hz, 1H), 7.15-7.23 (m, 3H), 7.25 (m, 1H, assumed, partially obscured by solvent peak), 7.39-7.48 (m, 2H), 7.66 (ddd, *J*=8.4, 7.3, 1.8 Hz, 1H), 8.35 (ddd, *J*=5.0, 1.8, 0 8 Hz, 1 H); 525.1 (M+1). |
| 126 | 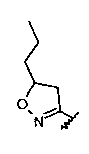 | Ex 4^{35$}; Cpd 2 | (5*R*,7*S*)-1-(3-fluorophenyl)-8-(3-isopropoxybenzyl)-7-methyl-3-(5-propyl-4,5-dihydroisoxazol-3-yl)-2-thia-1,3,8-triazaspiro[4.5]decane 2,2-dioxide | 0.97 (m, 3H), 1 07 (d, *J*=6.7 Hz, 3H), 1.31 (d, *J*=6.1Hz, 6H), 1.37-1 5 (m, 2H), 1.56-1.70 (m, 2H), 1.80 (m, 1H), 1.92-2.08 (m, 3H), 2.36 (m, 1H), 2.59 (m, 1H), 2.82 (m, 1H), 3.06 (m, 1H), 333 (br d, *J*=13.6Hz, 1H), 3.45 (m, 1H), 3.61 (br d, *J*=13.5 Hz, 1 H), 3.84 (br d, *J*=10.3Hz, 1 H), 4.04 (m, 1H), 4.51 (septet, *J*=6.1Hz, 1H), 4.73 (m, 1H), 6.74-6.78 (m, 3H), 7.11-7.24 (m, 4H), 7.43 (m, 1 H); 559.0 (M+1). |
| 127 | 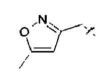 | B; Cpd 2 | (5R*,* 7S)-1-(3-fluorophenyl)-8-(3-isopropoxybenzyl)-7-methyl-3-[(5-methylisoxazol-3-yl)methyl]-2-thia-1,3,8-triazaspiro[4.5]decane 2,2-dioxide | 1.19 (d, *J*=6.2 Hz, 3H), 1.32 (d, *J*=5.8 Hz, 6H), 1.61 (t, *J*=13.7 Hz, 1H), 1.65-1.76 (m, 1H), 1.99 (d, 13.7 Hz, 2H), 2.26 (t, *J*=11.6Hz, 1 H), 2.44 (s, 3H), 2.53-2.62 (m, 1H), 2.63-2.70 (m, 1H), 3.04 (d, *J*=13.7 Hz, 1H), 3.48-3.62 (m, 1H), 3.70-3.81 (q, 2H), 4.09 (d, *J*=13.3 Hz, 1H), 4.5-4.58 (m, 1H), 6.38 (br s, 1H), 640-6.47 (m, 2H), 6.72-6.78 (m, 1H), 6.83-6.89 (m, 2H), 7.00-7.07 (q, 1H), 7.18 (t, *J*=7.9 Hz, 1H), 8.18 (s, 1H); 21.37, 22 16, 22.43, 33.78, 43.82, 47.78, 52.01, 52.27, 56.50, 58.35, 69.17, 69.97, 99.55, 99.81, 103.77, 105.09, 113.02, 114.07, 116.98, 121.47, 129.39, 130.18, 153.69, 162.40; APCI 543.2 (M+1), 479 (M-SO₂ +1). |
| 128 | 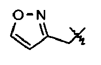 | B; Cpd 2 | (5*R*,7*S*)-1-(3-fluorophenyl)-8-(3-isopropoxybenzyl)-3-(isoxazol-3-ylmethyl)-7-methyl-2-thia-1,3,8-tnazaspiro[4.5]decane 2,2-dioxide | 0.99 (d, *J*=6.7 Hz, 3H), 1.30 (d, *J*=6.1 Hz, 6H), 1.61 (ddd, *J*=14, 5.4, 15 Hz, 1H), 1 82 (m, 1H), 193 (m, 1H), 2.02 (dd, *J*=13.7, 4.8 Hz, 1H), 2 29 (ddd, *J*=12.8, 6.3, 4.1 Hz, 1H), 2.46 (ddd, *J*=12.5, 8.9, 3.4 Hz, 1H), 2.78 (m, 1H), 3.28 (d, *J*=13.5 Hz, 1H), 3.34 (d, half of AB quartet, *J*=9.6 Hz, 1H), 3.45 (d, half of AB quartet, *J*=9.6 Hz, 1H), 3.55 (d, *J*=13.4 Hz, 1H), 4.38 (d, half of AB quartet, *J*=15.0 Hz, 1H), 4.45-4.54 (m, 2H), 6.57 (d, *J*=1.7 Hz, 1H), 6.73-6.75 (m, 3H), 7.12-7.22 (m, 4H), 7.41 (ddd, *J*=81, 81, 6.4 Hz, 1H), 8 45 (d, *J*=1.8 Hz, 1H); 529 4 (M+1). |
| 129 | 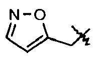 | B, Cpd 2 | (5*R*,7*S*)-1-(3-fluorophenyl)-8-(3-isopropoxybenzyl)-3-(isoxazol-5-ylmethyl-7-methyl-2-thia-1,3,8-triazaspiro[4.5]decane 2,2-dioxide | 1.02 (d, *J*=6.7 Hz, 3H), 1.31 (d, *J*=6.0 Hz, 6H), 1.63 (br dd, *J*=14, 5 Hz, 1H), 1.85 (m, 1H), 1.95 (m, 1H), 2.04 (dd, *J*=13.5, 4.7 Hz, 1H), 2.30 (m, 1H), 2.50 (m, 1H), 2.79 (m, 1H), 3.29 (d, half of AB quartet, *J*=13.4 Hz, 1H), 3.41 (d, half of AB quartet, *J*=9.4 Hz, 1H), 3 54 (d, half of AB quartet, *J*=9.4 Hz, 1H), 3 57 (d, half of AB quartet, *J*=13.4 Hz, 1H), 4 41 (d, half of AB quartet, *J*=15.6 Hz, 1H), 4.50 (septet, *J*=6.0 Hz, 1H), 4.54 (d, half of AB quartet, *J*=15.7 Hz, 1H), 6.41 (d, *J*=1.7 Hz, 1H), 6.73-6.75 (m, 3H), 7.11-7.21 (m, 4H), 7.41 (ddd, *J*=8.1, 8.1, 6.4 Hz, 1H), 8.29 (d, *J*=1.8 Hz, 1H); 529 0 (M+1). |
| 130 | 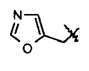 | B; Cpd 2 | (5*R*,7*S*)-1-(3-fluorophenyl)-8-(3-isopropoxybenzyl)-7-methyl-3-(1,3-oxazol-5-ylmethyl)-2-thia-1,3,8-triazaspiro[4.5]decane 2,2-dioxide | 1.01 (d, *J*=6.7 Hz, 3H), 1.31 (d, *J*=6.0 Hz, 6H), 1.63 (br dd, *J*=13.5, 5.5 Hz, 1 H), 1.84 (m, 1H), 1.95 (m, 1H), 2.05 (dd, *J*=13.5, 5.0 Hz, 1H), 2.29 (ddd, *J*=12.5, 6.5, 4.1 Hz, 1H), 2.48 (ddd, *J*=12.7, 8.6, 3.4 Hz, 1H), 2.78 (m, 1H), 3.28 (d, half of AB quartet, *J*=13.4 Hz, 1H), 3.33 (d, half of AB quartet *J*=9.3 Hz, 1H), 3.45 (d, half of AB quartet, *J*=9.4 Hz, 1H), 3.57 (d, half of AB quartet, *J*=13.6 Hz, 1H), 4.30 (dd, half of ABX pattern, *J*=15.2, 0.8 Hz, 1H), 4.44 (dd, half of ABX pattern, *J*=15.3, 0.8 Hz, 1H), 4.50 (septet, *J*=6.0 Hz, 1H), 6.73-6.76 (m, 3H), 712-7.21 (m, 5H), 7.40 (ddd, *J*=8.2, 8.2, 6.3 Hz, 1H), 7 93 (s, 1 H); 529.2 (M+1). |
| 131 | 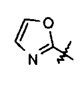 | Ex 4; Cpd 2 | (5*R*,7*S*)-1-(3-fluorophenyl)-8-(3-isopropoxybenzyl)-7-methyl-3-(1,3-oxazol-2-yl)-2-thia-1,3,8-triazaspiro[4.5]decane 2,2-dioxide | 1.11 (d, *J*=6.7 Hz, 3H), 1.32 (d, *J*=6.1 Hz, 6H), 1.71 (br dd, *J*=13 6, 6.0 Hz, 1H), 2.01 (m, 1H), 2.07-2 14 (m, 2H), 2.37 (ddd, *J*=12.8, 6.7, 3.7 Hz, 1H), 2.62 (ddd, *J*=12.6, 8.4, 3.5 Hz, 1H), 2.82 (m, 1H), 3.34 (d, half of AB quartet, *J*=13.5 Hz, 1H), 3.64 (d, half of AB quartet, *J*=13.5 Hz, 1H), 4.08 (d, half of AB quartet, *J*=9.9 Hz, 1H), 4.24 (d, half of AB quartet, *J*=9.8 Hz, 1H), 4.51 (septet, *J*=6.1 Hz, 1H), 6.75-6.78 (m, 3H), 7.01 (d, *J*=1.0 Hz, 1 H), 7.15-7.24 (m, 4H), 7.43 (ddd, *J*=8.1, 8.1, 6.2 Hz, 1H), 7.51 (d, *J*=1 1 Hz, 1H), 515.0 (M+1). |

| | | | | |
|---|---|---|---|---|
| 35$: The required bromide was prepared according to P. Caldirola et al., Tetrahedron Letters 1986, 27, 4647-4650. | | | | |

A synthetic APP substrate that can be cleaved by beta-secretase and having N-terminal biotin is used to assay beta-secretase activity in the presence or absence of the inhibitory compounds. The substrate can contain either the wildtype sequence around the BACE cleavage site or the Swedish mutation (Vassar, R., B. D. Bennett, et al. (1999). "Beta-secretase cleavage of Alzheimer's amyloid precursor protein by the transmembrane aspartic protease BACE." Science. 286(5440): 735-741). The substrate and test compounds are added to 384 well polypropylene plates. The reaction is initiated by the addition of soluble BACE enzyme to a final volume of 12.5 µL per well. The final assay conditions are: 0.001 - 300 µM compound inhibitor, 0.05M sodium acetate (pH 4.5), 3µM substrate, soluble human BACE, and 2% DMSO. Concentrated conditioned media from cells secreting human recombinant soluble BACE was titrated to provide a source of BACE enzyme. The cell media can be used as either a crude BACE prep or BACE can be purified using any number of techniques, including immobilized BACE inhibitor purification columns. The assay mixture is incubated for 1 hour at 37°C, and the reaction is quenched by the addition of an equal volume of 0.1M Tris, pH 8. Half of the quenched mix is incubated on clear streptavidin coated 384 well polystyrene plates for 1 hour. An ELISA is then performed using an in-house antibody that specifically recognizes the new C-terminus created after cleavage by BACE. Two in-house antibodies are available; each is cleavage specific, but one is raised against the wildtype sequence (APP 591-596) while the other is raised against the Swedish mutation (APP 590-596). (These polyclonal antibodies were raised in rabbits by immunizing with antigen comprised of six amino acid residues present at the carboxy terminus of the wild-type soluble APPbeta sequence (NH₂-ISEVKM-COOH) or seven amino acid residues present at the carboxy terminus of the Swedish mutation at the beta cleavage site (NH₂-EISEVNL-COOH) conjugated to keyhole limpet hemacyanin by methods known to those skilled in the art.) A secondary anti-species Horseradish Peroxidase (HRP) conjugated antibody is then utilized. The readout, following assay development with TMB substrate and quenching with 0.09M sulfuric acid, is absorbance at 450 nm.

**Table 4. Biological data**

| Example # | Inhibition of BACE IC₅₀ (uM) |
|---|---|
| 1a | 1.30 |
| 2 | 7.24 |
| 17 | 1.51 |
| 83 | 102 |

## Claims

1. A compound of formula I: 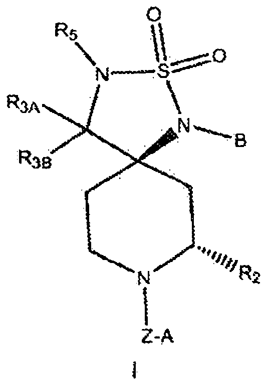 wherein the stereochemistry shown in formula I at the carbon bonded to R₂ and at the spirocyclic carbon is the absolute stereochemistry; B is aryl, heteroaryl, cycloalkyl, or heterocycloalkyl, wherein B is optionally substituted with zero to three R₄ groups;
Z is (CH₂)ₙ-Oₚ-(CH₂)_{q}, or a cycloalkylene moiety, provided that wen Z is (CH₂)ₙ-Oₚ-(CH₂)_{q}, the terminal methylene of the (CH₂)ₙ-chain is bonded to the nitrogen of the piperidinyl ring;
A is independently aryl, cycloalkyl, heterocycloalkyl or heteroaryl wherein said aryl, cycloalkyl, heterocycloalkyl or heteroaryl is optionally substituted with one to three R₁;
each R₁ is independently alkyl, halogen, cyano, SO₂NHR₉, CON(R₈)₂, N(R₈)COR₈, SO₂N(R₈)₂, N(R₈)SO₂R₈, COR₈, SO₂R₈, (CH₂)ᵣ-cycloalkyl, (CH₂)ₜ-heterocycloalkyl, (CH₂)ₜ-aryl, (CH₂)ₜ-heteroaryl, (CH₂)ₜ-N(R₉)₂, or (CH₂)ₜ-OR₆ wherein each R₁ alkyl, (CH₂)ₜ-cycloalkyl, (CH₂)ₜ-heterocycloalkyl, (CH₂)ₜ-aryl, or (CH₂)ₜ-heteroaryl is optionally independently substituted by cyano, alkyl, halogen, or OR₆;
R₂ is alkyl, cycloalkyl, or alkenyl wherein said alkyl, cycloalkyl, or alkenyl is optionally substituted with halogen, hydroxyl, or cyano;
R_{3A} and R_{3B} are each Independently hydrogen, aryl, heteroayl, or alkyl optionally substituted with R₁;
or R_{3A} and R_{3B} together with the carbon they are bonded to form a C=O, C=NR₈, a cycloalkylene moiety or a heterocycloalkylene moiety;
each R₄ is independently halogen, hydroxyl, cyano, halo, O-alkyl, O-cycloalkyl, SO₂R₈, N(R₈)₂, COR₈, CON(R₈)₂, alkyl, (CH₂)ₜ-cycloalkyl, (CH₂)ₜ-heterocycloalkyl, (CH₂)ₜ-aryl, or (CH₂)ₜ-heteroaryl wherein said R₄ alkyl, (CH₂)ₜ-cycloalkyl, (CH₂)ₜ-heterocycloalkyl, (CH₂)ₜ-aryl, or (CH₂)ₜ-heteroaryl is optionally substituted with R₁;
each R₅ is independently hydrogen, alkyl, alkenyl, (CH₂)ₜ-cycloalkyl, (CH₂)-heterocyloalkyl, (CH₂)ₜ-aryl, or (CH₂)ₜ-heteroaryl, wherein said alkyl, alkenyl, (CH₂)ₜcycloalkyl, (CH₂)ₜ-heterocycloalkyl, (CH₂)ₜ-aryl, or (CH₂)ₜ-heteroaryl R₅ substituent is Optionally substituted with one or more hydroxyl, aryl, heteroaryl, halogen, alkyl, cycloalkyl, SO₂R₈ -NR₈COR₈, -CON(R₈)₂, COOR₈, -C(O)R₈, -CN, or N(R₈)₂, wherein said aryl, alkyl, cycloalkyl and heteroaryl substituents is optionally substituted with one or more halogen, alkyl, hydroxyl, or -O-alkyl;
each R₆ is independently hydrogen, alkyl, (CH₂)ₜ-cycloalkyl, (CH₂)ₜ-heterocycloalkyl, (CH₂)ₜ-aryl, or (CH₂)ₜ-heteroaryl wherein said (CH₂)ₜ-cycloalkyl, (CH₂)ₜ-heterocycloalkyl, (CH₂)ₜ-aryl, or (CH₂)ₜ-heteroaryl is optionally substituted with one to three R₇;
each R₇ is independently alkyl, hydroxyl, alkoxy, halogen, cyano, amino, alkylamino, dialkylamino, (CH₂)ₜ-cycloalkyl, (CH₂)ₜ-heterocycloalkyl, (CH₂)ₜ-aryl, or (CH₂)ₜ-heteroaryl;
each R₈ is independently hydrogen, alkyl, (CH₂)ₜ-cycloalkyl, (CH₂)ₜ-heterocycloalkyl, (CH₂)ₜ-aryl, or (CH₂)ₜ-heteroaryl, wherein said (CH₂)ₜ-cycloalkyl, (CH₂)ₜ-heterocycloalkyl, (CH₂)ₜ-aryl, or (CH₂)ₜ-heteroaryl are optionally substituted with alkyl, halo, or cyano;
Rg is hydrogen or alkyl;
n is an integer selected from 1. 2 and 3;
p is an integer selected from 0 and 1, provided that if p is 1, then n is 2 or 3;
q is an integer selected from 0 and 1, provided that if p is 0, then q is 0; each t is an integer independently selected from 0, 1, 2 and 3; or pharmaceutically acceptable salts thereof,

2. A compound according to claim 1 or pharmaceutically acceptable salt thereof wherein A is aryl, heteroaryl, cycloalkyl or heterocycloalkyl, and A is optionally substituted with one R₁ substituent.

3. A compound according to claim 1 or pharmaceutically acceptables salt thereof wherein A is aryl, heteroaryl, cycloalkyl or heterocycloalkyl, and A is optionally substituted with two R₁ substituents.

4. A compound according to claim 3 or pharmaceutically acceptable salt thereof wherein A is aryl and is optionally substituted with two R₁ substituents.

5. A compound according to claim 3 or pharmaceutically acceptable salt thereof wherein A is heteroaryl and is optionally substituted with two R₁ substituents.

6. A compound or pharmaceutically acceptable salt thereof as in any of the proceeding claims wherein R₁ is independently alkyl, halogen, cyano, -N(R₈)COR₈, -COR₈, -(CH₂)ₜ-cycloalkyl, -(CH₂)ₜ-heterocycloalkyl, -(CH₂)ₜ-aryl, -(CH₂)ₜ-heteroaryl, -(CH₂)ₜ-(R₈)₂, or -(CH₂)ₜ-OR₆, wherein each R₁ alkyl, -(CH₂)ₜ-cycloalkyl, -(CH₂)ₜ-heterocycloalkyl, (CH₂)ₜ-aryl, or (CH₂)ₜ-heteroaryl is optionally independently substituted by cyano, alkyl, halogen, or -OR₆.

7. A compound or pharmaceutically acceptable salt thereof as in any one of the preceding claims wherein B is aryl and is substituted with only one R₄ substituent and R₄ is halogen.

8. A compound or pharmaceutical acceptable salt thereof as in any one of the preceding claim wherein Z is (CH₂)ₙ-Oₚ-(CH₂)_{q} and p is 0.

9. A compound or Pharmaceutical acceptable salt thereof as in any one of the preceding claims wherein R₂ is alkyl.

10. A compound or pharmaceutical acceptable salt thereof as in any one of the preceding claims wherein R_{3A} and R_{3B} are each hydrogen.

11. A compound according or pharmaceutically acceptable salt thereof as in any one of the preceding claims wherein R₅ is independently selected from the group consisting of hydrogen and alkyl

12. A compound of formula I according to claim 1 from the group consisting of:
(5R,7S)-1-(3-fluorophenyl)-8-(3-isopropoxybenzyl)-7-methyl-2-thia-1,3,8-triazaspiro[4.6]decane 2.2-dioxide;
(5*R*,7*S*)-1-(3-fluorophenyl)-8-(3-isopropoxybenzyl)-7-methyl-3-(pyridin-2-ylmethyl)-2-thia-1,3,8-triazaspiro[4.5]decane 2,2-dioxide;
5-{[(5*R*,7*S*)-1-(3-fluorophenyl)-3,7-dimethyl-2,2-dioxido-2-thia-1,3,8 triazaspiro[4.5]dec-8-yl]methyl}-2'-methylbiphenyl-2-ol;
(5*R*,7*S*)-1-(3-fluorophenyl)-7-methyl-8-[3-(4-methylpyridin-3-yl)benzyl]-2-thia-1,3,8-triazaspiro[4.5]decane 2,2-dioxide;
2-chloro-4-{[(5*R*,7*S*)-1-(3-fluorophenyl)-7-methyl-2,2-dioxido-2-thia-1,3,8-triazaspiro[4.5]dec-8-yl]methyl}phenol;
(5*R*,7*S*)-1-(3-fluorophenyl)-7-methyl-8-[(2'-methylbiphenyl-3-yl)methyl]-2-thia-1,3,8-triazaspiro[4.5]decane 2,2-dioxide;
(5*R*,7*S*)-8-[(2'-chlorobiphenyl-3-yl)methyl]-1-(3-fluorophenyl)-7-methyl-2-thia-1,3,8-triazaspiro[4.5]decane 2,2-dioxide;
2-ethoxy-4-{[(5*R*,7*S*)-1-(3-fluorophenyl)-7-methyl-2,2-dioxido-2-thia-1,3,8-triazaspiro[4,5]dec-8-yl]methyl}phenol;
(5*R*,7*S*)-8-[(2'-ethylbiphenyl-3-yl)methyl]-1-(3-fluorophenyl)-7-methyl-2-thia-1,3,8-triazaspiro[4.5]decane 2,2-dioxide;
(5*R*,7*S*)-1-(3-fluorophenyl)-7-methyl-8-(3-{[(1R)-1-methylpropyl]oxy}benzyl)-2-thia-1,3,8-triazaspiro[4.5]decane 2,2-dioxide;
4-{[(5*R*,7*S*)-1-(3-fluorophenyl)-3,7-dimethyl-2,2-dioxido-2-thia-1,3.8-triazaspiro[4.5]dec-8-yl]methyl}-2-isopropoxyphenol;
(5*R*,7*S*)-1-(3-fluorophenyl)-3,7-dimethyl-8-[(2'-methylbiphenyl-3-yl)methyl]-2-thia-1,3,8-triazaspiro[4.5]decane 2,2-dioxide;
(5*R*,7*S*)-8-{[4-(cyclopropylmethyl)-1,3-thiazol-5-yl]methyl}-1-(3-fiuorophenyl)-3,7-dimethyl-2-thia-1,3,8-triazaspiro[4.5]decane 2,2-dioxide:
(5*R*,7*S*)-1-(3-fluorophenyl)-8-[(4-isobutyl-1,3-thiazol-5-yl)methyl]-3,7-dimethyl-2-thia-1,3,8-triazaspiro[4.5]decane 2,2-dioxide;
(5*R*,7*S*)-8-{[4-(cyclobutylmethyl)-1,3-thiazol-5-yl]methyl}-1-(3-fluorophenyl)-3,7-dimethyl-2-thia-1,3,8-triazaspiro[4.5]decane 2,2-dioxide;
(5*R*,7*S*)-1-(3-fluorophenyl)-3,7-dimethyl-8-{3-[(2-methyl-1,3-benzoxazol-6-yl)oxy]benzyl}-2-thia-1,3,8-triazaspiro[4.5]decane 2,2-dioxide;
(5*R*,7*S*)-8-[(3-ethyl-1H-indazol-5-yl)methyl]-1-(3-fluorophenyl)-3,7-dimethyl-2-thia-1,3,8-triazaspiro[4.5]decane 2,2-dioxide;
(5*R*,7*S*)-1-(3-fluorophenyl)-3,7-dimethyl-8-{[3-(trifluoromethyl)-1H-indazol-5-yl]methyl}-2-thia-1,3,8-triazaspiro[4.5]decane 2,2-dioxide;
2'-ethyl-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol;
2'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol;
5'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol;
4'-fluoro-5-{[(5*R*,7*S*)-1-(3-fluorophenyl)-3,7-dimethyl-2,2-dioxido-2-thia-1,3,8-triazaspiro[4.5]dec-8-yl]methyl}-2'-methylbiphenyl-2-ol;
2'-chloro-5-{[(5*R*,7*S*)-1-(3-fluorophenyl)-3,7-dimethyl-2,2-dioxido-2-thia-1,3,8-triazaspiro[4.5]dec-8-yl]methyl}biphenyl-2-ol;
6-{[(5*R*,7*S*)-1-(3-fluorophenyl)-3,7-dimethyl-2,2-dioxido-2-thia-1,3,8-triazaspiro[4.5]dec-8-yl]methyl}-4-isopropoxypyridin-3-ol;
2-cyclopentyl-4{[(5*R*,7*S*)-1-(3-fluorophenyl)-3,7-dimethyl-2,2-dioxido-2-thia-1,3,8-triazaspiro[4.5]dec-8-yl]methyl}phenol;
4-{[(5*R*,7*S*)-1-(3-fluorophenyl)-3,7-dimethyl-2,2-dioxido-2-thia-1,3,8-triazaspiro[4.5]dec-8-yl]methyl}-2-isobutylphenol;
2-cyclohexyl-4-{[(5*R*,7*S*)-1-(3-fluorophenyl)-3,7-dimethyl-2,2-dioxido-2-thia-1,3,8-triazaspiro[4.5]dec-8-yl]methyl}phenol;
(5*R*,7*S*)-1-(3-flurophenyl)-8-(3-isopropoxybenzyl)-7-methyl-3-pyrimidin-2-yl-2-thia-1,3,8-triazaspiro[4.5]decane 2,2-dioxide;
(5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide;
(5*R*,7*S*)-1-(3-fluorophenyl)-8-(3-isopropoxybenzyl)-7-methyl-3-(pyrimidin-2-ylmethyl)-2-thia-1,3,8-triazaspiro[4.5]decane 2,2-dioxide;
(5*R*,7*S*)-1-(3-fluorophenyl)-8-(3-isopropoxybenzyl)-3-(isoxazol-3-ylmethyl)-7-methyl-2-thia-1,3,8-triazaspiro[4.5]decane 2,2-dioxide; or pharmaceutically acceptable salts thereof.

13. A compound according to any one of claims 1 to 12 for use in the treatment of a disease or condition selected from the group consisting of neurological and psychiatric disorders.

14. A compound for use according to claim 13 wherein the disease is Alzeimer's disease.

15. A pharmaceutical composition comprising a compound of claim 1 or a pharmaceutically acceptable salt thereof and a pharmaceutically acceptable carrier.

16. The composition of claim 15 further comprising an atypical antipsychotic, a cholinesterase inhibitor, dimebon or NMDA receptor antagonist.

## Patentansprüche

1. Verbindung der Formel I: 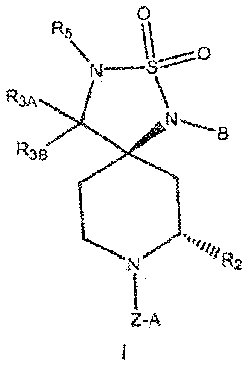 worin die in Formel I gezeigte Stereochemie an dem an R₂ gebundenen Kohlenstoff und an dem spirocyclischen Kohlenstoff die absolute Stereochemie ist; B Aryl, Heteroaryl, Cycloalkyl oder Heterocycloalkyl ist, wobei B gegebenenfalls mit null bis drei R₄-Gruppen substituiert ist;
Z (CH₂)ₙ-Oₚ-(CH₂)_{q} oder eine Cycloalkylengruppierung ist, mit der Maßgabe, dass, wenn Z (CH₂)ₙ-Oₚ-(CH₂)_{q} ist, das terminale Methylen der (CH₂)ₙ-Kette an den Stickstoff des Piperidinylrings gebunden ist;
A unabhängig Aryl, Cycloalkyl, Hetercycloalkyl oder Heteroaryl ist, wobei das Aryl, Cycloalkyl, Heterocycloalkyl oder Heteroaryl gegebenenfalls mit ein bis drei R₁ substituiert ist;
jedes R₁ unabhängig Alkyl, Halogen, Cyano, SO₂NHR₉, CON(R₈)₂, N(R₈)COR₈, SO₂N(R₈)₂, N(R₈)SO₂R₈, COR₈, SO₂R₈, (CH₂)ₜ-Cycloalkyl, (CH₂)ₜ-Heterocycloalkyl, (CH₂)ₜ-Aryl, (CH₂)ₜ-Heteroaryl, (CH₂)ₜ-N(R₈)₂ oder (CH₂)ₜ-OR₆ ist, wobei jedes Alkyl, (CH₂)ₜ-Cycloalkyl, (CH₂)ₜ-Heterocycloalkyl (CH₂)ₜ-Aryl oder (CH₂)ₜ-Heteroaryl für R₁ gegebenenfalls unabhängig mit Cyano, Alkyl, Halogen oder OR₆ substituiert ist;
R₂ Alkyl, Cycloalkyl oder Alkenyl ist, wobei das Alkyl, Cycloalkyl oder Alkenyl gegebenenfalls mit Halogen, Hydroxyl oder Cyano substituiert ist;
R_{3A} und R_{3B} jeweils unabhängig Wasserstoff, Aryl, Heteroaryl oder Alkyl, gegebenenfalls substituiert mit R₁, sind;
oder R_{3A} und R_{3B} zusammen mit dem Kohlenstoff, an den sie gebunden sind, ein C=O, C=NR₈ oder eine Cycloalkylengruppierung oder eine Heterocycloalkylengruppierung bilden;
jedes R₄ unabhängig Halogen, Hydroxyl, Cyano, Halogen, O-Alkyl, O-Cycloalkyl, SO₂R₈, N(R₈)₂, COR₈, CON(R₈)₂, Alkyl, (CH₂)ₜ-Cycloalkyl, (CH₂)ₜ-Heterocycloalkyl, (CH₂)ₜ-Aryl oder (CH₂)ₜ-Heteroaryl ist, wobei das Alkyl, (CH₂)ₜ-Cycloalkyl, (CH₂)ₜ-Heterocycloalkyl, (CH₂)ₜ-Aryl oder (CH₂)ₜ-Heteroaryl gegebenenfalls mit R₁ substituiert ist;
jedes R₅ unabhängig Wasserstoff, Alkyl, Alkenyl, (CH₂)ₜ-Cycloalkyl, (CH₂)ₜ-Heterocycloalkyl, (CH₂)ₜ-Aryl oder (CH₂)ₜ-Heteroaryl ist, wobei der Alkyl-, Alkenyl-, (CH₂)ₜ-Cycloalkyl-, (CH₂)ₜ-Heterocycloalkyl-, (CH₂)ₜ-Aryl- oder (CH₂)ₜ-Heteroaryl-R₅-Substituent gegebenenfalls mit einem oder mehreren Hydroxyl, Aryl, Heteroaryl, Halogen, Alkyl, Cycloalkyl, SO₅R₈, -NR₈COR₈, -CON(R₈)₂, COOR₈, -C(O)R₈ oder N (R₈)₂ substituiert ist, wobei der Aryl-, Alkyl-, Cycloalkyl- und Heteroaryl-Substituent gegebenenfalls mit einem oder mehreren Halogen, Alkyl, Hydroxyl oder -O-Alkyl substituiert ist;
jedes R₆ unabhängig Wasserstoff, Alkyl, (CH₂)ₜ-Cycloalkyl, (CH₂)ₜ-Heterocycloalkyl, (CH₂)ₜ-Aryl oder (CH₂)ₜ-Heteroaryl ist, wobei das (CH₂)ₜ-Cycloalkyl, (CH₂)ₜ-Heterocycloalkyl, (CH₂)ₜ-Aryl oder (CH₂)ₜ-Heteroaryl gegebenenfalls mit ein bis drei R₇ substituiert ist;
jedes R₇ unabhängig Alkyl, Hydroxyl, Alkoxy, Halogen, Cyano, Amino, Alkylamino, Dialkylamino, (CH₂)ₜ-Cycloalkyl, (CH₂)ₜ-Heterocycloalkyl, (CH₂)ₜ-Aryl oder (CH₂)ₜ-Heteroaryl ist;
jedes R₈ unabhängig Wasserstoff, Alkyl, (CH₂)ₜ-Cycloalkyl, (CH₂)ₜ-Heterocycloalkyl, (CH₂)ₜ-Aryl oder (CH₂)ₜ-Heteroaryl ist, wobei das (CH₂)ₜ-Cycloalkyl, (CH₂)ₜ-Heterocycloalkyl, (CH₂)ₜ-Aryl oder (CH₂)ₜ-Heteroaryl gegebenenfalls mit Alkyl, Halogen oder Cyano substituiert ist;
R₉ Wasserstoff oder Alkyl ist;
n eine ganze Zahl, ausgewählt aus 1, 2 und 3, ist;
p eine ganze Zahl, ausgewählt aus 0 und 1, ist, mit der Maßgabe, dass, wenn p 1 ist, dann n 2 oder 3 ist;
q eine ganze Zahl, ausgewählt aus 0 und 1, ist, mit der Maßgabe, dass, wenn p 0 ist, dann q 0 ist;
jedes t eine ganze Zahl, unabhängig ausgewählt aus 0, 1, 2 und 3, ist;
oder pharmazeutisch verträgliche Salze davon.

2. Verbindung gemäß Anspruch 1 oder pharmazeutisch verträgliches Salz davon, wobei A Aryl, Heteroaryl, Cycloalkyl oder Heterocycloalkyl ist und A gegebenenfalls mit einem R₁-Substituenten substituiert ist.

3. Verbindung gemäß Anspruch 1 oder pharmazeutisch verträgliches Salz davon, wobei A Aryl, Heteroaryl, Cycloalkyl oder Heterocycloalkyl ist und A gegebenenfalls mit zwei R₁-Substituenten substituiert ist.

4. Verbindung gemäß Anspruch 3 oder pharmazeutisch verträgliches Salz davon, wobei A Aryl ist und gegebenenfalls mit zwei R₁-Substituenten substituiert ist.

5. Verbindung gemäß Anspruch 3 oder pharmazeutisch verträgliches Salz davon, wobei A Heteraryl ist und gegebenenfalls mit zwei R₁-Substituenten substituiert ist.

6. Verbindung oder pharmazeutisch verträgliches Salz davon gemäß einem der vorangehenden Ansprüche, wobei R₁ unabhängig Alkyl, Halogen, Cyano, -N(R₈)COR₈, -COR₈, -(CH₂)ₜ-Cycloalkyl, - (CH₂)ₜ-Heterocycloalkyl, -(CH₂)ₜ-Aryl, -(CH₂)ₜ-Heteroaryl, -(CH₂)ₜ-N(R₈)₂ oder -(CH₂)ₜ-OR₆ ist, wobei jedes Alkyl, -(CH₂)ₜ-Cycloalkyl, - (CH₂)ₜ-Heterocycloalkyl, (CH₂)ₜ-Aryl, (CH₂)ₜ-Heteroaryl gegebenenfalls unabhängig mit Cyano, Alkyl, Halogen oder -OR₆ substituiert ist.

7. Verbindung oder pharmazeutisch verträgliches Salz davon gemäß einem der vorangehenden Ansprüche, wobei B Aryl ist und mit nur einem R₄-Substituenten substituiert ist und R₄ Halogen ist.

8. Verbindung oder pharmazeutisch verträgliches Salz davon gemäß einem der vorangehenden Ansprüche, wobei Z (CH₂)ₙ-Oₚ-(CH₂)_{q} ist und p 0 ist.

9. Verbindung oder pharmazeutisch verträgliches Salz davon gemäß einem der vorangehenden Ansprüche, wobei R₂ Alkyl ist.

10. Verbindung oder pharmazeutisch verträgliches Salz davon gemäß einem der vorangehenden Ansprüche, wobei R_{3A} und R_{3B} jeweils Wasserstoff sind.

11. Verbindung oder pharmazeutisch verträgliches Salz davon gemäß einem der vorangehenden Ansprüche, wobei R₅ unabhängig ausgewählt ist aus der Gruppe, bestehend aus Wasserstoff und Alkyl.

12. Verbindung der Formel I gemäß Anspruch 1, ausgewählt aus der Gruppe, bestehend aus:
(5R, 7S)-1-(3-Fluorphenyl)-8-(3-isopropoxybenzyl)-7-methyl-2-thia-1,3,8-triazaspiro[4.5]decan-2,2-dioxid;
(5R, 7S)-1-(3-Fluorphenyl)-8-(3-isopropoxybenzyl)-7-methyl-3-(pyridin-2-ylmethy)-2-thia-1,3,8-triazaspiro[4.5]decan-2,2-dioxid;
5-{[(5R, 7S)-1-(3-Fluorphenyl)-3,7-dimethyl-2,2-dioxido-2-thia-1,3,8-triazaspiro[4.5]dec-8-yl]methyl}-2'-methylbiphenyl-2-ol;
(5R, 7S)-1-(3-Fluorphenyl)-7-methyl-8-[3-(4-methylpyridin-3-yl)benzyl]-2-thia-1,3,8-triazaspiro[4.5]decan-2,2-dioxid;
2-Chlor-4-{[5R, 7S)-1-(3-fluorphenyl)-7-methyl-2,2-dioxido-2-thia-1,3,8-triazaspiro[4.5]dec-8-yl]methyl}phenol;
(5R, 7S)-1-(3-Fluorphenyl)-7-methyl-8-[(2'-methylbiphenyl-3-yl)methyl]-2-thia-1,3,8-triazaspiro[4.5]decan-2,2-dioxid;
(5R, 7S)-8-[(2'-Chlorbiphenyl-3-yl)methyl]-1-(3-fluorphenyl)-7-methyl-2-thia-1,3,8-triazaspiro[4.5]decan-2,2-dioxid;
2-Ethoxy-4-{[5R, 7S)-1-(3-fluorphenyl)-7-methyl-2,2-dioxido-2-thia-1,3,8-triazaspiro[4.5]dec-8-yl]methyl}phenol;
(5R, 7S)-8-[(2'-Ethylbiphenyl-3-yl)methyl]-1-(3-fluorphenyl)-7-methyl-2-thia-1,3,8-triazaspiro[4.5]decan-2,2-dioxid;
(5R, 7S)-1-(3-Fluorphenyl)-7-methyl-8-(3-{[(1R)-1-methylpropyl]oxy}benzyl)-2-thia-1,3,8-triazaspiro[4.5]decan-2,2-dioxid;
4-{[(5R, 7S)-1-(3-Fluorphenyl)-3,7-dimethyl-2,2-dioxido-2-thia-1,3,8-triazaspiro[4.5]dec-8-yl]methyl}-2-isopropoxyphenol;
(5R, 7S)-1-(3-Fluorphenyl)-3,7-dimethyl-8-[(2'-methylbiphenyl-3-yl)methyl]-2-thia-1,3,8-triazaspiro[4.5]decan-2,2-dioxid;
(5R, 7S)-8-{[4-(Cyclopropylmethyl)-1,3-thiazol-5-yl]methyl}-(3-fluorphenyl)-3,7-dimethyl-2-thia-1,3,8-triazaspiro[4.5]decan-2,2-dioxid;
(5R, 7S)-1-(3-Fluorphenyl)-8-[(4-isobutyl-1,3-thiazol-5-yl)methyl]-3,7-dimethyl-2-thia-1,3,8-triazaspiro[4.5]decan-2,2-dioxid;
(5R, 7S)-8-{[4-(Cyclobutylmethyl)-1,3-thiazol-5-yl]methyl}-1-(3-fluorphenyl)-3,7-dimethyl-2-thia-1,3,8-triazaspiro[4.5]decan-2,2-dioxid;
(5R, 7S)-1-(3-Fluorphenyl)-3,7-dimethyl-8-{3-[(2-methyl-1,3-benzoxazol-6-yl)oxy]benzyl}-2-thia-1,3,8-triazaspiro[4.5]decan-2,2-dioxid;
(5R, 7S)-8-[(3-Ethyl-1H-indazol-5-yl)methyl]-1-(3-fluorphenyl)-3,7-dimethyl-2-thia-1,3,8-triazaspiro[4.5]decan-2,2-dioxid;
(5R, 7S)-1-(3-Fluorphenyl)-3,7-dimethyl-8-{[3-(trifluormethyl)-1H-indazol-5-yl]methyl}-2-thia-1,3,8-triazaspiro[4.5]decan-2,2-dioxid;
2'-Ethyl-5-{[(5R, 7S)-1-(3-fluorphenyl)-3,7-dimethyl-2,2-dioxido-2-thia-1,3,8-triazaspiro[4.5]dec-8-yl]methyl}biphenyl-2-ol;
2'-Fluor-5-{[(5R, 7S)-1-(3-fluorphenyl)-3,7-dimethyl-2,2-dioxido-2-thia-1,3,8-triazaspiro[4.5]dec-8-yl]methyl}biphenyl-2-ol;
5'-Fluor-5-{[(5R, 7S)-1-(3-fluorphenyl)-3,7-dimethyl-2,2-dioxido-2-thia-1,3,8-triazaspiro[4,5]dec-8-yl]methyl)-2'-methylbiphenyl-2-ol;
4'-Fluor-5-{[(5R, 7S)-1-(3-fluorphenyl)-3,7-dimethyl-2,2-dioxido-2-thia-1,3,8-triazaspiro[4.5]dec-8-yl]methyl}-2'-methylbiphenyl-2-ol;
2'-Chlor-5-{[(5R, 7S)-1-(3-fluorphenyl)-3,7-dimethyl-2,2-dioxido-2-thia-1,3,8-triazaspiro[4.5]dec-8-yl]methyl}biphenyl-2-ol;
6-{[(5R, 7S)-1-(3-Fluorphenyl)-3,7-dimethyl-2,2-dioxido-2-thia-1,3,8-triazaspiro[4.5]dec-8-yl]methyl}-4-isopropoxypyridin-3-ol;
2-Cyclopentyl-4-{[(5R, 7S)-1-(3-fluorphenyl)-3,7-dimethyl-2,2-dioxido-2-thia-1,3,8-triazaspiro[4.5]dec-8-yl]methyl}phenol;
4-{[(5R, 7S)-1-(3-Fluorphenyl)-3,7-dimethyl-2,2-dioxido-2-thia-1,3,8-triazaspiro[4.5]dec-8-yl]methyl}-2-isobutylphenol;
2-Cyclohexyl-4-{[(5R, 7S)-1-(3-fluorphenyl)-3,7-dimethyl-2,2-dioxido-2-thia-1,3,8-triazaspiro[4.5]dec-8-yl]methyl}phenol;
(5R, 7S)-1-(3-Fluorphenyl)-8-(3-isopropoxybenzyl)-7-methyl-3-pyrimidin-2-yl-2-thia-1,3,8-triazaspiro[4.5]decan-2,2-dioxid;
(5R, 7S)-1-(3-Fluorphenyl)-8-(3-isopropoxybenzyl)-7-methyl-3-(pyrimidin-2-ylmethyl)-2-thia-1,3,8-triazaspiro[4.5]decan-2,2-dioxid;
(5R, 7S)-1-(3-Fluorphenyl)-8-(3-isopropoxybenzyl)-7-methyl-3-(pyrimidin-2-ylmethyl)-2-thia-1,3,8-triazaspiro[4.5]decan-2,2-dioxid;
(5R, 7S)-1-(3-Fluorphenyl)-8-(3-isopropoxybenzyl)-3-(isoxazol-3-ylmethyl)-7-methyl-2-thia-1,3,8-triazaspiro(4.5)decan-2,2-dioxid,
oder pharmazeutisch verträgliche Salze davon.

13. Verbindung gemäß einem der Ansprüche 1 bis 12 zur Verwendung bei der Behandlung einer Krankheit oder eines Zustandes, ausgewählt aus der Gruppe, bestehend aus neurologischen und psychiatrischen Störungen.

14. Verbindung zur Verwendung gemäß Anspruch 13, wobei die Krankheit Alzheimer'sche Krankheit ist.

15. Pharmazeutische Zusammensetzung, umfassend eine Verbindung gemäß Anspruch 1 oder ein pharmazeutisch verträgliches Salz davon und einen pharmazeutisch verträglichen Träger.

16. Zusammensetzung gemäß Anspruch 15, die außerdem ein atypisches Antipsychiotikum, einen Cholinesteraseinhibitor, Dimebon oder NMDA-Rezeptor-Antagonisten umfasst.

## Revendications

1. Composé de formule I : 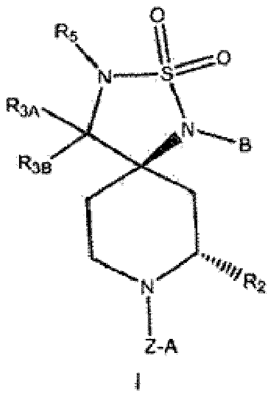 dans laquelle la stéréochimie illustrée dans la formule I au carbone lié à R₂, et au carbone spirocyclique est la stéréochimie absolue ; B représente un groupe aryle, hétéroaryle, cycloalkyle ou hétérocycloalkyle, où B est éventuellement substitué par zéro à trois groupes R₄ ;
Z représente (CH₂)ₙ-Oₚ-(CH₂)_{q}, ou un radical cycloalkylène, à condition que lorsque Z représente (CH₂)ₙ-Oₚ-(CH₂)_{q}, le méthylène terminal de la chaîne de (CH₂)ₙ soit lié à l'azote du cycle pipéridinyle ;
A représente indépendamment un groupe aryle, cycloalkyle, hétérocycloalkyle ou hétéroaryle où ledit groupe aryle, cycloalkyle, hétérocycloalkyle ou hétéroaryle est éventuellement substitué par un à trois R₁ ;
chaque R₁ représente indépendamment un groupe alkyle, un atome d'halogène, un groupe cyano, SO₂NHR₉, CON(R₈)₂, N(R₈)COR₈, SO₂N(R₈)₂, N(R₈)SO₂R₈, COR₈, SO₂R₈, (CH₂)ₜ-cycloalkyle, (CH₂)ₜ-hétérocycloalkyle, (CH₂)ₜ-aryle, (CH₂)ₜ-hétéroaryle, (CH₂)ₜ-N(R₈)₂ ou (CH₂)ₜ-OR₆, où chaque R₁ alkyle, (CH₂)ₜ-cycloalkyle, (CH₂)ₜ-hétérocycloalkyle, (CH₂)ₜ-aryle ou (CH₂)ₜ-hétéroaryle est éventuellement substitué indépendamment par un groupe cyano, alkyle, un atome d'halogène ou OR₆ ;
R₂ représente un groupe alkyle, cycloalkyle ou alcényle où ledit groupe alkyle, cycloalkyle ou alcynyle est éventuellement substitué par un atome d'halogène, un groupe hydroxyle ou cyano ;
R_{3A} et R_{3B} représentent chacun indépendamment un atome d'hydrogène, un groupe aryle, hétéroaryle ou alkyle éventuellement substitué par R₁ ;
ou R_{3A} et R_{3B} conjointement avec le carbone auquel ils sont liés, forment un C=O, C=NR₈, un radical cycloalkylène ou un radical hétérocycloalkylène ;
chaque R₄ représente indépendamment un atome d'halogène, un groupe hydroxyle, cyano, halogéno, O-alkyle, O-cycloalkyle, SO₂R₈, N(R₈)₂, COR₈, CON(R₈)₂, alkyle, (CH₂)ₜ-cycloalkyle, (CH₂)ₜ-hétérocycloalkyle, (CH₂)ₜ-aryle ou (CH₂)ₜ-hétéroaryle, où ledit R₄ alkyle, (CH₂)ₜ-cycloalkyle, (CH₂)ₜ-hétérocycloalkyle, (CH₂)ₜ-aryle ou (CH₂)ₜ-hétéroaryle est éventuellement substitué par R₁ ;
chaque R₅ représente indépendamment un atome d'hydrogène, un groupe alkyle, alcényle, (CH₂)ₜ-cycloalkyle, (CH₂)ₜ-hétérocycloalkyle, (CH₂)ₜ-aryle ou (CH₂)ₜ-hétéroaryle où ledit substituant R₅ alkyle, alcényle, (CH₂)ₜ-cycloalkyle, (CH₂)ₜ-hétérocycloalkyle, (CH₂)ₜ-aryle ou (CH₂)ₜ-hétéroaryle est éventuellement substitué par un ou plusieurs groupes hydroxyle, aryle, hétéroaryle, halogène, alkyle, cycloalkyle, SO₂R₈, -NR₈COR₈, -CON(R₈)₂, COOR₈, -C(O)R₈, -CN ou N(R₈)₂, où ledit substituant aryle, alkyle, cycloalkyle et hétéroaryle est éventuellement substitué par un ou plusieurs atomes d'halogène, groupes alkyle, hydroxyle ou -O-alkyle ;
chaque R₆ représente indépendamment un atome d'hydrogène, un groupe alkyle, (CH₂)ₜ-cycloalkyle, (CH₂)ₜ-hétérocycloalkyle, (CH₂)ₜ-aryle ou (CH₂)ₜ-hétéroaryle où ledit (CH₂)ₜ-cycloalkyle, (CH₂)ₜ-hétérocycloalkyle, (CH₂)ₜ-aryle ou (CH₂)ₜ-hétéroaryle est éventuellement substitué par un à trois R₇ ;
chaque R₇ représente indépendamment un groupe alkyle, hydroxyle, alcoxy, halogène, cyano, amino, alkylamino, dialkylamino, (CH₂)ₜ-cycloalkyle, (CH₂)ₜ-hétérocycloalkyle, (CH₂)ₜ-aryle ou (CH₂)ₜ-hétéroaryle ;
chaque R₈ représente indépendamment un atome d'hydrogène, un groupe alkyle, (CH₂)ₜ-cycloalkyle, (CH₂)ₜ-hétérocycloalkyle , (CH₂)ₜ-aryle ou (CH₂)ₜ-hétéroaryle, où ledit (CH₂)ₜ-cycloalkyle, (CH₂)ₜ-hétérocycloalkyle, (CH₂)ₜ-aryle ou (CH₂)ₜ-hétéroaryle est éventuellement substitué par un groupe alkyle, halogéno ou cyano ;
R₉ représente un atome d'hydrogène ou un groupe alkyle ;
n représente un nombre entier choisi parmi 1, 2 et 3 ;
p représente un nombre entier choisi parmi 0 et 1, à condition que si p vaut 1, alors n vaille 2 ou 3 ;
q représente un nombre entier choisi parmi 0 et 1, à condition que si p vaut 0, alors q vaille 0 ;
chaque t représente un nombre entier choisi indépendamment parmi 0, 1, 2 et 3 ; ou des sels pharmaceutiquement acceptables de celui-ci.

2. Composé selon la revendication 1 ou sel pharmaceutiquement acceptable de celui-ci, dans lequel A représente un groupe aryle, hétéroaryle, cycloalkyle ou hétérocycloalkyle, et A est éventuellement substitué par un substituant R₁.

3. Composé selon la revendication 1 ou sel pharmaceutiquement acceptable de celui-ci, dans lequel A représente un groupe aryle, hétéroaryle, cycloalkyle ou hétérocycloalkyle, et A est éventuellement substitué par deux substituants R₁.

4. Composé selon la revendication 3 ou sel pharmaceutiquement acceptable de celui-ci, dans lequel A représente un groupe aryle et est éventuellement substitué par deux substituants R₁.

5. Composé selon la revendication 3 ou sel pharmaceutiquement acceptable de celui-ci dans lequel A représente un groupe hétéroaryle et est éventuellement substitué par deux substituants R₁.

6. Composé ou sel pharmaceutiquement acceptable de celui-ci selon l'une quelconque des revendications précédentes, dans lequel R₁ représente indépendamment un groupe alkyle, un atome d'halogène, un groupe cyano, -N(R₈)COR₈, -COR₈, (CH₂)ₜ-cycloalkyle, (CH₂)ₜ-hétérocycloalkyle, (CH₂)ₜ-aryle, (CH₂)ₜ-hétéroaryle, (CH₂)ₜ-N(R₈)₂ ou (CH₂)ₜ-OR₆, où chaque R₁ alkyle, (CH₂)ₜ-cycloalkyle, (CH₂)ₜ-hétérocycloalkyle, (CH₂)ₜ-aryle ou (CH₂)ₜ-hétéroaryle est éventuellement substitué indépendamment par un groupe cyano, alkyle, un atome d'halogène ou -OR₆.

7. Composé ou sel pharmaceutiquement acceptable de celui-ci selon l'une quelconque des revendications précédentes, dans lequel B représente un groupe aryle et est substitué par un seul substituant R₄ et R₄ représente un atome d'halogène.

8. Composé ou sel pharmaceutiquement acceptable de celui-ci selon l'une quelconque des revendications précédentes, dans lequel Z représente (CH₂)ₙ-Oₚ-(CH₂)_{q} et p vaut 0.

9. Composé ou sel pharmaceutiquement acceptable de celui-ci selon l'une quelconque des revendications précédentes, dans lequel R₂ représente un groupe alkyle.

10. Composé ou sel pharmaceutiquement acceptable de celui-ci selon l'une quelconque des revendications précédentes, dans lequel R_{3A} et R_{3B} représentent chacun un atome d'hydrogène.

11. Composé ou sel pharmaceutiquement acceptable de celui-ci selon l'une quelconque des revendications précédentes, dans lequel R₅ est choisi indépendamment dans le groupe comprenant un atome d'hydrogène et un groupe alkyle.

12. Composé de formule I selon la revendication 1, choisi dans le groupe comprenant :
le 2,2-dioxyde de (5*R*, 7*S*)-1-(3-fluorophényl)-8-(3-isopropoxybenzyl)-7-méthyl-2-thia-1,3,8-triazaspiro-[4,5]décane ;
le 2,2-dioxyde de (5*R*, 7*S*)-1-(3-fluorophényl)-8-(3-isopropoxybenzyl)-7-méthyl-3-(pyridin-2-ylméthyl)-2-thia-1,3,8-triazaspiro-[4,5]décane ;
le 5-{[(5*R*, 7*S*)-1-(3-fluorophényl)-3,7-diméthyl-2,2-dioxydo-2-thia-1,3,8-triazaspiro[4,5]déc-8-yl]-méthyl}-2'-méthylbiphényl-2-ol ;
le 2,2-dioxyde de (5*R*, 7*S*)-1-(3-fluorophényl)-7-méthyl-8-[3-(4-méthylpyridin-3-yl)benzyl]-2-thia-1,3,8-triazaspiro-[4,5]décane ;
le 2-chloro-4-{[(5*R*, 7*S*)-1-(3-fluorophényl)-7-méthyl-2,2-dioxydo-2-thia-1,3,8-triazaspiro[4,5]déc-8-yl]méthyl}phénol ;
le 2,2-dioxyde de (5*R*, 7*S*)-1-(3-fluorophényl)-7-méthyl-8-[(2'-méthylbiphényl-3-yl)méthyl]-2-thia-1,3,8-triazaspiro[4,5]décane ;
le 2,2-dioxyde de (5*R*, 7*S*)-8-[(2'-chlorobiphényl-3-yl)méthyl]-1-(3-fluorophényl)-7-méthyl-2-thia-1,3,8-triazaspiro[4,5]décane ;
le 2-éthoxy-4-{[(5*R*, 7*S*)-1-(3-fluorophényl)-7-méthyl-2,2-dioxydo-2-thia-1,3,8-triazaspiro[4,5]déc-8-yl]méthyl}phénol ;
le 2,2-dioxyde de (5*R*, 7*S*)-8-[(2'-éthylbiphényl-3-yl)méthyl]-1-(3-fluorophényl)-7-méthyl-2-thia-1,3,8-triazaspiro[4,5]décane ;
le 2,2-dioxyde de (5*R*,7*S*)-1-(3-fluorophényl)-7-méthyl-8-(3-{[(1*R*)-1-méthylpropyl]oxy}benzyl)-2-thia-1,3,8-triazaspiro[4,5]décane ;
le 4-{[(5*R*, 7*S*)-1-(3-fluorophényl)-3,7-diméthyl-2,2-dioxydo-2-thia-1,3,8-triazaspiro[4,5]déc-8-yl]-méthyl}-2-isopropoxyphénol ;
le 2,2-dioxyde de (5*R*,7*S*)-1-(3-fluorophényl)-3,7-diméthyl-8-[(2'-méthylbiphényl-3-yl)méthyl]-2-thia-1,3,8-triazaspiro[4,5]décane ;
le 2,2-dioxyde de (5*R*, 7*S*)-8-{[4-(cyclopropyl-méthyl)-1,3-thiazol-5-yl]méthyl}-1-(3-fluorophényl)-3,7-diméthyl-2-thia-1,3,8-triazaspiro[4,5]décane ;
le 2,2-dioxyde de (5*R*,7*S*)-1-(3-fluorophényl)-8-[(4-isobutyl-1,3-thiazol-5-yl)méthyl]-3,7-diméthyl-2-thia-1,3,8-triazaspiro[4,5]décane ;
le 2,2-dioxyde de (5*R*,7*S*)-8-{[4-(cyclobutyl-méthyl)-1,3-thiazol-5-yl]méthyl}-1-(3-fluorophényl)-3,7-diméthyl-2-thia-1,3,8-triazaspiro[4,5]décane ;
le 2,2-dioxyde de (5*R*,7*S*)-1-(3-fluorophényl)-3,7-diméthyl-8-{3-[(2-méthyl-1,3-benzoxazol-6-yl)oxy]-benzyl}-2-thia-l,3,8-triazaspiro[4,5]décane ;
le 2,2-dioxyde de (5*R*, 7*S*)-8-[(3-éthyl-1H-indazol-5-yl)méthyl]-1-(3-fluorophényl)-3,7-diméthyl-2-thia-1,3,8-triazaspiro[4,5]décane ;
le 2,2-dioxyde de (5*R*,7*S*)-1-(3-fluorophényl)-3,7-diméthyl-8-{[3-(trifluorométhyl)-1H-indazol-5-yl]-méthyl}-2-thia-1,3,8-triazaspiro[4,5]décane ;
le 2'-éthyl-5-{[(5*R*,7*S*)-1-(3-fluorophényl)-3,7-diméthyl-2,2-dioxydo-2-thia-1,3,8-triazaspiro[4,5]déc-8-yl]méthyl}biphényl-2-ol ;
le 2'-fluoro-5-{[(5*R*, 7*S*)-1-(3-fluorophényl)-3,7-diméthyl-2,2-dioxydo-2-thia-1,3,8-triazaspiro[4,5]déc-8-yl]méthyl}biphényl-2-ol ;
le 5'-fluoro-5-{[(5*R*, 7*S*)-1-(3-fluorophényl)-3,7-diméthyl-2,2-dioxydo-2-thia-1,3,8-triazaspiro[4,5]déc-8-yl]méthyl}-2'-méthylbiphényl-2-ol ;
le 4'-fluoro-5-{[(5*R*,7*S*)-1-(3-fluorophényl)-3,7-diméthyl-2,2-dioxydo-2-thia-1,3,8-triazaspiro[4,5]déc-8-yl]méthyl}-2'-méthylbiphényl-2-ol ;
le 2'-chloro-5-{[(5*R*, 7*S*)-1-(3-fluorophényl)-3,7-diméthyl-2,2-dioxydo-2-thia-1,3,8-triazaspiro[4,5]déc-8-yl]méthyl}biphényl-2-ol ;
le 6-{[(5*R*, 7*S*)-1-(3-fluorophényl)-3,7-diméthyl-2,2-dioxydo-2-thia-1,3,8-triazaspiro[4,5]déc-8-yl]-méthyl}-4-isopropoxypyridin-3-ol ;
le 2-cyclopentyl-4-{[(5*R*, 7*S*)-1-(3-fluorophényl)-3,7-diméthyl-2,2-dioxydo-2-thia-1,3,8-triazaspiro[4,5]-déc-8-yl]méthyl}phénol ;
le 4-{[(5*R*, 7*S*)-1-(3-fluorophényl)-3,7-diméthyl-2,2-dioxydo-2-thia-1,3,8-triazaspiro[4,5]déc-8-yl]-méthyl}-2-isobutylphénol ;
le 2-cyclohexyl-4-{[(5*R*, 7*S*)-1-(3-fluorophényl)-3,7-diméthyl-2,2-dioxydo-2-thia-1,3,8-triazaspiro[4,5]-déc-8-yl]méthyl}phénol ;
le 2,2-dioxyde de (5*R*,7*S*)-1-(3-fluorophényl)-8-(3-isopropoxybenzyl)-7-méthyl-3-pyrimidin-2-yl-2-thia-1,3,8-triazaspiro[4,5]décane ;
le 2,2-dioxyde de (5*R*,7*S*)-1-(3-fluorophényl)-8-(3-isopropoxybenzyl)-7-méthyl-3-(pyrimidin-2-ylméthyl)-2-thia-1,3,8-triazaspiro[4,5]décane ;
le 2,2-dioxyde de (5*R*,7*S*)-1-(3-fluorophényl)-8-(3-isopropoxybenzyl)-7-méthyl-3-(pyrimidin-2-ylméthyl)-2-thia-1,3,8-triazaspiro[4,5]décane ;
le 2,2-dioxyde de (5*R*,7*S*)-1-(3-fluorophényl)-8-(3-isopropoxybenzyl)-3-(isoxazol-3-ylméthyl)-7-méthyl-2-thia-1,3,8-triazaspiro[4,5]décane ; ou
des sels pharmaceutiquement acceptables de ceux-ci.

13. Composé selon l'une quelconque des revendications 1 à 12, pour une utilisation dans le traitement d'une maladie ou d'un état choisi dans le groupe comprenant des troubles neurologiques et psychiatriques.

14. Composé pour une utilisation selon la revendication 13, où la maladie est la maladie d'Alzheimer.

15. Composition pharmaceutique comprenant un composé selon la revendication 1 ou un sel pharmaceutiquement acceptable de celui-ci et un support pharmaceutiquement acceptable.

16. Composition selon la revendication 15, comprenant en outre un antipsychotique atypique, un inhibiteur de la cholinestérase, du dimébon ou un antagoniste des récepteurs NMDA.
